(19) Europäisches Patentamt
European Patent Office
Office européen des brevets

(11) EP 0 496 617 B1

(12) **EUROPEAN PATENT SPECIFICATION**

(45) Date of publication and mention of the grant of the patent: **01.12.1999 Bulletin 1999/48**

(51) Int Cl.[6]: **C07H 19/16**, C07H 19/14, C07H 19/23, C07D 473/34, C07D 487/04, A61K 31/70, A61K 31/505

(21) Application number: **92300580.5**

(22) Date of filing: **23.01.1992**

(54) **Adenosine kinase inhibitors**

Adenosinkinaseinhibitoren

Inhibiteurs de kinase d'adénosine

(84) Designated Contracting States:
**AT BE CH DE DK ES FR GB GR IT LI LU MC NL PT SE**

(30) Priority: **23.01.1991 US 647117**
**23.12.1991 US 812916**

(43) Date of publication of application:
**29.07.1992 Bulletin 1992/31**

(73) Proprietor: **Metabasis Therapeutics Inc.**
**San Diego, CA 92121 (US)**

(72) Inventors:
- **Browne, Clinton E.**
  **Vista, California 92083 (US)**
- **Ugarkar, Bheemarao G.**
  **Escondido, CA 92025 (US)**
- **Mullane, Kevin M.**
  **De Mar, CA 92014 (US)**
- **Gruber, Harry E.**
  **San Diego, CA 92130 (US)**
- **Bullough, David A.**
  **San Diego, CA 92129 (US)**
- **Erion, Mark D.**
  **Del Mar, CA 92104 (US)**
- **Castellino, Angelo**
  **San Diego, CA 92111 (US)**

(74) Representative: **Sexton, Jane Helen et al**
**J.A. KEMP & CO.**
**14 South Square**
**Gray's Inn**
**London WC1R 5LX (GB)**

(56) References cited:
- **CHEMICAL ABSTRACTS, vol. 93, no. 17, October 27, 1980, Columbus, Ohio, USA R.F. BRUNS "Adenosine re- ceptor activation in human fibroblasts: nucleoside agonists and antagonists.", page 24, abstract-no. 160 970q**
- **CHEMICAL ABSTRACTS, vol. 91, no. 21, November 19, 1979, Columbus, Ohio, USA L.L. WOTRING et al. "Study of the cytotoxicity and meta- bolism of 4-amino-3-carbox- amido-1-(beta-D-ribo- furanosyl)pyrazolo(3,4-d)- pyrimidine using inhibitors of adenosine kinase and adenosine deaminase.", page 26, abstract-no. 168 263r**
- **CHEMICAL ABSTRACTS, vol. 79, no. 3, July 23, 1973, Columbus, Ohio, USA J.F. HENDERSON et al. "Inhibitors of nucleoside and nucleotide metabolism.", page 9, abstract-no. 13 413t**
- **CHEMICAL ABSTRACTS, vol. 75, no. 17, October 25, 1971, Columbus, Ohio, USA I.C. CALDWELL et al. "Inhibitors of adenosine kinase.", page 253, abstract-no. 108 070k**
- **CHEMICAL ABSTRACTS, vol. 95, no. 15, October 12, 1981, Columbus, Ohio, USA G.A. BHAT et al. "Pyrazolo- pyrimidine nucleosides. 12. Synthesis and biological activity of certain pyrazolo- (3,4-d)pyrimidine nucleosides related to adenosine.", page 20, abstract-no. 125 862a**
- **CHEMICAL ABSTRACTS, vol. 85, no. 23, December 06, 1976, Columbus, Ohio, USA H. SUEHIRO et al. "2-substituted thio- adenosines.", page 571, abstract-no. 177 889c**
- **CHEMICAL ABSTRACTS, vol. 82, no. 5, February 03, 1975, Columbus, Ohio, USA J.A. MONTGOMERY et al. "Analogs of 5'-deoxy-5'- -(methylthio)adenosine.", page 17, abstract-no. 25 686j**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 496 617 B1

Remarks:

The file contains technical information submitted after the application was filed and not included in this specification

## Description

This invention relates to adenosine kinase inhibitors and to novel nucleoside analogs, specifically to purine, pyrrolo[2,3-d] pyrimidine and pyrazolo[3,4-d]pyrimidine nucleoside analogs having activity as adenosine kinase inhibitors. The invention also relates to their preparation and the use of these and other adenosine kinase inhibitors in the treatment of cardiovascular, and cerebrovascular diseases, inflammation and other diseases which can be regulated by increasing the local concentration of adenosine.

## BACKGROUND OF THE INVENTION

Adenosine has been reported to have cardioprotective (Olafsson et al., Circulation, **1987,** 76:1135-1145) and neuroprotective properties (Dragunow and Faull, Trends in Pharmacol. Sci., **1988,** 9:193; Marangos, Medical Hypothesis, **1990**,32.:45). It is reportedly released from cells in response to alterations in the supply of or demand for oxygen (Schrader, Circulation, **1990,** 81:389-391), is said to be a potent vasodilator, and is believed to be involved in the metabolic regulation of blood flow (Berne, Circ. Res., **1980,** 47:808-813). However, adenosine has a short half life (< 1 sec) in human blood (Moser, et al., Am. J. Physiol., **1989,** 256:C799-C806), and therefore high doses of adenosine would need to be administered continuously to achieve effective levels. Adenosine has been reported to exhibit negative inotropic, chronotropic and dromotropic effects (Belardinelli et al., Prog. in Cardiovasc. Diseases, **1989**, 32:73-97) and to cause coronary steal by preferentially dilating vessels in nonischemic regions. Consequently, high doses of adenosine are toxic and severely limit its therapeutic potential. However, it is believed that by increasing adenosine concentration locally, i.e. at the target site within the target tissue, the beneficial effects of adenosine can be provided without the toxic systemic effects.

Adenosine kinase is a cytosolic enzyme which catalyzes the phosphorylation of adenosine to AMP. Inhibition of adenosine kinase can potentially reduce the ability of the cell to utilize adenosine, leading to increased adenosine outside of the cell where it is pharmacologically active. However, the regulation of adenosine concentration is complex and involves other adenosine-metabolizing enzymes each with different kinetic properties and mechanisms of regulation. Adenosine can also be deaminated to inosine by adenosine deaminase (ADA) and condensed with L-homocysteine to S-adenosylhomocysteine (SAH) by SAH hydrolase. The role of each of these enzymes in modulating adenosine concentration is dependent on the prevailing physiological conditions, is tissue specific and is not well understood.

A number of nucleosides including purine, pyrrolo[2,3-d]pyrimidine and pyrazolo[3,4-d]pyrimidine analogs have been evaluated for inhibition of adenosine kinase but were reported to have $K_i$'s of greater than 800 nM (Caldwell and Henderson Cancer Chemother. Rep., **1971** 2:237-246; Miller et al., J. Biol. Chem., **1979,** 254:2346-2352). A few compounds have been reported as potent inhibitors of adenosine kinase with $K_i$'s of less than 100 nM. These are the purine nucleosides, 5'-amino-5'-deoxyadenosine (Miller et al., J. Biol. Chem., **1979,** 254:2346-2352) and 1,12-bis(adenosin-$N_6$-yl)dodecane (Prescott et al., Nucleosides & Nucleotides, **1989,** 8:297), and the pyrrolopyrimidine nucleosides, 5-iodotubercidin (Henderson et al., Cancer Chemotherapy Rep. Part 2, **1972,** 3:71-85; Bontemps et al., Proc. Natl. Acad. Sci. USA, **1983,** 80:2829-2833; Davies et al., Biochem. Pharmacol., **1986,** 35:3021-3029) and 5'-deoxy-5-iodotubercidin (Davies et al., Biochem. Pharmacol., **1984,** 33:347-355; Davies et al., Biochem. Pharmacol., **1986,** 35:3021-3029).

Some of these compounds have been used to evaluate whether adenosine kinase inhibition might lead to increased extracellular adenosine concentrations. In rat cardiomyocytes, inhibition of adenosine deaminase by 2'-deoxycoformycin was reported to have no effect on adenosine release from the cells. In contrast, inhibition of ADA together with adenosine kinase by 5'-amino-5'-deoxyadenosine resulted in a 6-fold increase in adenosine release (Zoref-Shani et al., J. Mol. Cell. Cardiol., **1988,** 20:23-33). The effects of the adenosine kinase inhibitor alone were not. reported. Similar results were reported in isolated guinea pig hearts; in these studies addition of 5'-amino-5'-deoxyadenosine to the perfusion medium, in the presence of EHNA to inhibit deamination, was reported to result in a 15-fold increase of adenosine release (Schrader in Regulatory Function of Adenosine; (Berne et al.) eds. pp. 133-156, **1983**). These effects were not apparent in the absence of ADA inhibition and other studies using isolated rat hearts perfused with 5-iodotubercidin alone, have reported no increase in perfusate adenosine concentration under normoxic conditions (Newby et al., Biochem. J., **1983,** 214:317-323) or under hypoxic, anoxic or ischemic conditions (Achtenberg et al., Biochem. J., **1986,** 235:13-17). In other studies, adenosine release has been measured in neuroblastoma cells in culture and compared with that of a variant deficient in adenosine kinase ($AK^-$). The $AK^-$ cells used in this study were said to release adenosine at an accelerated rate; the concentration of adenosine in the growth medium was reported to be elevated compared to the normal cells (Green, J. Supramol. Structure, **1980,** 13:175-182). In rat and guinea pig brain slices, adenosine uptake was reportedly inhibited by the adenosine kinase inhibitors, 5-iodotubercidin and 5'-deoxy-5-iodotubercidin (Davis et al., Biochem. Pharmacol., **1984**, 33:347-355). However, inhibition of uptake and intracellular trapping via phosphorylation does not necessarily result in increased. extracellular adenosine, since the

adenosine could enter other metabolic pathways or the percentage of adenosine being phosphorylated could be insignificant compared to the total adenosine removed.

The effects of adenosine and certain inhibitors of adenosine catabolism, including 5-iodotubericidin were evaluated in an experiemental model in which dog hearts were subjected to ischemia and reperfusion; 5-iodotubericidin was reported to have inconsistent effects. (Wu, et al., Cytobios, **1987,** 50:7-12).

Although the adenosine kinase inhibitors, 5'-amino-5'-deoxyadenosine and 5-iodotubercidin have been widely used in experimental models, the susceptibility of 5'-amino-5'-deoxyadenosine to deamination, and hence its potentially short half life, and the cytotoxicity of 5-iodotubercidin make their clinical utility limited and may limit interpretations-based on these compounds. The pyrrolo[2,3-d]pyrimidines, 5-iodotubercidin and 5'-deoxy-5-iodotubercidin have been reported to cause pronounced general flaccidity and much-reduced spontaneous locomotor activity in mice, interpreted to be skeletal muscle relaxation; to cause hypothermia in mice; and to decrease blood pressure and heart rate in anesthetized rats (Daves et al., Biochem. Pharmacol., **1984,** 33:347-355; Daves et al., Biochem. Pharmacol., **1986,** 35:3021-3029; U.S. Patent No. 4,455,420). The skeletal muscle effects of these compounds have been poorly documented, while the other effects were considered significant toxicities. It is believed that studies using these compounds were curtailed due to these toxicities and also because of their limited availability.

## SUMMARY OF THE INVENTION

The present invention is directed to novel compounds which are potent and selective adenosine kinase inhibitors.

In one aspect, the present invention is directed to certain novel compounds which inhibit adenosine kinase, to the preparation of these compounds, and to the in vitro and in vivo adenosine kinase inhibition activity of these compounds. Another aspect of the present invention is directed to the clinical use of adenosine kinase inhibitors as a method of increasing-adenosine concentrations in biological systems. In vivo inhibition of adenosine kinase prevents phosphorylation of adenosine resulting in higher local concentrations of endogenous adenosine. As a result of the very short half-life of adenosine and very low quantities of adenosine in tissues, this effect is most pronounced in regions producing the most adenosine such as ischemic regions. Hence, the beneficial effects of adenosine are enhanced in a site and event specific manner and toxic systemic effects are reduced.

In particular, in one preferred aspect, the present invention is directed to novel nucleoside analogs which comprise a 5'-modified ribose linked to a substituted purine, pyrrolo[2,3-d]pyrimidine, or pyrazolo[3,4-d]pyrimidine base. Certain preferred compounds within these groups possess potencies many times greater than previously described inhibitors of adenosine kinase. The compounds of the present invention possess advantages for pharmaceutical use such as enhanced pharmacological selectivity, efficacy, bioavailability, ease of manufacture and compound stability. This invention also discloses novel processes for the preparation of these compounds.

The novel compounds of the present invention and other adenosine kinase inhibitors may be used clinically to treat medical conditions where an increased localized adenosine concentration is beneficial. Accordingly, the present invention is directed to the prophylactic and affirmative treatment of ischemic conditions such as myocardial infarction, angina, percutaneous transluminal coronary angiography (PTCA), stroke, other thrombotic and embolic conditions, neurological conditions such as seizures and psychosis, and other conditions benefitted by enhanced adenosine levels such as inflammation, arthritis, autoimmune diseases, cardiac arrhythmias, ulcers and irritable bowel syndrome. These compounds are useful as muscle relaxants and also in inducing sleep and in treating anxiety.

The present invention is also directed to prodrugs and pharmaceutically acceptable salts of the compounds described herein and to pharmaceutical compositions suitable for different routes of drug administration and which comprise a therapeutically effective amount of an adenosine kinase inhibitor compound described herein admixed with a pharmacologically acceptable carrier or diluent.

### Definitions

In accordance with the present invention and as used herein, the following terms, are defined with the following meanings, unless explicitly stated otherwise.

The term "hydrocarbyl" refers to an organic radical comprised of carbon chains to which hydrogen and other-elements are attached. The term includes alkyl, alkenyl, alkynyl and aryl groups, groups which have a mixture of saturated and unsaturated bonds, carbocyclic rings and includes combinations of such groups. It may refer to straight-chain, branched-chain cyclic structures or combinations thereof.

The term "aryl" refers to aromatic groups which have at least one ring having a conjugated pi electron system and includes carbocyclic aryl, heterocyclic aryl and biaryl groups, all of which may be optionally substituted.

Carbocyclic aryl groups are groups wherein the ring atoms on the aromatic ring are carbon atoms. Carbocyclic aryl groups include monocyclic carbocyclic aryl groups and optionally substituted naphthyl groups.

The term "monocyclic carbocyclic aryl" refers to optionally substituted phenyl, being preferably phenyl or phenyl substituted by one to three substituents, such being advantageously lower alkyl, hydroxy, lower alkoxy, lower alkanoyloxy, halogen, cyano, trihalomethyl, lower acylamino or lower alkoxycarbonyl.

"Optionally substituted naphthyl" refers to 1- or 2-naphthyl or 1- or 2-naphthyl preferably substituted by lower alkyl, lower alkoxy or halogen.

Heterocyclic aryl groups are groups having from 1 to 3 heteroatoms as ring atoms in the aromatic ring and the remainder of the ring atoms carbon atoms. Suitable heteroatoms include oxygen, sulfur, and nitrogen, and suitable heterocyclic aryl groups include furanyl, thienyl, pyridyl, pyrrolyl, N-lower alkyl pyrrolo, pyrimidyl, pyrazinyl, imidazolyl, and the like, all optionally substituted.

Optionally substituted furanyl represents 2- or 3-furanyl or 2- or 3-furanyl preferably substituted by lower alkyl or halogen.

Optionally substituted pyridyl represents 2-, 3- or 4-pyridyl or 2-, 3- or 4-pyridyl preferably substituted by lower alkyl or halogen.

Optionally substituted thienyl represents 2- or 3-thienyl, or 2- or 3-thienyl preferably substituted by lower alkyl or halogen.

The term "biaryl" represents phenyl substituted by carbocyclic aryl or heterocyclic aryl as defined herein, ortho, meta or para to the point of attachment of the phenyl ring, advantageously para; biaryl is also represented as the $-c_6H_4-Ar$ substituent where Ar is aryl.

The term "aralkyl" refers to an alkyl group substituted with an aryl group. Suitable aralkyl groups include benzyl, picolyl, and the like, and may be optionally substituted.

The term "lower" referred to herein in connection with organic radicals or compounds respectively defines such with up to and including 7, preferably up to and including 4 and advantageously one or two carbon atoms. Such groups may be straight chain or branched.

The terms (a) "alkyl amino", (b) "arylamino", and (c) "aralkylamino", respectively, refer to the groups -NRR' wherein respectively, (a) R is alkyl and R' is hydrogen or alkyl; (b) R is aryl and R' is hydrogen or aryl, and (c) R is aralkyl and R' is hydrogen or aralkyl.

The term "acyl" refers to hydrocarbyl-CO- or HCO-.

The terms "acylamino" refers to RC(O)N(R)- and $(RCO)_2N$-wherein each R is independently hydrogen or hydrocarbyl.

The term "α-alkoxyalkylidene" refers to hydrocarbyl-O-CR (an orthoester) wherein R is hydrogen or hydrocarbyl.

The term "hydrocarbyloxycarbonyloxy" refers to the group ROC(O)O- wherein R is hydrocarbyl.

The term "lower carboalkoxymethyl" or "lower hydrocarbyloxycarbonymethyl" refers to hydrocarbyl-OC(O) $CH_2$- with the hydrocarbyl group containing ten or less carbon atoms.

The term "carbonyl" refers to -C(O)-.

The term "carboxamide" or "carboxamido" refers to $-CONR_2$ wherein each R is independently hydrogen or hydrocarbyl.

The term "lower hydrocarbyl" refers to any hydrocarbyl group of ten or less carbon atoms.

The term "alkyl" refers to saturated aliphatic groups including straight-chain, branched chain and cyclic groups.

The term "alkenyl" refers to unsaturated hydrocarbyl groups which contain'at least one carbon-carbon double bond and includes straight-chain, branched-chain and cyclic groups.

The term "alkynyl" refers to unsaturated hydrocarbyl groups which contain at least one carbon-carbon triple bond and includes straight-chain, branched-chain and cyclic groups.

The term "halogen" refers to fluorine, chlorine, bromine or iodine.

The term "hydrocarbyloxycarbonylamino" refers to a urethane, hydrocarbyl-O-CONR- wherein R is H or hydrocarbyl and wherein each hydrocarbyl is independently selected.

The term "di(hydrocarbyloxycarbonyl)amino" refers to $(hydrocarbyl-O-CO)_2N$- wherein each hydrocarbyl is independently selected.

The term "hydrocarbylamino" refers to -NRR' wherein R is hydrocarbyl and R' is independently selected hydrocarbyl or hydrogen.

The term "mercapto" refers to SH or a tautomeric form.

The term "methine" refers to

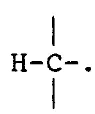

The term "methylene" refers to $-CH_2-$.
The term "alkylene" refers to a divalent straight chain or branched chain saturated aliphatic radical.
The term "oxy" refers to -O- (oxygen).
The term "thio" refers to -S- (sulfur).
The term "prodrug" as used herein refers to any compound that has less intrinsic activity than the "drug" but when administered to a biological system generates the "drug" substance either as a result of spontaneous chemical reaction or by enzyme catalyzed or metabolic reaction. Reference is made to various prodrugs such as acyl esters, carbonates, and urethanes, included herein as examples. The groups illustrated are exemplary, not exhaustive and one skilled in the art could prepare other known varieties of prodrugs. Such prodrugs of the compounds of Formula I, fall within the scope of the present invention.
The term "pharmaceutically acceptable salt" includes salts of compounds of Formula I derived from the combination of a compound of this invention and an organic or inorganic acid. The compounds of Formula I are useful in both free base and salt form. In practice the use of salt form amounts to use of base form; both forms are within the scope of the present invention.

BRIEF DESCRIPTION OF THE DRAWINGS

Figure 1 depicts the effects of the adenosine kinase inhibitor GP-1-238 on mean arterial pressure, heart rate and body temperature following intravenous administration to anesthetized or conscious rats.
Figure 2 depicts the dose-dependent inhibition of neutrophil adhesion to endothelial cells by the adenosine kinase inhibitors GP-1-272 and GP-1-456 and the reversal of this inhibition by co-treatment with adenosine deaminase ("ADA").
Figure 3 depicts the dose-dependent inhibition of contraction in the isolated ileum by the adenosine kinase inhibitors (A) GP-1-515 and (B) GP-1-547 and the reversal of this inhibition by co-treatment with the adenosine receptor antagonist, 8-sulfophenyltheophylline, or adenosine deaminase.
Figure 4 depicts (A) the dose-dependent inhibition of pentylenetetrazole (PTZ) induced seizures by the adenosine kinase inhibitor GP-1-456, and (B) the reversal of this inhibition by the central adenosine receptor antagonist theophylline but not the peripheral antagonist 8-sulfophenyltheophylline.
Figures 5 to 9 depict reaction schemes for preparing certain of these adenosine kinase inhibitors.
Figure 10 depicts the structures of certain preferred intermediates useful in the synthesis of adenosine kinase inhibitors.

**DETAILED DESCRIPTION OF THE INVENTION**

**NOVEL ADENOSINE KINASE INHIBITORS**

The present invention relates to novel adenosine kinase inhibitors which comprise compounds of the general formula I.

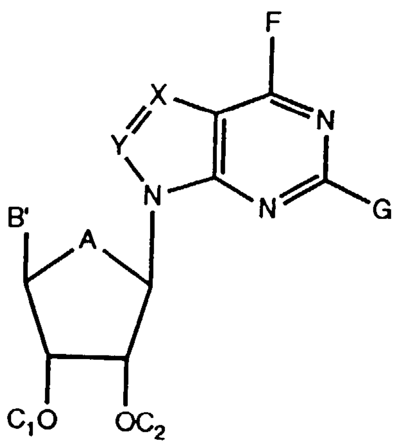

(I)

wherein:

(a) A is oxygen, methylene or sulfur;
(b) B' is $-(CH_2)_n-B$ wherein n is 1, 2, 3 or 4 and B is hydrogen, alkyl, alkoxy, amino, alkylamino, acylamino, hydrocarbyloxycarbonylamino, mercapto, alkylthio, azido, cyano, halogen, or B' is alkenyl or alkynyl;
(c) $C_1$ and $C_2$ are each independently hydrogen, acyl, hydrocarbyloxycarbonyl or taken together form a 5-membered ring wherein $C_1$ is a single bond to $C_2$ and $C_2$ is carbonyl or α-alkoxyalkylidene;
(d) X is

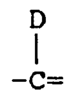

and Y is -N= or

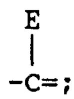

(e) D is hydrogen, halogen, alkyl, aryl, aralkyl, alkenyl, alkynyl, haloalkyl, cyano, cyanoalkyl, acyl, carboxamido, a carboxylic acid or carboxylic acid ester group, alkoxy, aryloxy, aralkyloxy, alkylthio, arylthio, aralkylthio, amino, alkylamino, arylamino, aralkylamino, acylamino, or nitro;
(f) E is hydrogen, halogen, alkyl, alkylamino, azido or alkylthio;
(g) F is alkyl, aryl, aralkyl, halogen, amino, alkylamino, arylamino, aralkylamino, cyano, cyanoalkyl, alkoxy, aryloxy, aralkoxy, alkylthio, arylthio, aralkylthio; optionally substituted indolinyl or indolyl; pyrrolidinyl or piperazinyl; and
(h) G is hydrogen, halogen, lower alkyl, lower alkoxy, lower alkylamino or lower alkylthio; and pharmaceutically acceptable salts thereof; with the proviso that:

when A is oxygen and

(i) X is

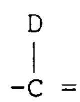

and Y is

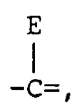

then if B' is methyl, D is halogen, cyano or carboxamido, F is amino, then G is not hydrogen; or if D is hydrogen, then F is not amino; or
(ii) X is

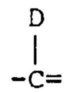

and Y is -N=, if B is hydrogen or halogen, D and G are hydrogen, then F is not amino; or when A is methylene, X is

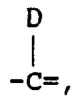

Y is

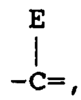

B, D, E and G are hydrogen, then F is not amino.

According to an alternative aspect of the present invention, novel adenosine kinase inhibitors are provided which have a 5'-group which comprises a hydroxyl or hydroxyl derivative. However, it is believed due to their overall structures, those compounds which have a 5'-hydroxyl would not act as substrates for phosphorylation enzymes and, thus, would be unlikely to undergo 5'-phosphorylation or would be phosphorylated at an extremely slow rate.

One preferred group of these adenosine kinase inhibitors comprise compounds of the formula:

(II)

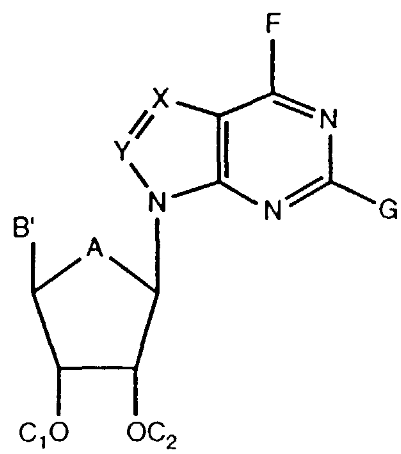

wherein:

(a) A is oxygen, methylene or sulfur;

(b) B' is $-(CH_2)_nB$ wherein n is 1, 2, 3 or 4 and B is hydroxy, acyloxy, hydrocarbyloxycarbonyloxy, or $-OCONR_2$ wherein each R is independently hydrogen or hydrocarbyl;

(c) $C_1$ and $C_2$ are each independently hydrogen, acyl, hydrocarbyloxycarbonyl or taken together form a 5-membered ring wherein $C_1$ is a single bond to $C_2$ and $C_2$ is carbonyl or α-alkoxyalkylidene;

(d) X is

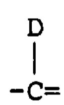

and Y is -N=;
(e) D is halogen, aryl or aralkyl;
(f) F is alkyl, aryl, aralkyl, halogen, amino, alkylamino, arylamino, aralkylamino, cyano, cyanoalkyl, alkoxy, aryloxy, aralkoxy, alkylthio, arylthio, aralkylthio, optionally substituted indolinyl or indolyl, pyrrolidinyl or piperazinyl; and
(g) G is hydrogen, halogen, lower alkyl, lower alkoxy, or lower alkylthio; and pharmaceutically acceptable salts thereof; with the proviso that when A is oxygen and D is halogen, then F is not amino.

Another preferred group of these adenosine kinase inhibitors comprise compounds of the formula:

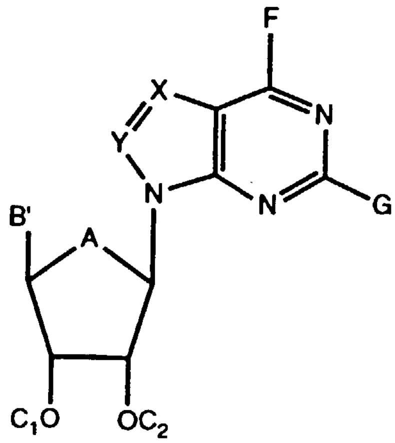

(III)

wherein:

(a) A is oxygen, methylene or sulfur;
(b) B' is $-(CH_2)_nB$ wherein n is 1, 2, 3 or 4 and B is hydroxy, acyloxy, hydrocarbyloxycarbonyloxy, or $-OCONR_2$ wherein each R is independently hydrogen or hydrocarbyl;
(c) $C_1$ and $C_2$ are each independently hydrogen, acyl, hydrocarbyloxycarbonyl or taken together form a 5-membered ring wherein $C_1$ is a single bond to $C_2$ and $C_2$ is carbonyl or α-alkoxyalkylidene;
(d) X is

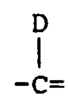

and Y is

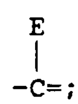

(e) D is aryl or aralkyl;
(f) E is hydrogen, halogen, alkyl, or alkylthio;
(g) F is alkyl, aryl, aralkyl, halogen, amino, alkylamino, arylamino, aralkylamino, cyano, cyanoalkyl, alkoxy, aryloxy, aralkyloxy, alkylthio, arylthio, aralkylthio, optionally substituted indolinyl or indolyl, pyrrolidinyl or piperazinyl; and
(h) G is hydrogen, halogen, lower alkyl, lower alkoxy, or lower alkylthio; and pharmaceutically acceptable salts thereof; with the proviso that: when A is oxygen, D is oxadiazolyl, triazolyl or triazinyl, and E and G are both hydrogen, then F is not amino.

Also included within the present invention are adenosine kinase inhibitors which comprise modified purine nucleosides of the formula:

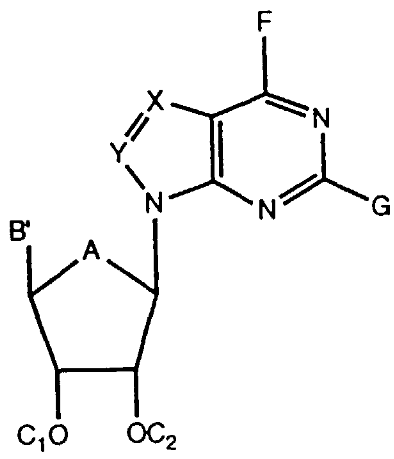

(IV)

wherein

(a) A is oxygen, methylene or sulfur;
(b) B' is $-CH_2B$ wherein and B is amino, hydrocarbylamino, or acylamino;
(c) $C_1$ and $C_2$ are each independently hydrogen, acyl, hydrocarbyloxycarbonyl or taken together form a 5-membered ring wherein $C_1$ is a single bond to $C_2$ and $C_2$ is carbonyl or $\alpha$-alkoxyalkylidene;
(d) X is -N= and Y is

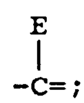

(e) E is hydrogen, halogen, alkyl, amino, alkylamino, azido, acylamino, alkoxy or alkylthio;
(f) F is halogen, amino, alkylamino, arylamino, aralkylamino, cyanoalkyl, alkoxy, aryloxy, aralkoxy, alkylthio, arylthio, aralkylthio, alkyl, aryl, aralkyl, optionally substituted indolinyl or indolyl, pyrrolidinyl or piperazinyl; and
(g) G is hydrogen, halogen, lower alkyl, lower alkoxy, or lower alkylthio and pharmaceutical acceptable salts thereof; with the proviso that:

when A is oxygen, B is amino or hydrocarbylamino, E and G are hydrogen, then F is not amino.

In general in the above formulae, preferred are compounds where G is hydrogen, halogen, alkyl particularly lower alkyl or alkylthio particularly lower alkylthio. Especially preferred G groups include hydrogen. Preferred $C_1$ and $C_2$ groups include hydrogen and acetyl.

Preferred E groups include hydrogen, halogen, alkyl, alkylamino azido or alkylthio, particularly hydrogen or halogen, especially preferred are compounds where E is hydrogen.

Preferred are compounds where A is oxygen.

Preferred are compounds where D is hydrogen, halogen alkyl, aryl, aralkyl, alkenyl or alkynyl, cyano, cyanoalkyl, alkoxy, aryloxy, aralkoxy, alkylthio, arylthio, aralkylthio, amino, alkylamino, arylamino, aralkylamino, amido, carboxamido, or hydrocarbyloxycarbonyl. Especially preferred D groups include hydrogen, halogen, alkyl, aryl, aralkyl, cyano, alkoxy, aryloxy, aralkoxy, alkenyl or alkynyl, more preferably hydrogen, halogen, aryl, cyano, alkoxy or aryloxy. A particularly preferred group of compounds include those wherein D is hydrogen, halogen or aryl especially halogen or aryl. According to one preferred aspect, D is aryl such as heterocyclic aryl or monocyclic carbocyclic aryl, such as optionally substituted phenyl.

Preferred compounds include those where B' is $-(CH_2)_nB$, and n is 1 or 2, more preferably n is 1. B may preferably include hydrogen, halogen, alkyl, amino, alkylamino, alkoxy, mercapto, alkylthio, azido or cyano; more preferably B is hydrogen, halogen, lower alkyl, amino, lower alkylamino, lower alkoxy, lower alkythio, azido or cyano. Particularly preferred B groups include hydrogen, amino or azido. Also preferred are compounds wherein B' is vinyl, ethynyl, or propargyl.

Preferred F groups include halogen, amino, alkylamino, arylamino, aralkylamino, alkylthio, arylthio, alkyl, aryl or aralkyl, more preferably amino or arylamino. Another subset of preferred F groups includes alkyl, aryl or aralkyl. Especially preferred F groups include optionally substituted anilino.

Preferred compounds of formula I include those in which Y is -N= and B is amino, D is bromo, F is amino, and $C_1$ and $C_2$ are both hydrogen or acetyl; B is amino, D is iodo, F is amino and $C_1$ and $C_2$ are both hydrogen; B is hydrogen, D is iodo, F is amino and $C_1$ and $C_2$ are both hydrogen; B is hydrogen, D is phenyl, F is anilino and $C_1$ and $C_2$ are both hydrogen; B is azido, D is p-methoxyphenyl, F is amino and $C_1$ and $C_2$ are both hydrogen; or B is azido, D is phenyl, F is anilino and $C_1$ and $C_2$ are both hydrogen.

Preferred compounds of formula I include those in which Y is -C(E)= and B is azido, D is bromo, E is hydrogen, F is amino, and $C_1$ and $C_2$ are both hydrogen; B is hydrogen, D is iodo, E is hydrogen, F is anilino, and $C_1$ and $C_2$ are both hydrogen; B is hydrogen, D is phenyl, E is hydrogen and F is anilino, and $C_1$ and $C_2$ are both hydrogen; B is hydrogen, D is phenyl, E is hydrogen, F is amino, and $C_1$ and $C_2$ are both hydrogen, B is methyl, D is iodo, E is hydrogen, F is amino and $C_1$ and $C_2$ are both hydrogen; n is 2, B is azido, D is iodo, E is hydrogen, F is amino, and $C_1$ and $C_2$ and both hydrogen; B' is vinyl, D is iodo, E is hydrogen, F is amino, G is hydrogen and $C_1$ and $C_2$ are both hydrogen; n is 2, B is hydrogen, D is iodo, E is hydrogen, F is amino and $C_1$ and $C_2$ are both hydrogen, n is 2, B is azido, D is iodo, E is hydrogen, F is amino and $C_1$ and $C_2$ are both hydrogen; or B' is vinyl, $C_1$ and $C_2$ are both hydrogen, D is iodo, E is hydrogen, F is amino, and G is hydrogen.

Preferred compounds of formula II include those in which n is 1, B is hydroxy, D is phenyl, F is anilino $C_1$ and C2 are both hydrogen, A is oxygen and G is hydrogen.

Preferred compounds of formula III include those in which A is oxygen, G is hydrogen, n is 1, B is hydroxy, D is 2-furanyl, E is hydrogen, F is anilino and $C_1$ and C2 are both hydrogen.

A. Preferred Compounds

The compounds of the present invention contain asymmetric carbon atoms and hence can exist as stereoisomers, both enantiomers and diastereomers. The individual preferred stereoisomers and mixtures thereof are considered to fall within the scope of the present invention. The compounds described by Formula I contain a 5-modified 1-β-D-ribofuranosyl group and that isomer comprises a particularly preferred diastereomeric and enantiomeric form for compounds of the present invention. Aptly, the synthetic examples cited herein provide the most preferred isomer. It is evident that in addition to the sugar moiety, additional asymmetric carbons may be present in compounds of Formula I, being present in moieties B', $C_1$ or $C_2$ or the substituted heterocyclic purine, pyrrolo[2,3-d]pyrimidine or pyrazolo [3,4-d]pyrimidine ring. In this event, both of the resulting diastereomers are considered to fall within the scope of the present invention.

It is noted that compounds of formula I where B is hydroxy (i.e. a 5'-hydroxyl moiety) are in many cases potent inhibitors of adenosine kinase. The use of compounds having formula I wherein B' replaced by $-CH_2OH$, as adenosine kinase inhibitors are included in the scope of this invention. However, since some of these compounds may be phosphorylated in vivo and since the resulting 5'-phosphates may be toxic, mutagenic or teratogenic, 5'-hydroxy compounds which can serve as substrates for phosphorylation enzymes may not comprise preferred compounds for clinical or therapeutic use. An important aspect of the novel compounds of the present invention is that these preferred compounds are either non-phosphorylatable at the 5' position or are not substrates of enzymes that lead to phosphorylation.

(i) Preferred Pyrazolo[3,4-d]pyrimidines

Preferred adenosine kinase inhibitor compounds of the present invention include certain pyrazolo[3,4-d]pyrimidine compounds of Formulas I and II.

Preferred pyrazolo[3,4-d]pyrimidine compunds of Formula I include those where G is hydrogen and A is oxygen. Preferred D groups include hydrogen, alkyl, aryl, aralkyl, cyano, alkoxy, aryloxy, aralkoxy, alkenyl or alkynyl, more preferably hydrogen, halogen, aryl, cyano, alkoxy or aryloxy, more particularly hydrogen, halogen or aryl especially halogen or aryl. An especially preferred group of compounds includes those where D is aryl, especially heterocyclic aryl or monocyclic carbocyclic aryl, more preferably optionally substituted phenyl. Preferred B' groups include $-(CH_2)_nB$ wherein B is hydrogen, halogen, alkyl, amino, alkylamino, alkoxy, mercapto, alkylthio, azido or cyano; more preferable B is hydrogen, halogen, lower alkyl, amino, lower alkylamino, azido or cyano. Particularly preferred B groups include hydrogen, amino or azido. Preferably, n is 1 or 2, more preferably 1. Other preferred B' groups include vinyl

and ethynyl. Preferred are compounds of formula I wherein F is halogen, amino, alkylamino, arylamino, aralkylamino, alkylthio, arylthio, alkyl, aryl or aralkyl, more preferably amino, arylamino, alkyl or aralkyl, aralkyl, more preferably amino, arylamino, alkyl or aralkyl, especially amino or arylamino. Certain preferred compounds include F groups which comprise an arylamino group, particularly optionally substituted anilino. $C_1$ and $C_2$ are preferably each independently hydrogen or acetyl, particularly hydrogen.

Examples of preferred pyrazolo[3,4-d]pyrimidine compounds include those noted as GP-1-515, GP-1-547, GP-1-560, GP-1-665, GP-1-666 GP-1-667, GP-1-695, and GP-1-704.

GP-1-665 is a compound of formula II in which B is hydrogen, D is phenyl, F is anilino and G is hydrogen.

Preferred pyrazolo[3,4-d]pyrimidine compunds of Formula II include those where G is hydrogen and A is oxygen. Preferred D groups include aryl. Prefered aryl groups include heterocyclic carbocyclic aryl groups, especially optionally substituted phenyl. Preferred F groups include halogen, amino, alkylamino, arylamino, aralkylamino, alkylthio, arylthio, alkyl, aryl, or aralkyl, more preferably amino or arylamino. Certain preferred compounds of Formula II may include F groups which comprise optionally substituted anilino groups.

(ii) <u>Preferred Pyrrolo[2,3-d]pyrimidines</u>

Preferred adenosine kinase compounds of the present invention include pyrrolo[2,3-d]pyrimidine compounds of Formulas I and III.

Preferred pyrrolo[2,3-d]pyrimidine compounds of Formula I include those wherein G is hydrogen. Preferred are compounds wherein E is hydrogen or halogen; more preferably E is hydrogen. Preferred are compounds where A is oxygen. Preferred compounds include those where D is hydrogen, halogen, alkyl, aryl, aralkyl, cyano, alkenyl or alkynyl. Preferred values for D include hydrogen, alkyl, aryl, aralkyl, cyano, alkenyl, or alkynyl, more preferably D is hydrogen, halogen or aryl. An especially preferred group of compounds includes those where D is aryl, especially heterocyclic aryl or monocyclic carbocyclic aryl, especially optionally substituted phenyl. Preferred B' groups include $-(CH_2)_nB$ wherein n is 1 or 2, preferably 1. Preferably, B is hydrogen, halogen, alkyl, amino, alkylamino, alkoxy, mercapto, alkylthio, azido or cyano, e.g. B may be hydrogen, halogen, lower alkyl, amino, lower alkylamino, azido or cyano, or preferably B is hydrogen, halogen, lower alkyl, amino, lower alkylamino, alkoxy especially lower alkoxy, alkylthio especially lower alkylthio, azido or cyano, more particularly hydrogen, lower alkyl, amino, lower alkylamino, or azido. Especially preferred B groups include hydrogen, amino or azido. Other preferred B' groups include vinyl and ethynyl. Preferred pyrrolo[2,3-d]pyrimidine compounds of Formula I include those wherein F is halogen, amino, alkylamino, arylamino, aralkylamino, alkylthio, arylthio, aralkylthio, alkyl, aryl or aralkyl, more preferably amino or arylamino. Certain preferred compounds include F groups which comprise optionally substituted anilino. Examples of preferred pyrrolo [2,3-d]pyrimidine compounds include those noted as GP-1-448, GP-1-606, GP-1-608, GP-1-639, GP-1-683, GP-1-684, GP-1-691, GP-1-711, GP-1-714, and GP-1-718.

Preferred pyrrolo[2,3-d]pyrimidines of Formula III include those where G is hydrogen and A is oxygen. Preferably E is hydrogen or halogen, more preferably hydrogen. Preferred D groups include aryl. Preferred aryl groups include heterocyclic aryl groups and monocyclic carbocyclic aryl groups, especially optionally substitued phenyl. Preferred heterocyclic aryl groups include 2-furanyl, 2-thienyl and 3-thienyl.

(iii) <u>Preferred Purines</u>

Preferred purine compounds include those where G is hydrogen, halogen, lower alkyl or lower alkylthio, more preferably hydrogen. Preferred are compounds wherein A is oxygen. Preferred E groups include hydrogen, halogen or alkylthio, Another subset of preferred E groups is halogen, alkyl or alkylthio. Preferred are compounds wherein B is amino. Preferred F groups include halogen, amino, alkylamino, arylamino, aralkylamino, alkylthio, arylthio, alkyl, aryl or aralkyl, more preferable subsets of the preferred groups include (i) amino or arylamino, and (ii) alkyl, aryl and aralkyl. E is preferably hydrogen or halogen.

## <u>SYNTHESIS OF PREFERRED COMPOUNDS</u>

### A. General Synthetic Methods

The present invention is also directed to processes for preparing compounds of Formula I. The present invention provides a process for preparing a compound of formula I which comprises reacting a compound of the formula:

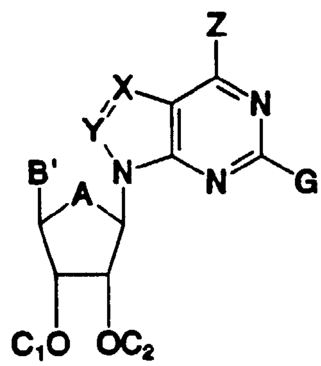

in which Z is a halogen and A, B', $C_1$, $C_2$, G, and X are as defined above or are in a protected form, and Y is -N=, with an appropriate nucleophilic agent and, if necessary, deprotecting the product to form a compound of formula I in which Y is -N=;

or which process comprises reacting a compound of the formula:

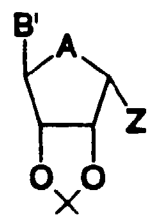

where A and B' are as defined above and Z is a halogen, with a compound of the formula:

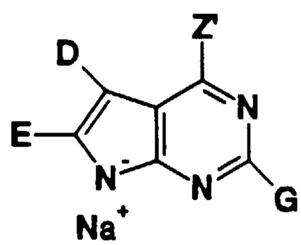

where E, D and G are as defined above and Z' is a halogen to give a compound of the formula:

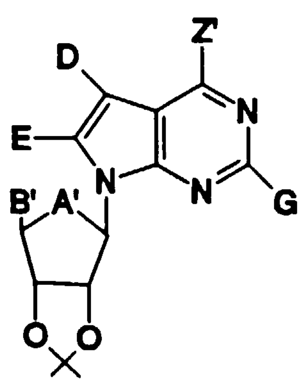

and deprotecting the compound to provide a compound of formula I in which Y is

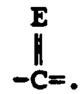

Disclosed herein are general synthetic routes for preparing variously substituted purine nucleosides or pyrrolo [2,3-d]pyrimidine nucleosides, including a novel and improved synthesis of 5'-deoxy-5-iodotubercidin; and pyrazolo [3,4-d]pyrimidine nucleosides of the present invention.

A process for preparing 5'-azido, 5'-amino and 5'-deoxy analogs of $N^6$- substituted purine ribosides is depicted in Figure 5. The protected azide (2a), prepared from 2',3'-O-isopropylideneinosine, is activated for nucleophilic attack at position six by chlorination with thionyl chloride/dimethylformamide. Other standard reagents may also be used to activate position six of 2 such as thionyl bromide, phosphorous oxychloride, triphenylphosphine dibromide-thiophenol-potassium permanganate or hexamethyldisilazane-ammonium sulfate. The chloride (3) or other activated intermediate (Br, $RSO_2$, $R_3SiO$, etc.) is then reacted with ammonia or an appropriate amine such as aniline, piperazine or indoline in solvents such as water, alcohols, THF and the like. The resulting protected $N^6$-substituted azide (4a) is deblocked using an aqueous acid such as 50% formic acid, to provide the $N^6$-substituted 5'-azido-5'-deoxyadenosine (5a). Reduction of the azide (5a) to the amine (6) is effected by catalytic hydrogenation with a catalyst such as platinum oxide, palladium on carbon and the like. For molecules containing other functional groups sensitive to hydrogenation, triphenylphosphine is used to selectively reduce the azide moiety to the amine. To prepare the N-acylamino (7a) and hydrocarbyloxycarbonylamino (7b) compounds, the azide 4a is reduced to the amine and treated with an acyl anhydride or acyl chloride or alkyl chloroformate and deblocked to give (7a) or (7b) respectively. Analogous processes are used to prepare the 2- and 8- substituted analogs beginning with appropriately substituted intermediates. An alternative synthesis of 5'-amino and 5'-hydrocarbylamino compounds comprises deblocking a 2',3'-isopropylidene-5'-tosylate with aqueous acid and then reacting the deblocked tosylate with ammonia or a lower hydrocarbylamine. Further description of these procedures is set forth in the Examples.

A similar process is used to prepare 5'-deoxy purine nucleosides. The appropriately substituted 5'-deoxy-2', 3'-O-isopropylideneinosine (2b) is chlorinated or activated using other reagents described above, aminated to 4b and subsequently deblocked to afford the 5'-deoxy nucleoside 5b.

The overall process for preparing 5'-modified pyrrolo[2,3-d]pyrimidine riboside compounds of Formula I, is depicted in Figure 8. A key step comprises the sodium salt glycosylation method (K. Ramasamy et al., Tetrahedron Letters, **1987,** 28:5107) using the anion of a substituted 4-chloropyrrolo[2,3-d]pyrimidine (18) and 1-chloro-2,3-O-isopropylidene-5-O-tert-butyldimethylsilyl-α-D-ribofuranoside (17). This method is also suitable for direct preparation of ribofuranosides wherein the 5-hydroxy group has been replaced with substituents such as hydrogen or azido or extended with additional carbons (Figure 8). The azide sugars further provide for facile synthesis of 5'-amino nucleosides by reductions of the azide function after ribosylation. An alternative to the sodium salt glycosylation method is a solid-liquid phase transfer reaction using the same substrates and potassium hydroxide in place of sodium hydride as described by Rosemeyer H., and Seela, F, Helvetica Chimica Acta, **1988**, 71:1573.

Preparation of the 5-substituted ribose analogs and homologs is outlined in Figures 6 and 7. The 5-substituted-5-deoxy ribose analogs 10 are prepared by tosylation of the protected ribose 8, displacement of the tosylate by appropriate nucleophiles and subsequent deblocking (Synder, J.; Serianni, A.; Carbohydrate Res., 1987, 163: 169). The ribose homologs (Figure 7) are prepared by oxidation of the protected ribose (8) to the aldehyde (11) (Moorman, A.; Borchaedt, R.; Nucleic Acid Chemistry-Part III, Townsend, L.; Tipson, R.; John Wiley & Sons, 1986). The aldehyde is homologated via the appropriate Wittig reagent to give the key intermediate protected vinyl sugar (12). The protected intermediate is deblocked to give the vinyl ribose homolog (16a) or reduced to (13) and then deblocked to give the saturated deoxy analog (16b). Alternatively, the vinylated intermediate (12) is hydroborated and oxidized affording the protected homologous ribose (14a) which is deblocked to the ribose homolog or converted to the azide (14b) via tosylation and displacement with azide. Deblocking of (14b) then affords the homologous azido ribose (16d). The protected 5-aldehyde (11) was also methylated to ultimately afford 6-deoxy-D-allofuranose (16e). The various 5- substituted riboses are then converted to the corresponding 2,3-0-isopropylidine ketals (Figure 8) which are chlorinated stereoselectively to 5-modified 1-chloro-α-D-ribofuranosides (17) using carbon tetrachloride and hexamethylphosphorous triamide (Wilcox, C.; Otaski, R.; Tetrahedron Lett., **1986,** 27:1011).

The preparation of various substituted 4-chloro-pyrrolo[2,3-d]pyrimidines is described in the Examples. The initial products from the ribosylation reactions, ribosyl protected 5-substituted-4-chloropyrrolo[2,3-d]pyrimidine nucleosides and the corresponding deblocked compounds are versatile intermediates and comprise an aspect of the present invention. As examples, the 4-chloro substituent of 19 can be displaced by sulfur (such as thiourea or mercaptide anions) leading to thionated and hydrocarbylthio compounds. More importantly, displacement of the 4-chloro substituent by ammonia or amines leads to 4-amino- and 4-arylaminopyrrolo[2,3-d]pyrimidine nucleosides. As further example, and an aspect of the present invention, an improved synthesis of the adenosine kinase inhibitor, 5'deoxy-5-iodotubercidin is described. According to this novel method, coupling of the sodium salt of 4-chloro-5-iodopyrrolo[2,3-d]pyrimidine with 1-chloro-5-deoxy-2,3-isopropylidene-α-D-ribofuranoside (17, B'=$CH_3$) in acetonitrile gives the protected 4-chloro compound. Amination of this product with ammonia, followed by deblocking affords 5'-deoxy-5-iodotubercidin.

Especially preferred intermediates are protected pyrrolo[2,3-d]pyrimidine nucleosides having a 4-chloro and a 5-iodo or bromo substituent.

Another aspect of the present invention is directed to the use of arylboronic acids to prepare 4- and 5-arylated pyrrolo[2,3-d]pyrimidine bases and nucleosides from the corresponding 4- and 5-halogenated compounds. Thus, a halogenated nucleoside such as 19 or the corresponding base was heated with an arylboronic acid and a palladium-phosphine catalyst such as palladium tetrakis(triphenylphosphine) to prepare the analogous arylated compound by displacement of halogen. Various 4- and 5- arylated pyrrolo[2,3-d]pyrimidines also can be prepared using arylstannyl compounds in place of the arylboronic acids (Flynn, B.; Macolino, B.; Crisp, G. Nucleosides & Nucleosides, **1991**, 10: 763). Synthesis of 5arylpyrrolo[2,3-d]pyrimidines can also be effected by condensation of arylamino ketones and malononitrile to arylated pyrroles and subsequent ring closure to 5-acylpyrrolo[2,3-d]pyrimidines. (Taylor, E.; Hendess, R., J. Am. Chem. Soc., **1965,** 87:1995).

The various above-mentioned products of ribosylation reactions may be deblocked at appropriate points with aqueous acids such as 50% formic acid or trifluoroacetic acid. Preparation of 5'-amino compounds consists of reducing an appropriate azide. The 5'-amides and urethanes are prepared analogously to those described previously for purine analogs. Further description of these procedures is set forth in the Examples.

Still another aspect of this invention is the preparation of 5'-substituted pyrazolo[3,4-d]pyrimidine ribosides of Formula I as depicted in Figure 9. Accordingly, a substituted pyrazolo[3,4-d]pyrimidine is ribosylated with an esterified 5-hydroxy, 5-azido or 5-deoxyribofuranoside in the presence of a Lewis acid such as boron trifluoride (Cottam, H., Petrie, C.; McKernan, P.; Goebel, R.; Dalley, N.; Davidson, R.; Robins, R.; Revankar, G.; J. Med. Chem., **1984,** 27: 1120). The 5-substituted sugar is prepared by esterification of the deblocked sugar 10a to 10c or 16a to 16e (See Figures 6 and 7). Suitable esters include the acetate, benzoate, toluate, anisoate and the like. The substituted pyrazolo [3,4-d]pyrimidine base (22) may be prepared by a variety of procedures as illustrated in the Examples. Two general routes to the compounds of the present invention are described below.

The first general route comprises coupling an esterified ribose (21), prepared from 10 or 16, with a 3-substituted pyrazolo[3,4-d]pyrimidin-4-one. After ribosylation the pyrimidone riboside (24a) may be activated by chlorination with thionyl chloride/dimethylformamide or other reagents previously described and then reacted with ammonia or an amine to provide a variety of substituted 5'-modified $N^4$-substituted-amino-pyrazolo[3,4-d]pyrimidine nucleosides (24b). Examples of this aspect of the invention, 3-iodopyrazolo[3,4-d]pyrimidone nucleosides, are prepared by nonaqueous diazotization-iodination of the 3-amino compounds using a nitrite ester such as isoamyl nitrite and methylene iodide. Previous attempts to diazotize the 3-aminopyrazolo[3,4-d]pyrimidones using aqueous nitrous acid gave only N-nitrosated pyrazolo[3,4-d]pyrimidin-3,4-diones (Cottam, H.; Petrie, C.; McKernan, P.; Goebel, R.; Dalley, N.; Davidson, R.; Robins, R.; Revankar, G.; J. Med. Chem., **1984,** 27:1119). Further modifications of 23 or 24 include reduction of the 5'-azido moiety to afford the 5'-amino compounds or the 5'-amides and urethanes as described in Figure 5. Ester prodrugs ($C_1$ and $C_2$) of various 5'-amino nucleosides are prepared by reduction of the 5'-azide esters (23) using previously described reagents.

Various C-4 alkylated pyrazolo[3,4-d]pyrimidine nucleosides are prepared by reaction of the above mentioned suitably protected 4-chloropyrazolo[3,4-d]pyrimidine nucleosides with carbanion nucleophiles. A specific catalyst for this alkylation reaction was found to be trimethylamine; these reactions either do not occur or proceed very slowly and in poor yield in the absence of trimethylamine. Suitable carbanions include those derived from diethyl malonate, ethyl cyanoacetate, malononitrile, nitromethane, cyanide salts and the like. This procedure is also used to prepare C-6 alkylated purine ribosides. The initial C-alkylated products were deblocked and optionally further modified by hydrolysis and decarboxylation to afford the desired products.

An alternative process for synthesis of 5'-azido- and 5'-amino-5'-deoxypyrazolo[3,4-d]pyrimidine ribosides is also described. Accordingly, a substituted allopurinol riboside (24a) is protected by conversion to the 2',3'-isopropylidene derivative, tosylated and reacted with sodium azide in DMSO or DMF to form the azide. Activation of position four by chlorination with thionyl chloride/dimethylformamide or other reagents as described, followed by displacement of the activating group by ammonia or an amine results in a protected 5'-azido-5'-deoxy riboside. The azide is deblocked to afford (24b, B=$N_3$) ahd subsequently reduced to the 5'-amino riboside using the previously described procedures.

The second general route for preparation of substituted pyrazolo[3,4-d]pyrimidine nucleosides comprises coupling the esterified ribose (21) with various substituted 4-amino or 4-hydrocarbylaminopyrazolo[3,4-d]pyrimidines. The resulting products are then further modified or deblocked to afford the desired compounds. The utility of this procedure is demonstrated in the Examples, by the preparation of 3-phenyl-4-(phenylamino)pyrazolo[3,4-d]pyrimidine 5'-modified

ribosides from 3-phenyl-4-(phenylamino)pyrazolo[3,4-d]pyrimidine and various 5'-modified sugars. In another aspect of the present invention, halogenated pyrazolo[3,4]pyrimidine ribosides can be arylated using arylboronic acids and palladium catalysts as described for the pyrrolo[2,3-d]pyrimidines. Alternatively, the base can be boronated and then coupled with an aryl halide. Further description of these procedures is set forth in the Examples.

B. Preferred Methods of Synthesis

According to another aspect of the present invention, certain preferred methods of preparing the adenosine kinase inhibiting compounds of Formula I are provided.

One preferred method of the present invention is a novel procedure for preparing C-6 alkylated purine nucleosides and C-4 alkylated pyrazolo[3,4-d]pyrimidine nucleosides from the 6-chloropurine and 4-chloropyrazolo[3,4-d] pyrimidine nucleosides, respectively, using various carbanions (enolates, cyanide anion, etc.) and trimethylamine as a specific catalyst. Previous methodology for C-alkylation of 6-chloropurines consisted of a multistep route involving alkylthiolation and oxidation to a. sulfone followed by nucleophilic displacement with a carbanion (Yame, A.; Matsuda, A.; Veda, T.; Chem. Pharm. Bull. (Jap.), 1980, 28:150). This multistep route can be accomplished in one step using the specific catalyst trimethylamine which reacts to form a quaternary salt and in turn is displaced by a carbanion in situ, regenerating trimethylamine. The reactions are specifically catalyzed by unhindered trialkylamines.

Another preferred method of the present invention is a process for preparing arylated bases and nucleosides by reaction of a halogenated pyrrolo[2,3-d]pyrimidine or pyrazolo[3,4-d]pyrimidine with an aryl boronic acid in the presence of a palladium-phosphine catalyst. In this process, the halogen atom of a brominated or preferably, iodinated pyrrolo[2,3-d]pyrimidine or pyrazolo[3,4-d]pyrimidine base or nucleoside, is replaced by an aryl moiety such as phenyl, substituted phenyl or a heteroaryl moiety such as furanyl. A catalyst consisting of a metal such as palladium, complexed to an arylphosphine such as triphenylphosphine must be present as well as a base such as sodium carbonate. The resulting arylated nucleosides are important examples of the present invention and this method is shorter and more versatile than alternative syntheses of arylated nucleosides.

Still another preferred method of the present invention is a process for preparing the previously unknown 3-iodo- and 3-chloropyrazolo[3,4-d]pyrimidine nucleoside by nonaqueous diazotization of 3-aminopyrazolo[3,4-d]pyrimidine nucleosides. According to this invention, a suitably substituted 3-aminopyrazolo[3,4-d]pyrimidine nucleoside is diazotized by heating with an alkyl nitrite such as isoamyl nitrite in the presence of an iodine source (such as methylene iodide) resulting in replacement of the 3-amino moiety with an iodine atom. Alternatively, methylene iodide can be replaced by a chlorine source such as carbon tetrachloride resulting in replacement of the amino moiety by a chlorine atom. A previously reported attempt to effect replacement of the amino moiety in a 3-aminopyrazolo[3,4-d]pyrimidine riboside with other moieties using nitrous acid resulted only in replacement of the amino moiety by a hydroxyl group. The resulting 3-chloro- and particularly 3-iodopyrazolo[3,4-d]pyrimidine nucleoside are an important subject of adenosine kinase inhibitors disclosed in the present invention.

The dimers can be made in the same way as the above monomers. When the group F has two amino groups a dimer can form (see e.g. Examples 10, 19, 103, 104, 105, 107 and 185 hereafter).

C. Preferred Intermediates

According to a further aspect of the present invention, certain novel intermediates are provided which are useful in the synthesis of the adenosine kinase inhibitors of the present invention.

(i) Intermediates for Pyrrolo[2,3-d]pyrimidines

Certain intermediates useful in the preparation of certain preferred adenosine kinase inhibitors which comprise substituted pyrrolo[2,3-d]pyrimidine nucleosides include compounds of the formula:

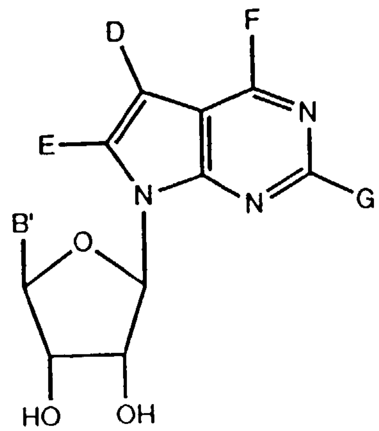

(VI)

wherein B' is lower alkyl or 1 to 3 carbon atoms optionally substituted with azido or hydroxy, or lower alkenyl of 1 to 3 carbon atoms; D is bromo or iodo, E is hydrogen, F is chloro, mercapto, arylamino and G is hydrogen. Certain especially preferred intermediates are set forth in Figure 10.

These preferred intermediates include the following compounds:
5-Bromo-4-chloro-7-(5-deoxy-1-β-D-ribofuranosyl)pyrrolo[2,3-d]pyrimidine;
4-Chloro-5-iodo-7-(5-deoxy-1-β-D-ribofuranosyl)pyrrolo-[2,3-d]pyrimidine;
5-Iodo-7-(5-deoxy-1-β-D-ribofuranosyl)pyrrolo[2,3-d]-pyrimidin-4(3H)-thione;
5-Bromo-4-chloro-7-(5,6-dideoxy-1)-β-D-allofuranosyl)pyrrolo[2,3-d]pyrimidine;
4-Chloro-5-iodo-7-(5,6-dideoxy-1-β-D-allofuranosyl)pyrrolo-[2,3-d]pyrimidine;
5-Bromo-4-chloro-7-(5,6-dideoxy-5,6-didehydro-1-β-D-allofuranosyl)pyrrolo[2,3-d]pyrimidine;
4-Chloro-5-iodo-7-(5,6-dideoxy-5,6-didehydro-1-β-D-allofuranosyl)pyrrolo[2,3-d]pyrimidine;
4-Chloro-5-iodo-7-(5-azido-5-deoxy-1-β-D-ribofuranosyl)pyrrolo[2,3-d]pyrimidine;
5-Bromo-4-chloro-7-(5-azido-5-deoxy-1-β-D-ribofuranosyl)pyrrolo[2,3-d]pyrimidine;
4-Chloro-5-iodo-7-(6-azido-5,6-dideoxy-1-β-D-allofuranosyl)pyrrolo[2,3-d]pyrimidine;
5-Bromo-4-arylamino-7-(5-deoxy-1-β-D-ribofuranosyl)pyrrolo[2,3-d]pyrimidine;
5-Iodo-4-arylamino-7-(5-deoxy-1-β-D-ribofuranosyl)pyrrolo[2,3-d]pyrimidine;
5-Iodo-4-arylamino-7-(1-β-D-ribofuranosyl)pyrrolo[2,3-d]pyrimidine; and
5-Bromo-4-arylamino-7-(1-β-D-ribofuranosyl)pyrrolo[2,3-d]pyrimidine.

In addition to being useful in the preparation of certain preferred adenosine kinase inhibitors, certain of these preferred intermediates exhibit activity as adenosine kinase inhibitors themselves.

(ii) Intermediates for Pyrazolo[3,4-d]pyrimidines

Certain intermediates useful in the preparation of certain preferred adenosine kinase inhibitor compounds comprise substituted pyrazolo[3,4-d]pyrimidines of the formula:

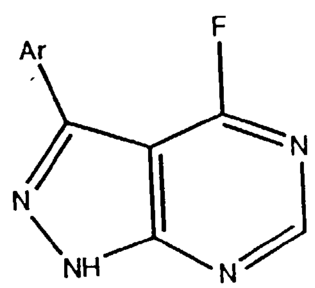

wherein Ar is an aryl group and F is halogen, preferably chloro. Preferred aryl groups include heterocyclic aryl groups and monocyclic carbocyclic aryl groups, including optionally substituted phenyl groups. These preferred intermediates

include the following compounds:
4-chloro-3-phenylpyrazolo[3,4-d]pyrimidine;
4-chloro-3-(2-thienyl)pyrazolo[3,4-d]pyrimidine;
4-chloro-3-(4-methoxyphenyl)pyrazolo[3,4-d]pyrimidine; and
4-chloro-3-(4-chlorophenyl)pyrazolo[3,4-d]pyrimidine.

## UTILITY

According to the present invention adenosine kinase inhibitors e.g. orally bioavailable pyrrolo [2,3-d]-pyrimidine riboside analogs such as 5'-deoxy-5-iodo tubericidin may be used in the treatment of a variety of clinical situations where increasing local levels of adenosine are beneficial i.e. increasing adenosine at a specific locus such as the heart or brain, particularly if the compound is capable of penetrating the central nervous system or blood-brain barrier. In particular, these compounds may be used in treating cardiovascular disorders in which injury or dysfunction is caused by ischemia and/or reperfusion (following a period of ischemia). These include (1) heart attack, a situation that arises from obstruction of one or more of the coronary arteries supplying blood to the heart muscle, and which, if prolonged, leads to irreversible tissue damage; (2) angina pectoris, a clinical condition in which the blood supply to the heart is sufficient to meet the normal needs of the heart but insufficient when the needs of the heart increase (e.g. during exercise), and/or when the blood supply becomes more limited (e.g. during coronary artery spasm); (3) unstable angina associated with pain at rest; and (4) silent ischemia. In each of these conditions, treatment adenosine, e.g. around cells whose ratio of rate of synthesis of adenosine triphosphate/rate of breakdown of adenosine triphosphate is undesirably low, and thereby blood flow to the ischemic tissue would be increased and tissue damage reduced and function improved. Therefore, according to the present invention adenosine kinase inhibitors may also be used to treat or prevent congestive heart failure.

In advanced coronary artery disease or persistent chest pain at rest, a number of clinical procedures are currently used to improve blood supply to the heart. These include percutaneous transluminal coronary angioplasty (PTCA), percutaneous transluminal directional coronary atherectomy, laser atherectomy, intravascular stents and coronary artery bypass graft surgery. The compounds of this invention will also be useful as adjunctive therapies to these techniques. Other clinical settings that involve ischemia would also be ameliorated by agents effecting regional blood flow including organ transplantation, skin flap grafting and other reconstructive surgery, peripheral vascular disease, endotoxemia, hemorrhagic shock, pulmonary emboli, pulmonary injury secondary to burns (thermal injury) or septicemia, pulmonary hypertension, microembolization, glomerulonephritis or progressive glomerulosclerosis, atherosclerosis, myocarditis, vasculitis, cardiomyopathies, intestinal ischemia, peripheral vascular disease, transient ischemic attacks, stroke and cardiopulmonary arrest. Adenosine kinase inhibitors will enhance protection afforded by preconditioning a tissue with a brief period of ischemia, before a more prolonged period of ischemia.

Thrombolytic therapy has been limited by a number of factors including the resistance of some thrombi to lysis, delays in reperfusion, and reocclusion following successful thrombolysis. These limitations are believed to be mediated, in part, by platelet aggregation (Born and Cross J. Physiol., **1963,** 166:29-30) and, since adenosine inhibits platelet aggregation in addition to its other effects on preventing ischemic injury, use of these adenosine kinase inhibitors may comprise a useful adjunctive therapy for conditions where thrombosis or emboli are prevalent. For example they may be useful in thrombolytic therapy or for the treatment or prevention of thrombotic diseases such as myocardial infarction, stroke, angina, deep vein thrombosis, transient ischemic attacks, and pulmonary embolus.

Adenosine has been reported to be an endogenous modulator of inflammation by virtue of its effects on stimulated granulocyte function (Cronstein et al., J. Clin. Invest., **1986,** 78:760-770) and on macrophage, lymphocyte and platelet function. The compounds of this invention may therefore be used in treating conditions in which inflammatory processes are prevalent such as arthritis, rheumatoid arthritis, osteoarthritis, autoimmune disease, adult respiratory distress syndrome (ARDS), inflammatory bowel disease, necrotizing enterocolitis, chronic obstructive pulmonary disease (COPD), psoriasis, conjunctivitis, iridocyditis, myositis, cerebritis, meningitis, dermatitis, renal inflammation, ischemia, reperfusion injury, peripheral vascular disease, vasculitis, atherosclerosis and other inflammatory disorders. Adenosine receptor agonists have been reported to be beneficial in an experimental model of inflammation. (Schrier, et al., J. Immunol., **1990**, 145:1874-1879).

Stroke and central nervous system ("CNS") trauma are conditions where tissue injury, e.g. damage to neural tissue such as that of the brain or spinal chord, results from reduced blood supply to the CNS and are thus amenable to an intervention that provides increased levels of adenosine to the compromised tissue. It is reported that a significant component of the neurodegeneration resulting from stroke or CNS trauma or neurodegenerative diseases is caused by increased excitatory amino acid release and sensitivity, which results in neurons being stimulated to death. In addition to its vasodilatory properties, adenosine has been reported to inhibit excitatory amino acid release (Burke and Nadler J. Neurochem., **1988,** 51:1541) and excitatory amino acid responsiveness. The compounds of this invention, which increase adenosine levels, may also be used in the treatment of conditions where release of or sensitivity to

excitatory amino acids is implicated such as Parkinson's disease, Amyotrophic Lateral Sclerosis, Huntington's chorea or Alzheimer's disease (Maragos et al., Trends Neurosci., **1987,** 10:65 and Sonsella et al. Science, **1989,** 243:398). These studies, together with results from experimental models of memory (Harris et al. Brain Res., **1984,** 323:132) suggest additional utilities of these compounds in the treatment of disorders related to the effects of the aging process on CNS function such as Alzheimer's disease. Other studies have also linked excitatory amino acids with the pathophysiology of schizophrenia. (Komhuber and Fischer, Neurosci. Lett., **1982**, 34:32; Kim et al. Eur. Neurol., **1983,** 22: 367). This suggests that adenosine kinase inhibitors may be useful in treating schizophrenia.

These adenosine kinase inhibitors may also be useful in reducing anxiety, as skeletal muscle relaxants and in preventing skeletal muscle spasm.

Adenosine has been proposed to serve as a natural anticonvulsant (Lee et al., Brain Res., **1984,** 321:160-164; Dunwiddie, Int. Rev. Neurobiol., **1985**, 27:63-139). Agents that enhance adenosine levels may be used in the treatment of seizure disorders. Adenosine kinase inhibitors may be used in the treatment of patients with seizures or epilepsy or who might have chronic low or insufficient adenosine levels or might benefit from increased adenosine such as those suffering from autism, cerebral palsy, insomnia or other neuropsychiatric symptoms. Other excitatory neuromuscular tissues such as smooth muscle and cardiac muscle may be treated using these adenosine kinase inhibitors. In particular, these adenosine kinase inhibitors may be used to decrease contraction in smooth muscle such as in the gastrointestinal tract, or in vascular tissue such as an artery to prevent vasospasm which may limit blood supply to a tissue. Thus, these adenosine kinase inhibitors may be used to treat conditions such as Buerger's disease, Raynaud's disease, thromboangiitis obliterans, angina, unstable angina, silent ischemia, or transient ischemic attacks. Other conditions suitable for such therapy include cardiac arrhythmias (including supraventricular tachycardia), irritable bowel syndrome, and impotence.

To assist in understanding the present invention and especially its properties and utilities, the results of a series of experiments are also included. These experiments demonstrated that a number of compounds of Formula I were potent inhibitors of a purified cardiac adenosine kinase with $IC_{50}$'s of less than 1 μM. Moreover, we have shown that these compounds are specific inhibitors of adenosine kinase with low affinity at the $A_1$ adenosine receptor and no significant adenosine deaminase (ADA) inhibition (Example A). We have demonstrated that a number of these compounds are also inhibitors of adenosine kinase in intact cells (Example B). These compounds include pyrrolo[2,3-d] pyrimidine nucleosides modified at the 5'-position or at other positions such that it is less likely to serve as a substrate for phosphorylation enzymes and that, in contrast to 5-iodotubercidin (GP-1-202), these compounds are unlikely to be phosphorylated at the 5'-position, incorporated into nucleotides or DNA, which may cause toxicity to cells or animals. We have demonstrated that inhibition of the cardiac adenosine kinase was achieved in vivo following systemic administration or, in some cases, oral administration of these compounds.

We have demonstrated the ability of these compounds to reduce damage resulting from ischemia and/or reperfusion in an experimental isolated heart model as shown in Example C. A more detailed study demonstrates that functional benefit may be achieved without vasodilatory effects on basal coronary flow which reflects increases in non-ischemic flow (indicative of the potential for coronary steal) or on heart rate. This result was unexpected given the previous descriptions by Newby et al (Biochem. J. **1983**, 214:317-323) and Schrader (Regulatory Functions of Adenosine, Berne et al. eds., pp. 133-156 **1983**) of the increases in basal coronary flow caused by adenosine kinase inhibitors. Selected compounds, such as GP-1-238, were also evaluated to determine the potential for toxic hemodynamic effects or hypothermia associated with administration of adenosine kinase inhibitors. GP-1-238 is a compound of formula IV in which B is amino, E is hydrogen, F is amino and G is hydrogen. No effects were observed in conscious animals on blood pressure, heart rate or temperature with doses of inhibitor greatly in excess of that required to inhibit the cardiac adenosine kinase (Example D).

Further experiments demonstrated that the adenosine kinase inhibitor GP-1-515 is beneficial in an experimental model of stable angina in dogs. In this study intravenous infusion of the compound attenuated the decline in function associated with repeated episodes of pacing-induced ischemia (Example E). The potential antithrombotic activity of adenosine kinase inhibitors is supported by the ability of GP-1-515 to abolish cyclic flow reductions (CFR's) in 3 out of 8 dogs examined in the Folts model of coronary artery thrombosis (Example F). These results support potential utility of these compounds in thrombotic diseases, such as angina and myocardial infarction.

In other experimental models, the ability of selected adenosine kinase inhibitors (GP-1-272 and GP-1-456) to inhibit neutrophil adherence to endothelial cells, an inflammatory response mediated at the cellular level was evaluated (Example G). Certain adenosine kinase inhibitors were found to exhibit anti-inflammatory activity in animal models of inflammation. For example compounds may show at least about 20% reduction of total mean hind paw edema at a dose of 300 mg/kg/day or less in an in vivo inflammation test method (see Handbook of Animal Models for the Rheumatic Diseases, Robert Greenwald and Herbert Diamond, EDS (CRC Press, 1988). The ability of adenosine kinase inhibitors (GB-1-515, GP-1-547) to attenuate contraction in the isolated ileum (Example H) supports the utility of these compounds in gastrointestinal disorders especially irritable bowel syndrome. In the central nervous system (CNS), the potent effects of selected adenosine kinase inhibitors (such as GP-1-456, GP-1-560) in attenuating chemical

and electroshock induced seizures in experimental animal models demonstrates that these compounds will be useful as anticonvulsants in epilepsy (Example I), as well as in other CNS diseases treatable by local increases in adenosine levels.

Compound GB-1-560 is a pyrazolapyrimidine of formula II in which B is hydrogen, D is iodine, F is amino and G is hydrogen.

## FORMULATIONS

Compounds of the invention are administered to the affected tissue at the rate of from 0.1 to 200 nmole/min/kg, preferably from 1 to 20 nmol/min/kg. Such rates are easily maintained when these compounds are intravenously administered as discussed below. When other methods are used (e.g., oral administration), use of time-release preparations to control the rate of release of the active ingredient may be preferred. These compounds are administered in a dose of about 0.01 mg/kg/day to about 100 mg/kg/day, preferably from about 0.1 mg/kg/day to about 10 mg/kg/day.

For the purposes of this invention, the compounds of the invention may be administered by a variety of means including orally, parenterally, by inhalation spray, topically, or rectally in formulations containing conventional non-toxic pharmaceutically acceptable carriers, adjuvants and vehicles. The term parenteral as used herein includes subcutaneous, intravenous, intramuscular, and intraarterial injections with a variety of infusion techniques. Intraarterial and intravenous injection as used herein includes administration through catheters. Preferred for certain indications are methods of administration which allow rapid access to the tissue or organ being treated, such as intravenous injections for the treatment of myocardial infarction. When an organ outside a body is being treated, perfusion is preferred.

Pharmaceutical compositions containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including those from the group consisting of sweetening agents, flavoring agents, coloring agents and preserving agents, in order to provide a palatable preparation. Tablets containing the active ingredient in admixture with non-toxic pharmaceutically acceptable excipient which are suitable for manufacture of tablets are acceptable. These excipients may be, for example, inert diluents, such as calcium carbonate, sodium carbonate, lactose, calcium phosphate or sodium phosphate; granulating and disintegrating agents, such as maize starch, or alginic acid; binding agents, such as starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

Formulations for oral use may be also presented as hard gelatin capsules wherein the active ingredient is mixed with an inert solid diluent, for example calcium phosphate or kaolin; or as soft gelatin capsules wherein the active ingredient is mixed with water or an oil medium, such as peanut oil, liquid paraffin or olive oil.

Aqueous suspensions of the invention contain the active materials in admixture with excipients suitable for the manufacture of aqueous suspensions. Such excipients include a suspending agent, such as sodium carboxymethylcellulose, methylcellulose, hydroxypropylmethylcelluose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadeaethyleneoxycetanol), a condensation product of ethylene oxide with a partial ester derived from a fatty acid and a hexitol anhydride (e.g., polyoxyethylene sorbitan mono-oleate). The aqueous suspension may also contain one or more preservative such as ethyl of n-propyl p-hydroxybenzoate, one or more coloring agent, one or more flavoring agent and one or more sweetening agent, such as sucrose or saccharin.

Oil suspensions may be formulated by suspending the active ingredient in a vegetable oil, such as arachis oil, olive oil, sesame oil or coconut oil, or in a mineral oil such as liquid paraffin. The oral suspensions may contain a thickening agent, such as beeswax, hard paraffin or cetyl alcohol. Sweetening agents, such as those set forth above, and flavoring agents may be added to provide a palable oral preparation. These compositions may be preserved by the addition of an antioxidant such as ascorbic acid.

Dispersible powders and granules of the invention suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.

The pharmaceutical compositions of the invention may also be in the form of oil-in-water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsion may also contain sweetening and flavoring agents.

Syrups and elixirs may be formulated with sweetening agents, such as glycerol, sorbitol or sucrose. Such formulations may also contain a demulcent, a preservative, a flavoring or a coloring agent.

The pharmaceutical compositions of the invention may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, such as a solution in 1,3-butanediol or prepared as a lyophylized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables.

The amount of active ingredient that may be combined with the carrier material to produce a single dosage form will vary depending upon the host treated and the particular mode of administration. For example, a time-release formulation intended for oral administration to humans may contain 20 to 200 moles of active material compounded with an appropriate and convenient amount of carrier material which may vary from about 5 to about 95% of the total compositions. It is preferred that pharmaceutical composition be prepared which provides easily measurable amounts for administration. For example, an aqueous solution intended for intravenous infusion should contain from about 20 to about 50 moles of the active ingredient per milliliter of solution in order that infusion of a suitable volume at a rate of about 30 ml/hr can occur.

As noted above, formations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be administered as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g., povidone, gelatin, hydroxypropylmethyl cellulose), lubricant, inert diluent, preservative, disintegrant (e.g., sodium starch glycolate, cross-linked povidone, cross-linked sodium carboxymethyl cellulose) surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach. This is particularly advantageous with the compounds of formula (I) as such compounds are susceptible to acid hydrolysis.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the ddPN ingredient such carriers as are known in the art to be appropriate.

Formations suitable for parenteral administration include aqueous and non-aqueous isotonic sterile injection solutions which may contain anti-oxidants, buffers, bacteriostats and solutes which render the formation isotonic with the blood of the intended recipient; and aqueous and non-aqueous sterile suspensions which may include suspending agents and thickening agents. The formulations may be presented in unit-dose or multidose sealed containers, for example, ampoules and vials, and may be sorted in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, or an appropriate fraction thereof, of an adenosine kinase inhibitor compound.

It will be understood, however, that the specific dose level for any particular patient will depend on a variety of factors including the activity of the specific compound employed; the age, body weight, general health, sex and diet of the individual being treated; the time and route of administration; the rate of excretion; other drugs which have previously been administered; and the severity of the particular disease undergoing therapy, as is well understood by

those skilled in the art.

Examples of use of the method of the invention includes the following. It will be understood that these examples are exemplary and that the method of the invention is not limited solely to these examples.

The method may be used following thrombolysis for coronary occlusion. The compound would be given as a sterile injectable preparation with water or isotonic sodium chloride as the solvent. The solution can be administered intravenously or directly into the coronary artery at the time of left heart catheterization or into a carotid artery. The rate of administration could vary from 1 to 20 nmole/min/kg with, for example, an infusion volume of 30 ml/hr. Duration of therapy would typically be about 96 hours.

Angina and early myocardial infarcts can be treated by intravenous administration using a sterile injectable preparation using the rates discussed above.

Capsules comprising adenosine kinase inhibitors suitable for oral administration according to the methods of the present invention may be prepared as follows: (1) for a 10,000 capsule preparation: 1500g of adenosine kinase inhibitor is blended with other ingredients (as described above) and filled into capsules which are suitable for administration depending on dose, from about 4 capsules per day (1 per 6 hours) to about 8 capsules per day (2 capsules per 6 hours), to an adult human.

The compounds of this invention and their preparation can be understood further by the examples which illustrate some of the processes by which these compounds are prepared. These examples should not however be construed as specifically limiting the invention and variations of the invention, now known or later developed, are considered to fall within the scope of the present invention as herein after claimed.

## EXAMPLES

### EXAMPLE 1

Preparation of 5'-Azido-5'-deoxy 2', 3'-O-(1-methylethylidene) inosine

This material was prepared by tosylation of 2', 3'-(1-methylethylidene)inosine and subsequent reaction with sodium azide in DMSO as described by Hampton, A.; J. Org. Chem., 1968, 11:1220.

### EXAMPLE 2

Preparation of 9-[5-Azido-5-deoxy-2,3-O-(1-methylethylidene)-1-β-D-ribofuranosyl]-6-chloropurine

A solution of the azide (Example 1) (12.01 g, 0.036 mole) in dry $CH_2Cl_2$ (500ml) was added, during 1 hr, to a warm solution of $SOCl_2$ (8.1 ml, 0.11 moles) and DMF (4.05 ml, 0.05 moles) in $CH_2Cl_2$ (50 ml). The resulting solution was refluxed for 6 hrs while a continuous stream of argon gas was bubbled through the reaction mixture to remove HCl. The reaction was cooled and added to a cold, rapidly stirred aqueous solution of $KHCO_3$. After stirring for 15 minutes the layers were separated and the organic layer was washed with cold aqueous $K_2CO_3$, $H_2O$(2x), dried ($Na_2SO_4$) and concentrated under vacuum. The residue was redissolved in $CH_2Cl_2$ and filtered through a plug of $SiO_2$ gel. Evaporation of the filtrate under vacuum gave 11.3 g (89% yield) of the title compound as a pale yellow oil.

### EXAMPLE 3

General Procedure for the Preparation of $N^6$-Substituted-5'-azido-5'-deoxy-2',3'-O-(1-methylethylidene)adenosines

To a solution of chloride (Example 2) (1 mmole/5 ml) in EtOH or n-BuOH was added amine (1.3 equivalents) and $Et_3N$ (1.8 equivalents). The solution was heated to reflux for 12-24 hours under Ar until judged complete by TLC. The reaction mixture was evaporated under vacuum, dissolved in $CH_2Cl_2$ and washed with aq. $K_2CO_3$ and $H_2O$. The $CH_2Cl_2$ solution was dried ($Na_2SO_4$) concentrated and used directly in the next step or chromatographed on $SiO_2$ gel using $CH_2Cl_2$-MeOH mixtures.

### EXAMPLES 4 to 14

General Procedure for the Preparation of $N^6$-Substituted-5'-azido-5'deoxyadenosines

The $N^6$-substituted isopropylidene azide (Example 3) (1.0 g) was dissolved in $HCO_2H$ (10-20 ml) and diluted with an equal volume of $H_2O$. The reaction was followed by TLC. After disappearance of the starting material (12-48 hr), the reaction mixture was evaporated under vacuum, coevaporated with $H_2O$ (3x) then ETOH (2x). The residue

was crystallized from $H_2O$, alcohol or mixtures.
The compounds in Table I (Examples 4-14) were prepared by this procedure:

TABLE I

| GP1-# | Example | F | MP (°C) |
|---|---|---|---|
| 266 | 4 | ϕNH | 121-126° |
| --- | 5 | $CH_3NH$ | 180°(d) |
| 317 | 6 | 4-ClϕNH | 209-210° |
| 299 | 7 | $ϕCH_2CH_2NH$ | 144-146° |
| 337 | 8 | N-indolinyl | 157-159° |
| 346 | 9 | 4-$(HOCH_2CH_2)$ϕNH | 147-151° |
| --- | 10 | -$NH(CH_2)_{12}NH$-(dimer) | foam |
| 385 | 11 | N-indolyl[1] | 186-188° |
| 391 | 12 | N-(5-bromoindolinyl) | 194-196° |
| 421 | 13 | N-(5-methoxyindolinyl) | 184-185° |
| 557 | 14 | 1,4-piperazinyl | 198-204° |

1/ Prepared from indole and sodium hydride in DMF.

EXAMPLES 15-20

General Procedure for the Preparation of $N^6$-Substituted-5'-amino-5'-deoxyadenosines and Hydrochloride Salts

A solution of the azide in EtOH or MeOH containing 10% Pd-C (25-50% weight of azide) was hydrogenated on a Parr shaker at 25 psi for 4-8 hours. The mixture was filtered, the catalyst rinsed well with solvent and the filtrate evaporated. The residue was recrystallized to give the free base or converted to the salt. The hydrochloride salt was prepared by slurrying or dissolving the free base in a small volume of EtOH, adding dry ethanolic HCl to a pH of 4-6, warming the mixture and then chilling to crystalize out the salt (in some cases $Et_2O$ was added to precipitate the salt). The compounds in Table II (Examples 15-20) were prepared by this procedure:

TABLE II

| GP1-# | Example | F | M.P.°C (salt) |
|---|---|---|---|
| 272 | 15 | $CH_3NH$ | 170-172° ($HCO_2H$) |
| 286 | 16 | ϕNH | 169-173° (HCl) |
| 328 | 17 | $ϕCH_2CH_2NH$ | 130°(d) (HCl) |
| 345 | 18 | N-indolinyl | 202-203° (HCl) |
| 373 | 19 | -$HN(CH_2)_{12}NH$-(dimer) | 151-153° (HCl) |
| 565 | 20 | 1,4-piperazinyl | 140-145° (HCl) |

EXAMPLE 21

Preparation of 5'-deoxy-2',3'-0-(1-methylethylidene)inosine

A solution of 5'-deoxy-5'-iodo-2',3'-0-(1-methylethylidene)inosine (5.45 g, 0.013 mol) in 80 ml of methanol containing triethylamine (2.0g) and 10% palladium on charcoal (737 mg) was hydrogenated for 2 hr under 50 psi $H_2$. The reaction mixture was filtered and the filtrate concentrated and allowed to crystalize. The product was collected by filtration and dried under vacuum to give 2.45 g (82% yield) of the title compound.

EXAMPLE 22

Preparation of 6-Chloro-9-[5-deoxy-2,3-O-(1-methylethylidene)-1-β-D-ribofuranosyl]purine

A solution of the blocked 5'-deoxyinosine (1.4 g, 4.8 mmol), tetraethylammonium chloride (1.9 g, 11.5 mmol), diethylaniline (1.2 ml, 7.2 mmol) and phosphorous oxychloride (3.35 ml, 36 mmole) in $CH_3CN$ (24 ml) was refluxed for 10 minutes then evaporated. The residue was dissolved in $CH_2Cl_2$, washed with water, aqueous $KHCO_3$ solution, water

and dried ($Na_2SO_4$). The solution was concentrated and filtered through a plug of $SiO_2$ gel. The filtrate was evaporated to give 860 mg (65% yield) of title compound as a yellow oil.

EXAMPLE 23

General Procedure for Preparation of $N^6$-substituted 5'-deoxyadenosines

The above identified,compounds were prepared using the procedures described in Example 3 and Examples 4-14.

The compounds listed in Table III were prepared by this procedure.

TABLE III

| GPI-# | EXAMPLE | F | M.P.(°C) |
|---|---|---|---|
| 595 | 23 | 1,4-piperazinyl | 220-225° |

EXAMPLE 24

Preparation of 8-Bromo-2',3'-O-(1-methylidene)-5'-O-(4-methylbenzenesulfonyl)adenosine

The above-identified compound may be prepared as described: Ikshara, M.; Kaneko, M.; Sagi, M.; Tetrahedron, **1970,** 26:5757.

EXAMPLE 25

Preparation of $N^6$-Formyl-8-bromo-2',3'-O-(1-methylethylidene)-5'-O-(4-methylbenzenesulfonyl) adenosine

To acetic-formic anhydride (prepared by stirring 25 ml of acetic anhydride and 12.5 ml of formic acid for 15 minutes at 45°C) at 0°C, was added the tosylate (Example 24) (4.0 g, 7.30 mmol). The resulting solution was allowed to warm to 22°C and stirred for 48 hours. The reaction mixture was evaporated, chased 2x with toluene and the residue dissolved in $CHCl_3$ and filtered through a plug of silica gel. Evaporation of the filtrate and crystallization of the residue from ethanol gave 4.0 g (96%) of the title compound.

EXAMPLE 26

Preparation of 5'-Deoxy-5',8-diazido-2',3'-O-(1-methylethylidene)adenosine

To a hot (75°C), well stirred slurry of $NaN_3$ (2.23 g, 34.3 mmol) in dimethylsulfoxide (35 ml) was added the formyl tosylate (Example 25) (4.00 g, 6.9 mmoles). The reaction temperature was held at 75°C for one hour then cooled to 25°C. The mixture was poured into stirring $H_2O$ (90 ml), slurried for ten minutes, the solid was collected by filtration, rinsed with $H_2O$ (3x), cold ethanol and dried. The crude product was dissolved in $CHCl_3$, filtered through silica gel and the filtrate evaporated to give the $N^6$-formyl derivative 1.20 g; m.p. 107-110°C.

The $N^6$-formyl derivative was deformylated by slurrying in MeOH, adding saturated methanolic ammonia (80 ml) and warming until homogenous. After 15 minutes the solution was evaporated, the residue recrystallized from EtOH and dried to give the title compound; 0.900 g (60% yield); m.p. 166-168°C.

EXAMPLE 27

Preparation of 5'-Deoxy-5',8-diazidoadenosine

The isopropylidene diazide of Example 26 (1.00 g, 3.15 mmol) was deblocked as described under Example 4 and recrystallized from $H_2O$; 760 mg (85% yield); m.p. 128-130°.

EXAMPLE 28

Preparation of 5'-Deoxy-5'-8-diaminoadenosine Formate

The diazide of Example 27 (0.660 g, 2.0 mmol) was hydrogenated as described under Example 15 and recrystallized from abs. EtOH to give after drying, 400 mg (61% yield). This material was further purified by conversion to the formate salt ($HCO_2$/EtOH/$Et_2O$); m.p. 98°C(d).

EXAMPLE 29

Preparation of 5'-Deoxy-5'-formylaminoadenosine

To cold (5°C) acetic-formic anhydride (10 ml acetic anhydride and 5 ml formic acid) was added 5'-amino-5'-deoxy-2', 3'-O-(1-methylethylidene)adenosine (670 mg, 2.0 mmol). The solution was stirred for 24 hours then evaporated, and coevaporated with toluene (2x) then EtOH. The residual foam was dissolved in methanolic ammonia containing $CH_2Cl_2$ and stirred overnight. TLC ($SiO_2$ gel, 9:1 $CHCl_3$-MeOH) indicated the initial product was converted to a more polar product. The solution was evaporated, the residue dissolved in $CHCl_3$ with 3% MeOH and filtered through a plug of $SiO_2$ gel. Evaporation of the filtrate gave 520 mg of a white foam. This material (500 mg) was deblocked with $HCO_2H$ as described for Example 4 and recrystallized from EtOH-$H_2O$ to give the title compound: yield 0.35 g (55%); m.p. 212-213°C.

EXAMPLE 30

Preparation of 6-(N-Indolinyl)-9-[2,3-O-(1-methylethylidene) -1-β-D-ribofuranosyl]purine

A mixture of the isopropylidene of 6-chloropurine riboside (11.5 g, 0.035 mol), indoline (5.13 ml, 0.046 mole) and triethylamine (8.82 ml, 0.063 mole) in n-butanol (60 ml) was stirred and heated to reflux for 24 hours. The reaction was cooled and the solid collected by filtration, rinsed with EtOH and dried to give the title compound: 10.70 g (75% yield); m.p. 119-125°C.

EXAMPLE 31

Preparation of 6-(N-Indolinyl)-9-[2,3-O-(1-methylethylidene) -5-O-(4-methylbenzenesulfonyl)-1-β-D-ribofuranosyl)] purine

To a cold (0°C) solution of the alcohol (Example 30) (6.0 g, 0.015 mol) in dry pyridine (40 ml) was added with stirring, p-toluenesulfonyl chloride (6.96 g, 0.36 mol). The solution was sealed and stored at 0-10°C for 72 hours then poured with stirring into cold $H_2O$ (30 ml). The solid was collected by filtration and rinsed 3x with $H_2O$. After drying at 25°C under vacuum, the title compound was obtained: 6.85 g (83% yield); m.p. 195°C (d).

EXAMPLE 32

Preparation of 6-(N-Indolinyl)-9-[5-O-(4-methylbenzenesulfonyl)-1-β-D-ribofuranosyl)]purine

A slurry of the protected tosylate (Example 31) (5.80 g, 10.0 mmole) in hydrochloric acid (23 ml) and EtOH (255 ml) was heated until homogenous then refluxed for 15 minutes. The solution was cooled in an ice bath and neutralized with $KHCO_3$. The solid was collected by filtration, washed with $H_2O$, EtOH then MeOH. After drying, 3.38 g (65% yield) of the title compound were obtained; m.p. 112°C(d).

EXAMPLE 33

Preparation of 6-(N-Indolinyl)-9-(5-methylamino-5-deoxy-1-β-D-ribofuranosyl)purine Hydrochloride

To 40% aqueous methylamine (40 ml) was added the tosylate (Example 32) (2.0 g, 3.8 mmol) and sufficient MeOH to give a clear solution. The solution was stirred for one week then concentrated under vacuum. The residue was coevaporated 3x with MeOH then recrystallized from MeOH to give the free base, 0.310 g (21% yield). A portion of this material was converted (ethanolic HCl) to the hydrochloride salt, m.p. 170-172°.

EXAMPLE 34

Preparation of 4-Chloro-7H-pyrrolo[2,3-d]pyrimidine

The above-identified compound was prepared as described: Davoll, J.; J. Chem. Soc., **1960**, 131.

EXAMPLE 35

Preparation of 5-Bromo-4-chloro-7H-pyrrolo[2,3-d]pyrimidine

The above identified compound was prepared as described: Hinshaw, B.; Gerster, J.; Robins, R.; Townsend, L.; J. Heterocyclic Chem., **1969**, 215.

EXAMPLE 36

Preparation of 4-Chloro-5-iodo-7H-pyrrolo[2,3-d]pyrimidine

The above-identified compound was prepared as described: Pudlo, J.; Nassiri, M.; Kern, E.; Wartiny, L.; Drach, J.; Townsend, L.; J. Med. Chem., **1990**, 33, 1984.

EXAMPLE 37

Preparation of 4-Chloro-5-methyl-7H-pyrrolo[2,3-d]pyrimidine

The above-identified compounds was prepared as described: Pudlo, J.; Nassir, M.; Kern, E.; Wotring, L.; Drach, J.; Townsend, L.; J. Med. Chem., **1990,** 33, 1984.

EXAMPLE 38

Preparation of 4-Chloro-2-methylthio-7H-pyrrolo[2,3-d] pyrimidine

The above-identified compound was prepared as described: Noel, C., Robins, R.; J. Heterocyclic Chem., 1964, 1, 34.

EXAMPLE 39

Preparation of 2-Amino-4-chloro-7H-pyrrolo[2,3-d]pyrimidine

The above-identified compound was prepared as described: Pudlo, J.; Nassiri, M.; Kern, E.; Wotrlng, L.; Drach, J.; Townsend, L.; J. Med. Chem., **1990**, 33, 1984.

EXAMPLE 40

Preparation of 2-Amino-4-chloro-7H-pyrrolo[2,3-d]pyrimidine

The above-identified compound was prepared as described. Seela, F.; Stiker, H.; Driller, H.; Binding, N.; Liebigs Ann. Chem., **1987,** 15.

EXAMPLE 41

Preparation of 4-Chloro-5-methylthio-7H-pyrrolo[2,3-d]pyrimidine

A solution of 5-bromo-4-chloro-7H-pyrrolo[2,3-d]pyrimidine (Example 35) (2.53 g, 10 mmol) in anhydrous THF (30 mL) was cooled to -78°C under argon and a solution of n-butyl lithium (12 mL of 2.3 M solution, 25 mmol) was added at such a rate that the reaction temperature remained below -72°C. After the addition, the reaction mixture was stirred at -78°C for 45 min, a solution of methyl disulfide (0.95 ml, 10 mmol) in tetrahydrofuran (10 mL) was added over a period of 30 minutes maintaining the temperature below -72°C. The reaction mixture was stirred at -78°C for 2.5 hours then allowed to warm to room temperature. A saturated solution of ammonium chloride (40 mL) was added to the reaction with stirring. The organic layer was separated, the aqueous layer extracted with ethyl acetate (2x40 mL) and the combined organic extracts were dried ($MgSO_4$), filtered and evaporated to obtain a pale yellow solid which was crystallized from EtOH: yield 1.65 g; (70%) m.p. 166-167°C.

EXAMPLE 42

Preparation of 4-Chloro-5-cyano-7H-pyrrolo[2,3-d]pyrimidine

A 2.31 M solution of n-butyllithium in hexane (6.1 mL, 14.0 mmol) was added dropwise to a solution of 4-chloro-5-bromopyrrolopyrimidine (Example 35) (1.481 g, 6.37 mmol) in 65 mL THF at -78°C and the resulting light yellow suspension stirred at this temperature for 1 hour. A cold (-78°C) solution of p-tolylsulfonylcyanide (2.08 g, 11.5 mmol) in 35 mL THF was added dropwise via cannula and the resulting mixture stirred at this temperature for 1 hour. Aqueous $NH_4Cl$ was added and the resulting solution was diluted with 100 mL $CH_2Cl_2$. The organic layer was separated, washed with water, brine, dried ($MgSO_4$) and evaporated to provide a tan solid (1.3 g) which appeared to be a 1:1 mixture of the title nitrile and 4-chloropyrrolo-pyrimidine by $^1H$ NMR. This material was recrystallized from 25 mL ethanol to provide 435 mg (38%) of the title nitrile as a tan solid: m.p. 300°C.

This compound may also be prepared as described: Tollman et al., J. Amer. Chem. Soc., **1969,** 91:2102.

EXAMPLE 43

Preparation of 4-Chloro-5-ethoxycarbonyl-7H-pyrrolo[2,3-d]pyrimidine

A solution of 5-bromo-4-chloropyrrolo[2,3-d]pyrimidine (Example 35) (232 mg; 1 mmol) in anhydrous THF (5 mL) was cooled to -78°C under argon and a solution of n-butyl lithium (1.3 mL of 2.31 M) was added at such a rate that the temperature of the reaction mixture remained below -72°C. After stirring the reaction mixture at -78°C for 45 minutes, a solution of ethyl chloroformate (0.15 mL) in THF (2 ml) was added slowly, maintaining the reaction temperature below -72°C. The reaction mixture was stirred at -78°C for 2 hours then allowed to warm to room temperature. A saturated solution of $NH_4Cl$ (20 mL) was added to the reaction mixture. The organic layer was separated and the aqueous layer was extracted with ethyl acetate (2x25 mL). The combined organic extracts were dried and evaporated to a white solid, yield 210 mg (92%): m.p. 140-141°C.

EXAMPLE 44

Preparation of 5-O-[(1,1-Dimethylethyl)dimethylsilyl]-2,3-O-(1-methylethylidene)-D-ribofuranose

The above-identified compound was prepared as described: H. Rosemeyer, H.; Seela, Helv. Chim. Acta., **1988,** 71, 1573.

EXAMPLE 45

Preparation of 5-Deoxy-D-ribofuranose

The above-identified compound was prepared as described: Snyder J.; Serianni, A.; Carbohydrate Research, **1987,** 163, 169.

EXAMPLE 46

Preparation of 5-O-Methyl-D-ribofuranose

The above-identified compound was prepared as described: Snyder, J.; Serianni, A.; Carbohydrate Research, **1987**, 163, 169.

EXAMPLE 47

Preparation of 1-O-Methyl-2,3-O-(1-methylethylidene) -5-O-(4-methylbenzenesulfonyl)-D-ribofuranoside

The above-identified compound was prepared as described: Snyder, J.; Serianni, A.; Carbohydrate Research, **1987,** 163, 169.

EXAMPLE 48

Preparation of 5-Deoxy-2,3-O-(1-methylethylidene)-D-ribofuranose

5-Deoxy-D-ribofuranose (8.g, 60 mmole) was dissolved in DMF (25 ml) and to the solution was added dimethoxypropane (10 ml) and p-toluenesulfonic acid (150 mg). The reaction was stirred overnight then neutralized with Amberlite 400 (OH resin. The mixture was filtered, concentrated and the residue chromatographed on $SiO_2$ gel using 15:1 $CH_2Cl_2$-MeOH. The appropriate fractions were collected and evaporated to yield 4.1 g (39% yield) of viscous liquid.

EXAMPLE 49

Preparation of 5-O-Methyl-2,3-O-(1-methylethylidene)-D-ribofuranose

To a solution of 5-O-methyl-D-ribofuranose (6.0 g, 36 mmole) in dry DMF (25 mL), 2,2-dimethoxypropane (25 mL) and p-toluenesulfonic acid (250 mg) were added and the solution was stirred at room temperature for 20 hours. Volatile materials were evaporated under reduced pressure and the residue was chromatographed over $SiO_2$ gel using 3:1 hexane:ethyl acetate. Appropriate fractions were pooled and evaporated to give the title compound as an oily product, yield: 5.0 g (68%).

EXAMPLE 50

5-Azido-5-deoxy-1-O-methyl-2,3-O-(1-methylethylidene) -D-ribofuranoside

A mixture of 1-O-methyl-2,3-O-(1-methylethylidene)-5-O-(4-methylbenzenesulfonyl)-D-ribofuranoside (8.0 g, 22 mmol), dry DMF (40 mL) and $NaN_3$ (4.0 g, 62 mmol) was heated at 80°C under anhydrous conditions for 12 hours. The solvent was evaporated under high vacuum and the residue was chromatographed over silica gel using $CH_2Cl_2$. The fractions containing the faster moving product were pooled and evaporated to obtain 4.8 g (94% yield) of a syrupy product.

EXAMPLE 51

Preparation of 5-Azido-5-deoxy-D-ribofuranose

A solution of 5-azido-5-deoxy-1-O-methyl-2,3-O-(1-methylethylidene)-D-ribofuranoside (4.6 g, 20 mmol) in 0.1% $H_2SO_4$ (300 mL) was gently refluxed for 3 hours. The acid was neutralized (pH ~5) with Amberlite 400 ($OH^-$ form) and the resin filtered and washed with ethanol (2x20 mL). The filtrate was evaporated to dryness under high vacuum to give the title compound as a syrupy residue; $^1H$ and $^{13}C$ NMR confirmed the identity of the product as a mixture of $\alpha$ and $\beta$ anomers.

EXAMPLE 52

Preparation of 5-Azido-5-deoxy-2,3-O-(1-methylethylidene)-β-D-ribofuranose

The crude 5-azido-5-deoxyribose (Example 51) was dissolved in dry DMF (10 mL) and treated with 2,2-dimethoxypropane (10 mL) and p-toluenesulfonic acid (100 mg). The solution was stirred at room temperature for 20 hours then evaporated under high vacuum. The residue was chromatographed over $SiO_2$ gel using 3:1 hexane:ethyl acetate. The appropriate fractions were pooled and evaporated to obtain the title compound, yield 2.4 g (56% yield).

EXAMPLE 53

Preparation of 1-O-Methyl-2,3-O-(1-methylethylidene) D-pentodialdo-1,4-furanoside

The above-identified compound was prepared as described: Moorman, A.,; Borchardt, R.; Nucleic Acid Chemistry-Part III, Ed. Towsend, L., Tipson, R.; John Wiley and Sons, N.Y.; **1986,** pages 38-41.

EXAMPLE 54

Preparation of 5-Benzoyl-D-allofuranose

The sugar aldehyde from Example 53 (100 mmole) was dissolved in anhydrous THF and treated with a commercially available solution of methyl magnesium bromide (100 m mol) under anhydrous conditions. After 2 hours of stirring at room temperature, a saturated solution of ammonium chloride in water (180 mL) was added. The organic layer was separated and the aqueous layer was extracted with ether (2 x 100 mL). The combined organic layers were dried and evaporated to obtain an oily product whose NMR was consistent with methyl-6-deoxy-2,3-isopropylidene-D-allofuranoside. The crude product was dissolved in pyridine (50 ml) and treated with benzoic anhydride (120 mmole). After stirring for 18 hours, methanol (2 ml) was added and the reaction mixture was evaporated under high vacuum. The residue was dissolved in ethyl acetate (300 ml) and washed successively with water, saturated bicarbonate solution, and brine. The organic layer was dried and evaporated to obtain a glassy product which was purified by column chromatography. Identity of the product was confirmed by IR and NMR spectroscopy. The intermediate protected sugar was then heated with aqueous sulfuric acid solution (0.01 N in water, 300 ml) to @ 80°C for 2 hours and neutralized with strongly basic ion exchange resin. The aqueous layer was separated and evaporated under high vacuum to obtain the title compound as a sticky mass. The product was confirmed by NMR and used in the next step without further purification.

EXAMPLE 55

Preparation of 5-Benzoyl-6-deoxy-2,3-O-(1-methylethylidene)-D-allofuranose

The benzoylated sugar (Example 54) was dissolved in a mixture of dry DMF (20 ml), 2,2-dimethoxypropane (20 ml) and p-toluenesulfonic acid (200 mg) and stirred at room temperature with the exclusion of moisture. The reaction was complete within two hours as evidenced by the absence of the starting material (TLC). The acid was neutralized by strongly basic ion exchange resin and the resin removed by filtration and washed. The combined washings and filtrate were evaporated under high vacuum and the residue was purified by chromatography. The pure product obtained was a glassy solid. IR and NMR were consistent with the title compound.

EXAMPLE 56

Preparation of 5,6-Dideoxy-5,6-didehydro-1-O-methyl-2,3-O-(1-methylethyldene)-D-allofuranoside

To a suspension of potassium-tert-butoxide (9.36 g) in anhydrous ether (300 ml), methyltriphenylphosphonium bromide (29.6 g) was added in small portions over 5 minute period with stirring under anhydrous conditions. The bright yellow colored solution (with some solid separated) was stirred for 1 1/2 hours then a solution of methyl-2,3-isopropylidene-D-pentodialdo-1,4-furanoside (8.0 g), Example 53, in anhydrous ether (75 ml) was added over 5 minute period. The reaction mixture was stirred overnight at room temperature. The solid material that formed in the reaction mixture was removed by filtration and washed repeatedly with ether. The combined washings and filtrate were evaporated and the residue purified by chromatography to obtain 6.5 g of product as an oil; TLC $R_f + 0.5$ (Silica gel, 97:3 hexane: EtoAc).

EXAMPLE 57

Preparation of 5,6-Dideoxy-5,6-didehydro-2,3-O-(1-methylethylidene)-D-allofuranose

A mixture of 5,6-dideoxy-5,6 didehydro-1-O-methyl-2,3-O-(1-methylethylidene)-D-allofuranose (Example 56) (2.0 g), and aqueous $H_2SO_4$ (0.1%, 50 ml) was heated to 90° for 3 hours at which time the indicated the starting material was consumed. The pH of the solution was adjusted to 7.5 with 1N NaOH solution, and evaporated to dryness. The residue was evaporated with DMF (2 X 20 ml) and the resulting semi solid product was slurried in methanol (25 ml). The undissolved solid was removed by filtration and washed with methanol (2 X 20 ml). The combined washings and the filtrate were evaporated to dryness and the resulting residue was dissolved in a mixture of dry DMF (10 ml), 2,2-dimethoxypropane (6 ml) and p-toluenesulfonic acid (50 mg). After stirring for 2 hours at room temperature the acid was neutralized with ion exchange resin (strongly basic type) and the resin was removed by filtration. Evaporation of the filtrate gave a product which was purified by chromatography over silica gel using 97:3 hexane:EtOAc as the a mobile phase; $R_f$ of the product 0.8 (silica gel, 9:1 hexane;EtOAc).

EXAMPLE 58

Preparation of 5,6-Dideoxy-1-O-methyl-2,3-O-(1-methylethylidene)-D-allofuranoside

A solution of the vinylic sugar (6.2 g), Example 56, in methanol (55 ml) was purged with argon and hydrogenated using 10% platinum on carbon as catalyst at 80 psi for 100 hours. The catalyst was removed by filtration and washed with methanol. The combined washings and filtrate were evaporated to obtain a colorless oil used directly for the next step. Yield: quantitative. TLC $R_f$ = 0.48 (Silica gel, 97:3 Hexane:EtOAc).

EXAMPLE 59

Preparation of 5,6-Dideoxy-2,3-O-(1-methylethylidene)-D-allofuranose

A mixture of 5,6-dideoxy-1-methyl-2,3-O-(1-methylethylidene)-D-allofuranoside (5.8 g), (Example 58), and water (160 ml) containing 0.16 ml of conc $H_2SO_4$ was heated to 90°C for 3 hours. The pH of the reaction mixture was adjusted to 7.5 with 1N NaOH solution and evaporated to dryness under high vacuum. The residue was coevaporated with DMF (2 x 50 ml) then dissolved in methanol (25 ml) and filtered. The filtrate was evaporated, dissolved in a mixture of dry DMF (10 ml), 2,2-dimethoxypropane (10 ml) and p-toluenesulfonic acid (200 mg) and stirred for 2 hours. The acid was neutralized with strongly basic ion exchange resin and the resin removed by filtration. The filtrate was evaporated and the residue chromatographed to obtain the title product; yield: 4.3 g.; TLC, $R_f$ = 0.6 (Silica gel, 2:1 hexane: EtOAc).

EXAMPLE 60

Preparation of 5-deoxy-1-O-methyl-2,3-O-(1-methylethylidene)-D-allofuranoside

To a solution of the vinylic sugar Example 56 (4.1 g) in THF (20 ml), borane:THF solution (10.65 ml of 1 M solution in THF) was added over 10 minutes. After stirring 2 hours at room temperature the reaction vessel was immersed in a cooling bath (ice-water) and an aqueous solution of NaOH (8 ml of 3M solution) was added with stirring. After 15 minutes a solution of $H_2O_2$ (30% aq. 4 ml) was added dropwise, and stirring was continued for an additional 15 minutes. Then the flask was immersed for 30 minutes in a water bath maintained at 55°C and cooled. The contents were extracted with methylene chloride (3 X 150 ml) and the organic layer was dried over anhydrous $MgSO_4$. The solvent was evaporated and the residue was chromatographed over silica gel using a hexane:ethyl acetate gradient as solvent. The fast moving minor product, $R_f$=0.7, (10%) was identified as 6-deoxy-1-methyl-2,3-0-(1-methylethylidene)-D-allofuranoside. The slower moving major product (90%), $R_f$=0.3 was identified by proton NMR to be the title compound; yield 3.7 g; $R_f$=0.3 (silica gel, 2:1 hexane:EtOAc).

EXAMPLE 61

Preparation of 6-O-(t-Butyldimethylsilyl)-2,3-O-(1-methylethylidene)-D-allofuranose

This compound may be prepared by t-butyldi- methylsilylation of the corresponding 6-hydroxy sugar, using t-butyldimethylsilyl chloride and imidazole in DMF.

EXAMPLE 62

Preparation of 5-deoxy-1-methyl-2,3-O-(1-methylethylidene)-6-p-toluenesulfonyl-D-allofuranoside

To an ice-cold solution of the hydroxy sugar Example 60, (3.69 g) in anhydrous pyridine (25 ml), p-toluenesulfonyl chloride (3.7 g) was added in small portions. The reaction mixture was stirred and allowed to warm to room temperature over 2 hours, at which time the reaction was complete (TLC). Unreacted p-toluenesulfonyl-chloride was quenched by adding 1 ml of methanol and the volatile components were evaporated under high vacuum. The residue was evaporated with DMF (2 x 20 ml), then dissolved in ethyl acetate (350 ml). The solution was washed successively with water and bicarbonate solution and the organic layer was dried over anhydrous $MgSO_4$. Evaporation of the solvent gave a residue which was purified by silica gel column chromatography; yield 4.1 g; $R_f$=0.8 (silica gel 2:1 hexane: EtOAc). Proton NMR indicated the product to be a mixture of $\alpha$ and $\beta$ anomers.

EXAMPLE 63

Preparation of 6-azido-5,6-dideoxy-1-O-methyl-2,3-0-(1-methylethylidene)-D-allofuranoside

A mixture of the tosyl sugar, Example 62, (4.0 g), dry DMF (20 ml) and sodium azide (1.5 g) was heated to 100°C in an oil bath under anhydrous conditions for 24 hours. The solvents were evaporated under high vacuum and the residue was dissolved in ethyl acetate (200 ml) and washed with water. The organic layer was dried ($MgSO_4$) and evaporated to obtain a colorless oil which was sufficiently pure by TLC and NMR for use in the next reaction; yield 2.09 g; $R_f$=0.35 (silica gel, 93:7 hexane:EtOAc).

EXAMPLE 64

Preparation of 6-azido-5,6-dideoxy-2,3-0-(1-methylethylidene)-D-allofuranose

A mixture of the azido sugar Example 63 (2.8 g), and aqueous sulfuric acid solution (100 ml of 0.1% by volume) was heated to 90°C for 3 1/2 hours at which time the starting material was found (TLC) to be consumed. The pH of the reaction mixture was adjusted to at 7.5 with 1N NaOH and evaporated to dryness under high vacuum. The residue was coevaporated with DMF (2 x 20 ml) and treated with methanol (25 ml). The insoluble solids were removed by filtration and washed with methanol (2 X 20 ml). The combined filtrates were evaporated to dryness. The oily product thus obtained was dissolved in a mixture of dry DMF (10 ml), 2,2-dimethoxy propane (6 ml) and p-toluene sulfonic acid. (50 mg) and stirred for 2 hours at room temperature. The solvents were evaporated under high vacuum and the residue was chromatographed over a silica gel column using a 4:1 hexane:EtOAc mixture as the mobile phase. After a fast moving spot, fractions containing the main product were combined and evaporated to obtain the title compound as a colorless oil; Yield 1.89 g; $R_f$=0.5 (silica gel, 2:1 hexane:EtOAc).

EXAMPLES 65-81

General procedure for the preparation of 5'-substituted -4-chloropyrrolo[2,3-d]pyrimidine-7-(1-β-D-ribosides)

A solution of the 5-substituted (H, $OCH_3$, $N_3$ or TBDMS-O-) 5-deoxy-isopropylideneribose (1 eq) in $CCl_4$ (1.4 eq) and THF was cooled to -78°C. Hexamethylphosphorous triamide (1.2 eq) was added dropwise and the reaction mixture stirred for 2 hours at -78°C. This solution of 1-α-chloro sugar was used directly in the next step.

To a slurry or solution of the substituted 4-chloropyrrolo[2,3-d]pyrimidine (1.4 eq corresponding to the sugar) in DMF, was added in four portions, NaH (1.4 eq) over 10 minutes. The solution was stirred 30 minutes then the above solution of chloro sugar (-25°C) was added and the reaction was stirred for 24 hours. The mixture was concentrated, diluted with EtOAc, filtered and the filtrate concentrated under vacuum. The residue was chromatographed on $SiO_2$ gel using 2:1 hexane-EtOAc. The appropriate fractions were collected and evaporated to yield the protected nucleoside.

The protected nucleoside was deblocked by dissolving in 90% trifluoroacetic acid and stirring for 2 hours. The solvent was evaporated and chased with methanol (3x). The product was crystallized from EtOH or EtOAc.

The compounds in Table IV (Examples 65-81) were prepared by this procedure:

TABLE IV

| GP1-# | EXAMPLE | B' | D | G | m.p(°C) |
|---|---|---|---|---|---|
| 475 | 65 | $CH_2OH$ | I | H | 183-181° |
| | 66 | $CH_2N_3$ | I | $NH_2$ | 203-205° |
| 406 | 67 | $CH_2OH$ | Br | H | >230° |
| 448 | 68 | $CH_3$ | I | H | 180-181° |
| 449 | 69 | $CH_3$ | $CH_3$ | H | 155-157° |
| 462 | 70 | $CH_2OCH_3$ | $CH_3$ | H | 142-144° |
| 460 | 71 | $CH_2OCH_3$ | I | H | 179-180° |
| 464 | 72 | $CH_2OCH_3$ | H | H | 122-124° |
| 692 | 73 | $-CH_2CH_3$ | Br | H | 163-165° |
| 690 | 74 | $-CH_2CH_3$ | I | H | 181-183° |
| 529 | 75 | $CH_2N_3$ | I | H | 203-205° |
| 554 | 76 | $CH_3$ | Br | H | 174-175° |

TABLE IV (continued)

| GP1-# | EXAMPLE | B' | D | G | m.p(°C) |
|---|---|---|---|---|---|
| 555 | 77 | $CH_3$ | H | $CH_3S$ | 140-142° |
| 569 | 78 | $CH_3$ | $SCH_3$ | H | 147-148° |
| 605 | 79 | $CH_2N_3$ | Br | H | 156-158° |
| --- | 80 | $CH_2CH_2N_3$ | I | H | foam |
| 713 | 81 | $CH=CH_2$ | I | H | 183-185° |

EXAMPLES 82 - 83

Preparation of 4-Amino-7-(5-amino-5-deoxy-1-β -D-ribofuranosy)-5-halopyrrolo[2,3-d]pyrimidines

A mixture of 4-chloro-5-iodo-7-[5-azido-5-deoxy-1-β-D-ribofuranosyl]pyrrolo[2,3-d]pyrimidine (Example 75 or 79) (500 mg), triphenylphosphine (550 mg) and pyridine (6 ml) was stirred under an argon atmosphere at room temperature for 24 hours. Pyridine was evaporated under high vacuum and the residue was triturated with ether. The residual semi-solid was treated with ammonium hydroxide (5 ml). A small amount of ethanol was added to cause complete dissolution of the compound. After stirring for 5 hours at room temperature the mixture was evaporated under vacuum and the residue was triturated with water (10 ml). The insoluble material was removed by filtration and the pH of the filtrate was adjusted to 5.5 with dilute HCl. The solution was refiltered and lyophilized to obtain a hygroscopic solid, whose NMR was compatible with the structure.

The above hygroscopic solid was dissolved in methanol, saturated with dry ammonia at at -15°C, then heated in a steel bomb at 80°C for 24 hours. The bomb was cooled and opened. Ammonia and methanol were evaporated and the residue was dissolved in water, charcoaled and filtered. The filtrate was lyophilized to obtain the title compound as a hygroscopic solids. The compounds listed in Table V were obtained by this procedure.

TABLE V

| GP 1-# | EXAMPLE | D | F | m.p(°C) |
|---|---|---|---|---|
| 550 | 82 | I | H | 166-206° |
| 649 | 83 | Br | H | 217-219° |

EXAMPLE 84

Preparation of 4-Amino-5-iodo-7-(5-acetylamino-5-deoxy-1-β-D-ribofuranosyl)pyrrolo[2,3-d]pyrimidine

To an ice cold solution of the 5'-amino compound from Example 54 (50 mg), in dry pyridine (5 ml), acetic anhydride (0.5 ml) was added with stirring. The reaction mixture was allowed to warm to room temperature over a period of 1 hour at which time the reaction was complete. The flask was reimmersed into the cooling bath and 15 ml of methanol was added to the reaction mixture to neutralize unreacted acetic anhydride. The solvent was evaporated under reduced pressure and the residue was purified by a short column chromatography to give the above-identified product, m.p. 160-163°C.

EXAMPLES 85 TO 113B

General Procedure for the Preparation of $N^4$-Substituted-4-aminopyrrolo[2,3-d]pyrimidine Nucleosides

A suspension of the substituted 4-Cl-pyrrolo[2,3-d]pyrimidine nucleoside (1 eq) in EtOH containing the amine (3 eq) and triethylamine (5 eq) was added to a small stainless steel bomb (in the case of diamines a 25% excess of chloride was used). The bomb was heated overnight (bath temperature 70-120°C), cooled, opened and the reaction mixture evaporated.

The product was crystallized from ethanol or ethyl acetate. The compounds in Table VI (Examples 85 to 113B) were prepared by this procedure:

TABLE VI

| GP1-# | Example | B' | D | F | G | m.p. (°C) |
|---|---|---|---|---|---|---|
| 334 | 85 | $CH_2OH$ | H | $NH_2$ | H | 249-250° |
| 394 | 86 | $CH_2OH$ | H | N-indolinyl | H | Foam |
| 393 | 87 | $CH_2OH$ | H | N-prolinyl | H | Foam |
| 296 | 88 | $CH_2OH$ | H | cyclopentyl-NH | H | Foam |
| 376 | 89 | $CH_2OH$ | Br | $NH_2$ | H | Foam |
| 321 | 90 | $CH_2OH$ | H | NHϕ | H | Foam |
| 476 | 91 | $CH_2OH$ | I | N-Indolinyl | H | 185-188° |
| 456 | 92 | $CH_3$ | I | $NH_2$ | H | 245-246° |
| 470 | 93 | $CH_3$ | I | N-indolinyl | H | 188-190° |
| 457 | 94 | $CH_3$ | I | $CH_3NH$ | H | 226-228° |
| 485 | 95 | $CH_3$ | I | $N_3$ | H | 213-214° |
| 498 | 96 | $CH_3$ | $CH_3$ | $NH_2$ | H | 212-214° |
| 461 | 97 | $CH_3$ | $CH_3$ | N-indolinyl | H | 171-173° |
| 463 | 98 | $CH_2OCH_3$ | I | $NH_2$ | H | 216-218° |
| 465 | 99 | $CH_2OCH_3$ | I | $CH_3NH$ | H | 188-189° |
| 474 | 100 | $CH_2OCH_3$ | H | N-indolinyl | H | 205-208° |
| 480 | 101 | $CH_2OCH_3$ | H | $CH_3NH$ | H | 163-164° |
| 513 | 102 | $CH_3$ | H | N-piperazinyl | H | 216-219° |
| 499 | 103 | $CH_3$ | I | N,N'-piperazinyl[1] | H | 220-223° |
| 500 | 104 | $CH_3$ | I | $-NH(CH_2)_6NH-$1/ | H | 227-229° |
| 512 | 105 | $CH_3$ | I | $-NH(CH_2)_2NH-$1/ | H | > 230° |
| 559 | 106 | $CH_3$ | I | $NH_2$ | $CH_3S$ | 200-202° |
| 561 | 107 | $CH_3$ | H | 1,4-piperazinyl[2] | H | foam |
| 606 | 108 | $CH_2N_3$ | Br | $NH_2$ | H | 182-184° |
| 639 | 109 | $CH_3$ | I | NHϕ | H | >230° |
| 581 | 110 | $CH_3$ | $CO_2C_2H_5$ | $NH_2$ | H | 162-168° |
| 681 | 111 | $CH_2OH$ | I | NHϕ | H | 224-225° |
| 680 | 112 | $CH_2OH$ | I | NH(4-Clϕ) | H | 234-235° |
| 689 | 113 | $CH_2OH$ | I | NH(4-$CH_3O$-ϕ) | H | 212-214° |
| 711 | 113A | $CH_2CH_2N_3$ | I | $NH_2$ | H | 151-153° |
| 714 | 113B | $CH=CH_2$ | I | $NH_2$ | H | 224-226° |

1/ Dimers having two pyrrolo[2,3-d]pyrimidine riboside moieties linked by the listed diamine.
2/ Dimer with purine riboside.

EXAMPLE 114

Preparation of 5-Iodo-7-(5-deoxy-1-β-D-ribofuranosyl) pyrrolo[2,3-d]pyrimidin-4(3H)-thione

A solution of 4-chloro-5-iodo-7-[5-deoxy-1-β-D-ribofuranosyl]pyrrolo[2,3-d]pyrimidine (Example 68) (250 mg, 0.60 mmol) and thiourea (250 mg) in absolute EtOH was refluxed gently under an argon atmosphere for 16 hours. The solvent was evaporated under reduced pressure and the residue was triturated with water (10 ml). The solid was collected by filtration, washed with water and dried in air: Yield 200 mg (81%); m.p. 161-163°C.

EXAMPLES 115 TO 120

General Procedure for S-Alkylation of 5-Iodo-7-(5-deoxy-1-β-D-ribofuranosyl)pyrrolo[2,3-d]pyrimidin-4(3H)-thione

To a solution of 5-iodo-7-(5-deoxy-1-β-D-ribofuranosyl) pyrrolo[2,3-d]pyrimidine-4-thione (Example 114) (50 mg) in concentrated $NH_4OH$ (10 ml), the appropriate alkylating agent (e.g. methyl iodide, alkyl or substituted benzyl bromide) was added and the mixture stirred at room temperature for 20 hours. Volatile material was evaporated under reduced pressure and the residue triturated with ether. To the residue, water (5 ml) was added and the solid was

collected by filtration and washed with water.

The products obtained by this procedure (Examples 115 to 120) are listed in Table VII:

TABLE VII

| GP1-# | Example | B' | D | F | m.p. |
|---|---|---|---|---|---|
| 482 | 115 | $CH_3$ | I | $SCH_3$ | 212-233° |
| 493 | 116 | $CH_3$ | I | $SCH_2CH{=}CH_2$ | 192-193° |
| 494 | 117 | $CH_3$ | I | $SCH_2\phi NO_2(4)$ | 224-226° |
| 502 | 118 | $CH_3$ | I | $SC_4H_9$ | 186-187° |
| 503 | 119 | $CH_3$ | I | $SCH_2\phi$ | 212-213° |
| 511 | 120 | $CH_2OH$ | I | $SCH_3$ | 214-150° |

EXAMPLE 121

Preparation of 4-Phenyl-7-(1-β-D-ribofuranosyl)pyrrolo [2,3-d]pyrimidine

To a solution of 4-chloro-7-[2,3-O-(1-methylethylidene)-1-β-D-ribofuranosyl]pyrrolo[2,3-d]pyrimidine (200 mg) and phenylboronic acid (250 mg) in dry diglyme (10 ml) was added palladium-tetrakis-triphenylphosphine (30 mg), followed by aqueous $Na_2CO_3$ solution (0.2 ml of 2M solution). The reaction mixture was heated to 90°C under anhydrous conditions for 6 hours. The solvent was evaporated under high vacuum and the residue was purified by HPLC on a reverse phase C-18 column. The purified intermediate was treated with 2 ml of trifluoracetic acid (80%) and stirred for 15 minutes then evaporated under high vacuum and the residue crystallized from ethanol; yield 20 mg, m.p. 163-164°C.

EXAMPLES 122 TO 124

General Procedure for the Preparation of 4-Amino- and 4-Arylamino-5-aryl-7-(1-β-D-ribofuranosyl)pyrrolo-[2,3-d] pyrimidines

To stirred mixture of the 4-amino- or 4-arylamino-5-iodopyrrolo[2,3-d]pyrimidine riboside (or corresponding hydroxyl protected compound) (0.1 m mole), $Pd(PPh_3)_4$ (10 mg, 0.01 mole) in diglyme was added a solution of the arylboronic acid (0.4 mmol) in EtOH and 0.4 ml of aqueous 2 M $Na_2CO_3$. The mixture was heated to 100°C and the reaction monitored by TLC. After the reaction was complete, the cooled mixture was filtered and concentrated under vacuum. The residue was chromatographed over $SiO_2$, eluting with $CH_2Cl_2$-MeOH mixtures or by HPLC on a Bondapak C-18 column with a MeOH-$H_2O$ gradient.

The compounds in Table VIII may be prepared by this procedure:

TABLE VIII

| GP1 # | Example | D | F | M.P.(°C) |
|---|---|---|---|---|
| ----- | 122 | ϕ | ϕNH | |
| _____ | 123 | ϕ | $NH_2$ | |
| 718 | 124 | 2-furanyl | ϕNH | foam |

EXAMPLES 125-126

General Procedure for the Preparation of 4-Amino-and 4-Arylamino-5-aryl-7-(5-deoxy-1-β-d-ribofuranosyl)pyrrolo [2,3-d]pyrimidines

The above-identified compounds were prepared as described in Example 122-124 from the 4-amino- or 4-arylamino-5-iodo-7-(5-deoxy-1-β-D-ribofuranosyl)pyrrolo[2,3-d]pyrimidine and an arylboronic acid.

The compounds in Table IX were prepared by this procedure:

TABLE IX

| GP1 # | Example | B' | D | F | G | M.P. (°C) |
|---|---|---|---|---|---|---|
| 684 | 125 | $CH_3$ | Phenyl | $NH_2$ | H | 106-109 |
| 683 | 126 | $CH_3$ | Phenyl | NH-ϕ | H | 207-208 |

EXAMPLE 127

Preparation of 4-Chloro-5-iodo-7-(6-deoxy-1-β-D-allofuranosyl)pyrrolo[2,3-d]pyrimidine

The above-identified compound was prepared according to the general procedure used for Examples 65-81; m.p. 211-213°C.

EXAMPLE 128

Preparation of 4-Amino-5-iodo-7-(6-deoxy-1-β-D-allofuranosyl)pyrrolo[2,3-d]pyrimidine

The 4-chloro compound, Example 127, was heated in a steel bomb with methanolic ammonia at 120°C for 12 hours followed by the usual work up (see Example 85). The product was obtained as a white crystalline solid; m.p. 206-208'C.

EXAMPLES 129-130

General Procedure for the Preparation of 4-Amino-5-halo--7-(5,6-dideoxy-1-β-D-allofuranosyl)pyrrolo[2,3-d]pyrimidine

The above-described compounds were prepared by the general procedures for Examples 65 to 81.
The compounds obtained by this procedure are listed in Table X.

TABLE X

| GP1 # | Example | B' | D | F | G | M.P. (°C) |
|---|---|---|---|---|---|---|
| 693 | 129 | $CH_2CH_3$ | Br | $NH_2$ | H | 229-230 |
| 691 | 130 | $CH_2CH_3$ | I | $NH_2$ | H | 233-234 |

EXAMPLE 131

Preparation of 4-Amino-3-bromopyrazolo[3,4-d]pyrimidine

The above-identified compound was prepared as described: Leonova, T.; Yashunskii, V.; Khim. Get. Soed., **1982**, 982.

EXAMPLE 132

Preparation of 4-Amino-3-(cyanomethyl)pyrazolo[3,4-d]pyrimidine

The above-identified compound was prepared as described: Carboni, R.,; Coffman, D.,; Howard, E.; J.Am. Chem. Soc., **1958** 80:2838.

EXAMPLE 133

Preparation of 4-Amino-3-cyanopyrazolo[3,4-d]pyrimidine

The above-identified compound was prepared as described: Taylor, E.; Abul-Hsan, A.; J. Org. Chem., **1966**, 31:342.

EXAMPLE 134

Preparation of 4-Amino-3-phenylpyrazolo[3,4-d]pyrimidine

The above-identified compound was prepared from trimethyl orthobenzoate as described: Kobayashi, S.; Chem. Pharm. Bull. (Jap.) **1973,** 21:941.

EXAMPLES 135-139

General Procedure for the Preparation of Aryl Thiomorpholides

A mixture of the aromatic carboxaldehyde (0.1 mole), sulfur (4.8 g, 0.15 mole) and morpholine (18 mL, 0.15 mole) was heated at 180°C for 3-5 hours then cooled and diluted with $H_2O$. The solid was collected by filtration or, if oily, extracted with $CH_2Cl_2$, dried ($Na_2SO_4$) and concentrated. The crude product was recrystallized from alcohol, alcohol-$H_2O$ mixtures or chromatographed over $SiO_2$.

The compounds in Table XI were prepared by this procedure:

TABLE XI

| Example | Aryl | M.P. (°C) |
|---|---|---|
| 135 | 4-$CH_3O\phi$ | 95-98° |
| 136 | 4-Clϕ | 137-140° |
| 137 | 2-Brϕ | ---- |
| 138 | 2-thienyl | 75-77° |
| | 3-thienyl | 84-87° |
| 139 | 3-$CH_3O\phi$ | 134-139° |

EXAMPLES 140-144

General Procedure for the Preparation of 5-Amino-3-aryl-4-cyanopyrazoles

The above-identified compounds were prepared from the corresponding aryl thiomorpholides (Examples 135-139) following the general procedure described: Tominaga, Y.,; et al.; J.Heterocyclic Chem., **1990,** 27:647.

The compounds listed in Table XII were prepared by this procedure:

TABLE XII

| Example | Aryl | M.P. (°C) |
|---|---|---|
| 140 | 4-$CH_3O\phi$ | 155-160° |
| 141 | 4-Clϕ | 218-222° |
| 142 | 2-Brϕ | ---- |
| 143 | 2-thienyl | 260-265° |
| 144 | 3-thienyl | 229-231° |

EXAMPLES 145-148

General Procedure for the Preparation of 5-Amino-3-aryl-4-carboxamidopyrazoles

The above-identified compounds were obtained from the corresponding cyano compounds (Example 140-144) following the general procedure described: Kobayashi, S.; Chem. Pharm. Bull. (Jap.), **1973,** 21:941.

The compounds listed in Table XIII were prepared by this procedure:

TABLE XIII

| Example | Aryl | M.P. (°C) |
|---|---|---|
| 145 | ϕ | 203-205° |
| 146 | 4-$CH_3O\phi$ | ---- |

TABLE XIII (continued)

| Example | Aryl | M.P. (°C) |
|---|---|---|
| 147 | 4-Clϕ | 210-215° |
| 148 | 2-Brϕ | ---- |

EXAMPLE 149-154

General Procedure for the Preparation of 4-Amino-3-arylpyrazolo[3,4-d]pyrimidines

A mixture of the 5-amino-3-aryl-4-cyanopyrazole and formamide (5 ml/g) under $N_2$, was refluxed (190-200°C) with stirring for 4 hours. The cooled mixture was diluted with $H_2O$ and the solid collected by filtration. The crude products were used directly for subsequent steps or purified by recrystallization. The compounds listed in Table XIV were prepared by this procedure.

TABLE XIV

| Example | Aryl | M.P. (°C) |
|---|---|---|
| 149 | ϕ | >220 |
| 150 | 4-$CH_3O$ϕ | >220 |
| 151 | 4-Clϕ | >220 |
| 152 | 2-Brϕ | >220 |
| 153 | 2-Thienyl | >220 |
| 154 | 3-Thienyl | >283 (dec.) |

EXAMPLES 155-158

General Procedure for the Preparation of 3-Arylpyrazolo-[3,4-d]pyrimidin-4-ones from 5-Amino-3-aryl-4-carboxamidopyrazoles

A mixture of the 5-amino-3-aryl-4-carboxamidopyrazole and formamide (5 ml/g) was refluxed at 190°-200°C for 2 hours, cooled and diluted with $H_2O$. The solid was collected by filtration and dried under vacuum. Further purification was effected by dissolving the compound in dilute sodium hydroxide, followed by charcoal treatment and precipitation with acetic acid.

The compounds listed in Table XV were prepared by this procedure:

TABLE XV

| Example | Aryl | M.P. (°C) |
|---|---|---|
| 155 | ϕ | >200° |
| 156 | 4-$CH_3O$ϕ | >220° |
| 157 | 4-Clϕ | >220° |
| 158 | 2-Thienyl | >220° |

EXAMPLES 159-160

General Procedure for Preparation of 3-Arylpyrazolo [3,4-d]pyrimidin-4-ones from 4-Amino-3-aryl pyrazolo[3,4-d] pyrimidines

To a well stirred slurry of the 3-aryl-4-aminopyrazolo[3,4-d]pyrimidine (25 mmoles) in 175 ml of 9% HCl at 0 to 5°C, was added dropwise, over 45 minutes, an aqueous solution of sodium nitrite (15.0 g in 30 ml). The mixture was allowed to warm to room temperature and solid sodium nitrite (5.0 g) was added. After 15 minutes the mixture was cautiously heated to boiling (foaming!), then cooled. The product was collected by filtration, rinsed with $H_2O$ and dried at 50°C under vacuum.

The compounds listed in Table XVI were prepared by this procedure:

TABLE XVI

| Example | Aryl | M.P.(°C) |
|---|---|---|
| 159 | φ | >220° |
| 160 | 2-thienyl | >220° |

EXAMPLES 161-163

General Procedure for the Preparation of $N^4$-Aryl and $N^4$-Alkyl substituted 4-amino-3-aryl-pyrazolo[3,4-d]pyrimidines

A mixture of the 3-arylpyrazolo[3,4-d]pyrimidin-4-one (15 mmoles), $POCl_3$(18 ml, 195 mmoles) and diethylaniline (5 ml, 31 mmoles) was refluxed under $N_2$, for 4 hours then concentrated under vacuum. The residue was decomposed by addition of ice and extracted (4x) with 3:1 ether-ethyl acetate. The combined organic extracts were washed with water and dried ($Na_2SO_4$). The solution was concentrated under vacuum and the crude 4-chloro-3-arylpyrazolo[3,4-d]pyrimidine (50-70% yield) was added to a solution of amine (2.2 equivalents) in EtOH (25 ml/mmole chloro compound). The mixture was heated to reflux for 30 minutes then cooled and the product collected by filtration and rinsed with EtOH. Recrystallization from alcohol-ethyl acetate mixtures gave the title compounds.
The compounds listed in Table XVII were prepared by this procedure:

TABLE XVII

| Example | 3-Aryl | 4-Arylamino | M.P.(°C) |
|---|---|---|---|
| 161 | φ | φ | 229-232° |
| 162 | φ | 4-ClφNH | 232-233° |
| 163 | φ | 4-$CH_3$0φNH | 218-220° |

EXAMPLE 164

Preparation of 3-Bromopyrazolo[3,4-d]pyrimidin-4-one

The above-identified compound was prepared as described Chu, I.; Lynch, B.; J. Med. Chem., **1975,** 18:161.

EXAMPLE 165

Preparation of 3-Bromo-1-(2,3,5-O-tribenzoyl-1-β-D-ribofuranosyl)pyrazolo[3,4-d]pyrimidin-4-one

The above-identified compound was prepared as described: Cottam, H.; Petrie, C.; McKernan, P.; Goebel, R.; Dailey, N.; Davidson, R.; Robins, R.; Revankar, G.; J. Med. Chem., **1984**, 27:1120.

EXAMPLE 166

Preparation of 3-Substituted-4-chloro-1-(2,3,5,-O-tribenzoyl -1-β-D-ribofuranosyl)pyrazolo[3,4-d]pyrimidin-4-ones

The above-identified compounds may be prepared from the corresponding pyrazolo[3,4-d]-pyrimidones analogously to the procedure described in Example 2.

EXAMPLES 167 TO 169

General Procedure for Preparation of 3-Substituted 4-amino- and 4-(arylamino)-1-(1-β-D-ribofuranosyl)pyrazolo[3,4-d] pyrimidines

To a slurry of the 3-substituted-4-chloropyrazolo[3,4-d]pyrimidine nucleoside tribenzoate (1.0 eq) (Example 166) in a mixture of EtOH and THF, was added ethanolic ammonia or the amine (1.5 eq) and $Et_3N$ (3.5 eq). The reaction rapidly became homogenous and after 0.5-12 hours, was evaporated. The residue was dissolved in $CH_2Cl_2$, washed with aqueous $K_2CO_3$ then $H_2O$ and the solution dried ($Na_2SO_4$). After evaporation, the residue was recrystallized or chromatographed on $SiO_2$ gel using $CH_2Cl_2$-Me0H mixtures. The resulting tribenzoate of the title compound was deblocked by stirring in methanolic NaOMe. The mixture was neutralized with amberlite IR-120(+) resin, filtered and

evaporated. The residue was recrystallized to give the title compounds.

Examples 167 to 169 listed in Table XVIII were prepared by this procedure:

TABLE XVIII

| GP1-# | Example | 3- | 4- | m.p.(°C) |
|---|---|---|---|---|
| 596 | 167 | I | $NH_2$ | 180-185° |
| 469 | 168 | Br | N-indolinyl | 195-196° |
| 536 | 169 | $CH_3$ | $NH_2$ | 241-242° |

EXAMPLE 170

Preparation of 4-(N-Indolinyl)-1-(1-β-D-ribofuranosyl)pyrazolo[3,4-d]pyrimidine

A solution of the bromide (Example 168) (351 mg, 0.78 mmol) in methanol containing 300mg 10% Pd-C and Raney Ni was hydrogenated at 40 psi until the reaction was complete as judged by TLC. The mixture was filtered, the filtrate concentrated and the product collected by filtration to give the title compound: 120 mg (42%); m.p. 215-219°.

EXAMPLE 171

Preparation of 3-Bromo-1-[2,3-O-(1-methylethylidene) -1-β-D-ribofuranosyl]pyrazolo[3,4-d]pyrimidin-4-one

Crude 3-bromoallopurinol riboside (prepared from 33.0 g of tribenzoate and NaOMe/MeOH (Example 83) was added to a cold (5°C), stirred solution of 1 M ethanolic HCl (6.5 ml) and dimethoxypropane (20 ml) in 1.1 L of $Me_2CO$. The mixture rapidly became homogenous and was stirred 45 minutes until complete (TLC). To the solution was added $Na_2CO_3$ (5.0 g), concentrated $NH_4OH$ (5 ml) and the mixture was stirred until the pH reached 6-7. The reaction was filtered and evaporated to a solid. The residual solid was dissolved in 300 ml of boiling EtOH and the solution concentrated by distilling EtOH to a final volume of 150 ml. The solution was chilled overnight and the solid collected by filtration and rinsed with cold EtOH. After drying (50°C), 16.7 g (86%) of the title compound were obtained; m.p. 221-224°C.

EXAMPLE 172

Preparation of 3-Bromo-1-[2,3-O-(1-methylethylidene)-5-O-(4-methylbenzenesulfonyl)-1-β-D-ribofuranosyl]pyrazolo [3,4-d]pyrimidin-4-one

To a solution of the isopropylidene alcohol (Example 171) (3.0 g, 7.74 mmol) in pyridine (18 ml) at 0°C was added p-toluenesulfonyl chloride (1.77 g, 9.30 mmol). The reaction'was held at 0°C for 3 hours then poured into 160 ml of cold $H_2O$ with stirring. The mixture was allowed to settle, the $H_2O$ decanted and the residue dissolved in $CH_2Cl_2$. The $CH_2Cl_2$ solution was washed with 0.5 N $H_2SO_4$, 5% aqueous $K_2CO_3$ and dried ($Na_2SO_4$). After evaporation under vacuum, 4.03g (96% yield) of the title compound were obtained as a foam.

EXAMPLE 173

Preparation of 1-[5-Azido-5-deoxy-2,3-O-(1-methylethylidene)-1-β-D-ribofuranosyl]-3-bromopyrazolo-[3,4-d] pyrimidin-4-one

To a warm stirred solution of $NaN_3$ (7.69 g, 0.12 moles) in DMSO (70 ml) was added the tosylate (Example 172) (16.0 g, 0.03 mol). The solution was rapidly heated to 80°C and maintained at this temperature for 45 minutes. After cooling, the reaction mixture was added with stirring to $H_2O$ (600 ml). The mixture was extracted 4x with $CHCl_3$ (75 ml) and the combined $CHCl_3$ extracts were washed with $H_2O$, dilute brine, dried ($Na_2SO_4$) and concentrated to give 11.0 g of a white foam. TLC (9:1 $CH_2Cl_2$-MeOH on $SiO_2$) indicated a mixture of three products in the approximate ratio of 1:2:1. The middle spot was subsequently determined to be the desired azide.

The mixture was purified by chromatography on a 10 x 15 cm column of $SiO_2$ eluting with 1% $Me_2CO$ in $CH_2Cl_2$ then increasing concentrations of $Me_2CO$. The first product eluted with 4% acetone. $^{13}C$-NMR indicated a lack of tosyl and azide functions at C-5'. Chemical shifts of the ribose C5' suggested a cyclonucleoside. The desired azide eluted with 6% $Me_2CO$. Its identity was confirmed by $^1H$ and $^{13}C$ NMR. Further elution afforded the third product which

appeared by $^{13}C$-NMR to also be a cyclonucleoside.

The fractions containing the desired product (middle TLC spot) were combined and evaporated to give the title compound; 5.90 g (48% yield), m.p. 168°C (d).

EXAMPLE 174

Preparation of 3-Amino-1-[2,3-O-(1-methylethylidene) -1-β-D-ribofuranosyl]pyazolo[3,4-d]pyrimidin-4-one

A mixture of bromide (Example 171) (2.35 g, 6.1 mmol) CuCl (88 mg) and Cu (101 mg) in MeOH (45 ml) was placed in a bomb and saturated with gaseous $NH_3$. The bomb was sealed and heated to 110°C for 10 hours. After cooling, the bomb was opened, the contents filtered and the filtrate evaporated. The residue was chromatographed on $SiO_2$ gel using 9:1 $CH_2Cl_2$-MeOH. The appropriate fractions were combined and evaporated to yield the title compound as a solid; 2.1 g (98% yield); m.p. 142-144°C.

EXAMPLE 175

Preparation of 3-Iodo-1-[2,3-O-(1-methylethylidene) -1-β-D-ribofuranosyl]pyrazolo[3,4-d]pyrimidin-4-one

A mixture of the amine (Example 174) (2.58 g, 8.0 mmole), isoamyl nitrite (30 ml), methylene iodide (20 ml) and $CH_3CN$ was refluxed under argon for 10 minutes. The cooled mixture was evaporated and chromatographed on $SiO_2$ gel using 2% methanol in methylene chloride. Appropriate fractions were combined and evaporated to give the title compound: 1.08 g (66% yield); m.p. > 220°C.

EXAMPLE 176

Preparation of 3-Iodo-1-[2,3-O-(1-methylethylidene)-5-O-(4-methylbenzenesulfonyl)-1-β-D-ribofuranosyl]pyrazolo-[3,4-d]pyrimidin-4-one

The above identified compound was prepared analogously to the procedure described for Example 172.

EXAMPLE 177

Preparation of 3-Iodo-1-[5-azido-5-deoxy-2,3-O-(1-methylethylidene)-1-β-D-ribofuranosyl]pyrazolo-[3,4-d]pyrimidin-4-one

The above identified compound was prepared analogously to the procedure described for Example 173 in 45% yield; m.p. 203°C(d).

EXAMPLE 178

Preparation of 3-Halo-4-chloro-1-[5-azido-5-deoxy-2,3-O-(1-methylethylidene)-1-β-D-ribofuranosyl)]pyrazolo[3,4-d] pyrimidine

The above-identified compounds were prepared analogously to the procedure described for Example 2 from the pyrimidin-4-one (Example 173 or 177). The title compounds were obtained as unstable yellow oils and used immediately in the next step.

EXAMPLES 179 to 181

General Procedure for the Preparation of 4-Amino and 4-hydrocarbyl-amino-1-(5-azido-5-deoxy-1-β-D-ribofuranosyl) pyrazolo[3,4-d]pyrimidines

To a solution of the chloro azide (Example 178) (1 eq) in 1:1 THF-EtOH (10% w/v) was added to the amine (1.2-2.0 eq) and excess $Et_3N$ (for the 4-amino compounds the solution was saturated with $NH_3$ gas). The resulting solution was stirred for 2-24 hours and checked for complete reaction by TLC ($SiO_2$; 9:1 $CH_2Cl_2$:$Me_2CO$).

The reactions using amines were worked up in the following manner. The reaction mixture was evaporated, the residue dissolved in $CH_2Cl_2$ and the solution washed with aqueous $NaHCO_3$, then $H_2O$ and dried ($Na_2SO_4$). Concentration of the $CH_2Cl_2$ solution and chromatography of the residue over $SiO_2$ gel using $CH_2Cl_2$-$Me_2CO$ mixtures

gave the purified isopropylidene N[4]-substituted compounds. The isopropylidene 4-amino compounds were isolated by evaporating the reaction mixture and recrystallizing the residue from EtOH.

The isopropylidene compounds were deblocked using the procedure described under Example 3.

The compounds in Table XIX (Examples 179 to 181) were prepared by this procedure:

TABLE XIX

| GP1-# | Example | D | F | m.p. (°C) |
|---|---|---|---|---|
| 507 | 179 | Br | $NH_2$ | 169-170° |
| 501 | 180 | Br | N-indolinyl | 133-138° |
| --- | 181 | I | $NH_2$ | 193-195° |

EXAMPLES 182 TO 185

General Procedure for the Preparation of 4-Amino- and 4-Substituted-amino-1-(5-amino-5-deoxy-1-β-D-ribofuranosyl)-3-halo-pyrazolo[3,4-d]pyrimidines and Their Hydrochloride Salts

A solution of the azide (Examples 179 to 181) (1.0 equivalent) and triphenylphosphine (1.5 equivalents) in pyridine (5 ml/g of azide) was stirred for 2 hours and checked by TLC (9:1 $CH_2Cl_2$-MeOH) for completion. To the reaction mixture was added concentrated $NH_4OH$ (1.25 ml/g of azide) and the solution stirred overnight. The solution was evaporated to dryness, slurried in $Et_2O$, filtered (3x) and the insoluble residue dried under vacuum. The resulting solid was recrystallized or converted to its HCl salt (0.1 N HCl, EtOH) and crystallized to give the title compounds.

The compounds in Table XX (Examples 182-185) may be prepared by this procedure:

TABLE XX

| GP1-# | Example # | D | F | m.p.(°C (HCl salt)) |
|---|---|---|---|---|
| 515 | 182 | Br | $NH_2$ | >230° |
| 516 | 183 | Br | N-indolinyl | 170° (broad) |
| 547 | 184 | I | $NH_2$ | 188° |
| 558 | 185 | Br | 1,4-piperazinyl[1] | 195° (broad) |

1/ a dimer

EXAMPLE 186

Preparation of 1,2,3-O-Triacetyl-5-deoxy-D-ribofuranoside

The above-identified compound was prepared as described: Snyder, J.; Serianni, A.; Carbohydrate Research, **1987**, 163:169.

EXAMPLE 187

Preparation of 1,2,3-O-Triacetyl-5-azido-5-deoxy-D-ribofuranoside

To a cooled solution of 5-azido-5-deoxyribose (6.2 g, 0.035 mole) (Example 51) in 10 ml of pyridine was added acetic anhydride (18 ml) and the mixture stirred for 24 hours at room temperature. The mixture was concentrated under vacuum, the residue dissolved in $CH_2Cl_2$ and the solution washed with 5% $NaHCO_3$. The organic layer was then washed with 0.5 N $H_2SO_4$, dried ($Na_2SO_4$) and evaporated. The residue was filtered through a plug of $SiO_2$ gel ($CH_2Cl_2$) and the filtrate concentrated to afford the title compound, 9.0 g (98% yield) as a semisolid mixture of α and β isomers.

EXAMPLES 188-203

General Procedure for the Preparation of 5'-Substituted-3,4-disubstituted-pyrazolo[3,4-d]pyrimidine Nucleosides

To a slurry of the 3,4-disubstituted pyrazolo[3,4-d]pyrimidine (5.0 mmol) in nitromethane, nitroethane or benzonitrile under $N_2$, was added the acyl-protected ribose (5.0-7.0 mmoles). To the stirred mixture, was added $BF_3.Et_2O$ (7.0 mmoles) and the mixture was refluxed for 90 minutes, then cooled and evaporated under vacuum. If a 5'-deoxy

derivative was used, $Et_3N$ was added prior to the evaporation of the solvent (to complex $BF_3$. $Et_2O$).

The residue was taken up in $CH_2Cl_2$, filtered and chromatographed over $SiO_2$ gel using $CH_2Cl_2$-MeOH gradients. Later fractions contained the N-2 isomer. Fractions containing the desired N-1 isomer were combined and evaporated to yield the title compounds as foams.

The compounds in Table XXI (Examples 188-203) were prepared by this procedure:

TABLE XXI

| Example # | B' | D | F | M.P. (°C) |
|---|---|---|---|---|
| 188 | $\phi CO_2CH_2$ | CN | $NH_2$ | foam |
| 189 | $\phi CO_2CH_2$ | $CH_2CN$ | $NH_2$ | foam |
| 190 | $\phi CO_2CH_2$ | $\phi$ | $NH\phi$ | foam |
| 191 | $CH_2N_3$ | Br | $NH_2$ | foam |
| 192 | $CH_2N_3$ | CN | $NH_2$ | foam |
| 193 | $CH_2N_3$ | $CH_2CN$ | $NH_2$ | foam |
| 194 | $CH_2N_3$ | $\phi$ | $NH_2$ | foam |
| 195 | $CH_2N_3$ | 4-Cl$\phi$ | $NH_2$ | foam |
| 196 | $CH_2N_3$ | 4-$CH_3O\phi$ | $NH_2$ | foam |
| 197 | $CH_2N_3$ | 2-thienyl | $NH_2$ | foam |
| 198 | $CH_3$ | $\phi$ | $NH_2$ | foam |
| 199 | $CH_3$ | 4-$CH_3O\phi$ | $NH_2$ | foam |
| 200 | $CH_3$ | 4-Cl$\phi$ | $NH_2$ | foam |
| 201 | $CH_3$ | 2-thienyl | $NH_2$ | foam |
| 202 | $CH_3$ | 3-thienyl | $NH_2$ | foam |
| 203 | $CH_3$ | $\phi$ | $NH\phi$ | foam |

EXAMPLE 204

General Procedure for the Preparation of 3-Substituted 1-(5-azido-5-deoxy-2,3-O-diacetyl-1-β-D-ribofuranosyl)-4-chloropyrazolo[3,4-d]pyrimidines, 5'-Deoxy Analogs and Protected 5'-Hydroxy Analogs

The above identified compounds were prepared from the pyrazolo[3,4-d]pyrimidone esters analogously to the procedure described in Example 2 and were used immediately in the next step.

EXAMPLES 205-221

General Procedure for the Preparation of 3,4-Disubstituted-1-(5-azido-5-deoxy-1-β-D-ribofuranosyl))pyrazolo[3,4-d]pyrimidines, 5'-Deoxy Analogs and 5'-Hydroxy Analogs

The above identified compounds were prepared from the diesters analogously to the procedure described in Example 167-169. Methanolic $NH_3$ (method A) or NaOMe (method B) was used to deblock the acyl-protected nucleosides (Examples 188-204). In the case of the cyano-substituted compounds, these methods led to different products by further reaction of the cyano group. The title compounds were isolated by conventional techniques.

The compounds listed in Table XXII (Examples 205-221) were prepared by this procedure.

TABLE XXII

| Example | GP1-# | B' | D | F | Method | M.P. (°C) |
|---|---|---|---|---|---|---|
| 205 | 612 | $CH_2OH$ | $CH_2CN$ | $NH_2$ | A | 220° (dec) |
| 206 | 613 | $CH_2OH$ | $CH_2C(=NH)OCH_3$ | $NH_2$ | B | 75° (dec) |
| 207 | 695 | $CH_2OH$ | Ø | NHØ | B | 220-224° |
| 208 | 507 | $CH_2N_3$ | Br | $NH_2$ | B | 172° (d) |
| 209 | 623 | $CH_2N_3$ | $C(=NH)NH_2$ | $NH_2$ | A | 203-206° |
| 210 | 624 | $CH_2N_3$ | $CH_2CN$ | $NH_2$ | A | 153-156° |
| 211 | 641 | $CH_2N_3$ | Ø | $NH_2$ | B | 203-205° |
| 212 | 662 | $CH_2N_3$ | 4-Clφ | $NH_2$ | B | 175-177° |
| 213 | 666 | $CH_2N_3$ | 4-$CH_3$Oφ | $NH_2$ | B | 153-155° |
| 214 | 654 | $CH_2N_3$ | 2-Thienyl | $NH_2$ | B | 180-181° |
| 215 | 667 | $CH_2N_3$ | Ø | NHØ | B | 120-125° |
| 216 | 663 | $CH_3$ | Ø | $NH_2$ | B | 223-224° |
| 217 | 678 | $CH_3$ | 4-Clφ | $NH_2$ | B | 130-133° |

TABLE XXII

| Example | GP1-# | B' | D | F | Method | M.P. (°C) |
|---|---|---|---|---|---|---|
| 218 | 679 | $CH_3$ | 4-$CH_3O\phi$ | $NH_2$ | B | 175-176° |
| 219 | 664 | $CH_3$ | 2-Thienyl | $NH_2$ | B | 174-175° |
| 220 | 685 | $CH_3$ | 3-Thienyl | $NH_2$ | B | 153-154° |
| 221 | 683 | $CH_3$ | Ø | NHØ | B | 207-208° |

EXAMPLES 222-229

General Procedure for the Preparation of 4-Amino- and 4-Arylamino-3-substituted-1-(5-amino-5-deoxy -1-β-D-ribofuranosyl)pyrazolo[3,4-d]pyrimidines and Their Salts

The above-identified compounds were prepared from the 5'-azides (Examples 205-221) by catalytic hydrogenation as described in Examples 15-20 (method A) or triphenylphosphine followed by ammonium hydroxide as described in Examples 82-83 (method B). The salts were prepared by standard methods.

The compounds listed in Table XXIII were be prepared by these methods:

TABLE XXIII

| GP1-# | Example | 3- | 4- | Method | Salt | M.P.(°C) |
|---|---|---|---|---|---|---|
| 515 | 222 | Br | $NH_2$ | B | HCl | >230° |
| 614 | 223 | H | $NH_2$ | A | HBr | 260-165° |
| 625 | 224 | $CH_2CN$ | $NH_2$ | B | —1 | 175°(d) |
| 642 | 225 | ϕ | $NH_2$ | A | HCl | 218-219° |
| 682 | 226 | 2-thienyl | $NH_2$ | B | HCl | >220° |
| 694 | 227 | 4-$CH_3O$ϕ | $NH_2$ | A | --- | 222-225° |
| 701 | 228 | 4-Clϕ | $NH_2$ | B | HCl | 189-194° |
| 704 | 229 | ϕ | $NH_ϕ$ | A | $CF_3CO_2H$ | 185-190° |

1/ Not a salt.

EXAMPLES 230-231

General Procedure for the Preparation of 4-Amino- and 4-Arylamino-1-(5-amino-2,3-O-diacetyl-5-deoxy-1-β-D-ribofuranosyl)-3-substitutedpyrazolo [3,4-d]pyrimidines

A slurry of 10% Pd-C in a solution (MeOH or EtOH with THF, dioxane or EtOAc) of the 5'-azido-2',3'-diacetate nucleoside (Examples 191-198) is hydrogenated in a Parr shaker at 40 psi. After disappearance of the starting material (TLC), the mixture is filtered and concentrated under vacuum at a temperature of less than 40°C. The residual product is purified by recrystallization or HPLC.

The compounds listed in Table XXIV may be prepared by this method:

TABLE XXIV

| GP1-# | Example | D | F | $C_1$,$C_2$ | M.P.(°C) |
|---|---|---|---|---|---|
| --- | 230 | Br | $NH_2$ | OAc | --- |
| --- | 231 | ϕ | NHϕ | OAc | --- |

EXAMPLES 232-233

General Procedure for the Preparation of 3-Substituted-4-(1,1-dicarboethoxyalkyl)-1-(2,3,5-O-tribenzoyl-1-β-D-ribofuranosyl)pyrazolo [3,4-d]pyrimidines

To a stirred solution of the diethyl(alkyl)malonate (0.10 mol) in dry DMF (100 ml) under $N_2$ was added 80% NaH in mineral oil (0.125 mol). After stirring (cooling) for 10 minutes, a solution of the 3-substituted-4-chloro-1-(2,3,5-tribenzoyl-1-β-D-ribofuranosyl)pyrazolo[3,4-d]pyrimidine (0.09 mol) (Example 178) in DMF (75 ml) was added dropwise. The solution was cooled and anhydrous trimethylamine was bubbled into the solution for 4 minutes. The solution was stirred for 3 hours at room temperature (heating may be required in some cases) then quenched with dilute acetic acid. The mixture was extracted with ether-ethyl acetate (9:1) and the organic extract dried ($Na_2S_4$), concentrated and pumped under vacuum. The residue was chromatographed on $SiO_2$ gel with $CH_2Cl_2$-acetone mixtures and the appropriate fractions combined and evaporated to yield the title compounds. The identity of the compounds were confirmed by $^1H$ and $^{13}C$ NMR.

The compounds listed in Table XXV were prepared by this procedure:

TABLE XXV

| Example | D | F | M.P. (°C) |
|---|---|---|---|
| 232 | Br | $CH(CO_2C_2H_5)_2$ | foam |
| 233 | Br | Cϕ$(CO_2C_2H_5)_2$ | foam |

EXAMPLES 234-235

General Procedure for Preparation of 4-Alkyl, 4-Arylalkyl- and 3,4-disubstituted-1-(1-β-D-ribofuranosyl)pyrazolo[3,4-d] pyrimidines

The diester (Examples 232-233) was dissolved in aqueous ethanolic sodium hydroxide and heated. The solution was neutralized with acetic acid, evaporated, extracted with hot ethanol and the extract then evaporated and recrystallized or evaporated on $SiO_2$ gel. The $SiO_2$ gel was loaded on a column of $SiO_2$ gel and the product eluted with $CH_2Cl_2$-MeOH mixtures. The appropriate fractions more combined and evaporated to yield the title compounds.
The compounds described in Table XXVI were prepared by the procedure:

TABLE XXVI

| GP-1-# | Example | D | F | M.P.(°C) |
|---|---|---|---|---|
| 719 | 234 | Br | $CH_3$ | 204-205° |
| | 235 | Br | $CH_2\phi$ | ----- |

EXAMPLES 236-237

General Procedure for Preparation of 3-Substituted-4-(1,1-dicarboethoxyalkyl)-1-(5-azido-5-deoxy-2,3-O-diacetyl-1-β-D-ribofuranosyl)pyrazolo[3,4-d]pyrimidines

The above-identified compounds are prepared analogously by the procedure described in Examples 232-233 using the 3-substituted-(5-azido-5-deoxy-2,3-O-diacetyl-1-β-D-ribofuranosyl)-4-chloropyrazolo[3,4-d]pyrimidine.
The compounds listed in Table XXVII may be prepared by this procedure:

TABLE XXVII

| Example | D | F | M.P. (°C) |
|---|---|---|---|
| 236 | Br | $C\phi(CO_2C_2H_5)_2$ | ---- |
| 237 | Br | $CH(CO_2C_2H_5)_2$ | ---- |

EXAMPLES 238-239

General Procedure for Preparation of 4-Alkyl-, 4-Phenylalkyl-and 4-Substituted-3-Substituted-1-(5-azido-5-deoxy-1-β-D-ribofuranosyl)pyrazolo[3,4-d]pyrimidines

The above identified compounds are prepared analogously by the procedure described in Examples 234-235 from the 5'-azide esters described in Examples 236-237.
The following compounds listed in Table XXVIII may be prepared by this procedure:

TABLE XXVIII

| GP1-# | Example | D | F | M.P.(°C) |
|---|---|---|---|---|
| --- | 238 | Br | $CH_3$ | ----- |
| --- | 239 | Br | $CH_2\phi$ | ----- |

EXAMPLES 240-241

General Procedure for the Preparation of 4-Alkyl-, 4-Phenylalkyl- and 3,4-Disubstituted-1-(5-amino-5-deoxy-1-β-D-ribofuranosyl) pyrazolo[3,4-d]pyrimidines

The above-identified compounds may be prepared by reduction of the 5-azido ribosides listed in Examples 238-239 by catalytic hydrogenation as described in Examples 15-20 or by treatment with triphenylphosphine and ammonium hydroxide as described in Examples 182-185.
The compounds listed in Table XXIX are prepared by this procedure:

TABLE XXIX

| GP1-# | Example | D | F | M.P.(°C) |
|---|---|---|---|---|
| ---- | 240 | Br | $CH_3$ | ----- |
| ---- | 241 | Br | $CH_2\phi$ | ----- |

EXAMPLES 242-243

General Procedures for the Preparation of 3-Substituted-1-(5-deoxy-2,3-O-diacetyl-1-β-D-ribofuranosyl)-4-(1,1-dicarboethoxyalkyl)pyrazolo[3,4-d]pyrimidines

The above-identified compounds are prepared analogously to the procedure described in Examples 232-233 using 3-substituted-4-chloro-1-(5-deoxy-2,3-O-diacetyl-1-β-D-ribofuranosyl)pyrazolo[3,4-d]pyrimidines (Examples 198-203).

The compounds listed in Table XXX may be prepared by this procedure:

TABLE XXX

| GP1-# | Example | D | F | M.P.(°C) |
|---|---|---|---|---|
| --- | 242 | Br | $CH(CO_2C_2H_5)_2$ | ---- |
| --- | 243 | Br | $C(CO_2C_2H_5)_2CH\phi$ | ----- |

EXAMPLES 244-245

General Procedure for Preparation of 4-Alkyl-, 4-Phenylalkyl-, or 4-Substituted-3-substituted-1-(5-deoxy-1-β-D-ribofuranosyl)pyrazolo[3,4-d]pyrimidines

The above-identified compounds may be prepared from the esters (Examples 242-243) using the procedure described in Examples 234-235.

The compounds listed in Table XXXI may be prepared by this method:

TABLE XXXI

| GP1-# | Example | D | F | M.P. (°C) |
|---|---|---|---|---|
| ---- | 244 | Br | $CH_2CH_2\phi$ | ---- |
| ---- | 245 | Br | $CH_2\phi$ | ---- |

By following the procedures described in the Detailed Description of the Invention and Examples 1 to 245 and using the appropriate starting materials and reagents, the following compounds are made:
4-Amino-7-(5-deoxy-1-β-D-ribofuranosyl)-5-vinylpyrrolo[2,3-d]pyrimidine;
4-Amino-5-ethynyl-7-(5-deoxy-1-β-D-ribofuranosyl)pyrrolo[2,3-d]pyrimidine;
5-(2-Chlorophenyl)-7-(5-deoxy-1-β-D-ribofuranosyl)-4-phenylaminopyrrolo[2,3-d]pyrimidine;
5-(3-Chlorophenyl)-7-(5-deoxy-1-β-D-ribofuranosyl)-4-phenylaminopyrrolo[2,3-d]pyrimidine;
5-(4-Chlorophenyl)-7-(5-deoxy-1-β-D-ribofuranosyl)-4-phenylaminopyrrolo[2,3-d]pyrimidine;
5-(2-Methoxyphenyl)-7-(5-deoxy-1-β-D-ribofuranosyl)-4-phenylaminopyrrolo[2,3-d]pyrimidine;
5-(4-Methoxyphenyl)-7-(5-deoxy-1-β-D-ribofuranosyl)-4-phenylaminopyrrolo[2,3-d]pyrimdine;
5-(2-Furanyl)-7-(5-deoxy-1-β-D-ribofuranosyl)-4-phenylaminopyrrolo[2,3-d]pyrimidine;
7-(5-Deoxy-1-β-D-ribofuranosyl)-4-phenyamino-5-(2-pyridyl)pyrrolo[2,3-d]pyrimidine;
7-(5-Deoxy-1-β-D-ribofuranosyl)-4-phenylamino-5-(4-pyridyl)pyrrolo[2,3-d]pyrimidine.
7-(5-Deoxy-1-β-D-ribofuranosyl)-5-phenyl-4-(4-pyridylamino)pyrrolo[2,3-d]pyrimidine;
7-(5-Deoxy-1-β-D-ribofuranosyl)-5-phenyl-4-(2-pyridylamino)-pyrrolo[2,3-d]pyrimidine;
7-(5-Deoxy-1-β-D-ribofuranosyl)-5-phenyl-4-(1-piperazinyl)-pyrrolo[2,3-d]pyrimidine;
4-(2-Chlorophenyl)-7-(5-deoxy-1-β-D-ribofuranosyl)-5-phenylpyrrolo[2,3-d]pyrimidine;
4-(3-Chlorophenyl)-7-(5-deoxy-1-β-D-ribofuranosyl)-5-phenylpyrrolo[2,3-d]pyrimidine;
7-(5-Deoxy-1-β-D-ribofuranosyl)-5-phenyl-4-(2-thiazolyl-amino)pyrrolo[2,3-d]pyrimidine;
4-Cyclohexylamino-7-(5-deoxy-1-β-D-ribofuranosyl)-5-phenylpyrrolo[2,3-d]pyrimidine;
7-(5-Deoxy-1-β-D-ribofuranosyl)-5-phenyl-4-phenylthio-pyrrolo[2,3-d]pyrimidine;

4-Benzyl-7-(5-deoxy-1-β-D-ribofuranosyl)-5-phenyl-pyrrolo[2,3-d]pyrimidine;
7-(5-Deoxy-1-β-D-ribofuranosyl)-4-ethynyl-5-phenyl-pyrrolo[2,3-d]pyrimidine;
7-(5-Deoxy-1-β-D-ribofuranosyl)-4-methyl-5-phenyl-pyrrolo[2,3-d]pyrimidine;
4-Benzyl-7-(5-deoxy-1-β-D-ribofuranosyl)-5-iodopyrrolo[2,3-d]pyrimidine;
7-(5-Deoxy-1-β-D-ribofuranosyl)-5-iodo-4-methyl-pyrrolo[2,3-d]pyrimidine;
7-(5-Deoxy-1-β-D-ribofuranosyl)-5-phenyl-4-phenylamino-pyrrolo[2,3-d]pyrimidine;
4-Amino-5-phenyl-7-(1-β-D-ribofuranosyl)pyrrolo[2,3-d]-pyrimidine;
4-Amino-7-(5-deoxy-5-mercapto-1-β-D-ribofuranosyl)-5-iodopyrrolo[2,3-d]pyrimidine;
7-(5-Deoxy-5-mercapto-1-β-D-ribofuranosyl)-5-iodo-4-phenylaminopyrrolo[2,3-d]pyrimidine;
7-(5-Deoxy-5-mercapto-1-β-D-ribofuranosyl)-5-phenyl-4-phenylaminopyrrolo[2,3-d]pyrimidine;
7-(5-Amino-5-deoxy-1-β-D-ribofuranosyl)-5-phenyl-4-phenylaminopyrrolo[2,3-d]pyrimidine;
7-(5,6-dideoxy-1-β-D-allofuranosyl)-5-iodo-4-phenylamino-pyrrolo[2,3-d]pyrimidine;
7-(5,6-Dideoxy-1-β-D-allofuranosyl)-5-phenyl-4-phenylalminopyrrolo[2,3-d]pyrimidine;
4-Amino-7-(5,6-dideoxy-1-β-D-allofuranosyl)-5-phenyl-pyrrolo[2,3-d]pyrimidine;
4-Amino-7-(5-deoxy-5-fluoro-1-β-D-ribofuranosyl)-5-iodopyrrolo[2,3-d]pyrimidine;
4-Amino-7-(5-deoxy-5-chloro-1-β-D-ribofuranosyl)-pyrrolo[2,3-d]pyrimidine;
7-(5-Deoxy-5-fluoro-1-β-D-ribofuranosyl)-5-phenyl-4-phenylaminopyrrolo[2,3-d]pyrimidine;
4-Amino-7-(6-azido-5,6-dideoxy-1-β-D-allofuranosyl)-5-iodopyrrolo[2,3-d]pyrimidine;
7-(6-Azido-5,6-dideoxy-1-β-D-allofuranosyl)-5-phenyl-4-phenylaminopyrrolo[2,3-d]pyrimidine;
4-Amino-7-(6-amino-5,6-dideoxy-1-β-D-allofuranosyl)-5-iodopyrrolo[2,3-d]pyrimidine;
7-(6-Amino-5,6-dideoxy-1-β-D-allofuranosyl)-5-phenyl-4-phenylaminopyrrolo[2,3-d]pyrimidine;
5-(2-Methoxyphenyl)-7-[1-β-D-ribofuranosyl]-4-phenylamino-pyrrolo[2,3-d]pyrimidine;
4-Amino-5-bromo-7-(5,6-didehydro-5,6-dideoxy-1-β-D-allofuranosyl)pyrrolo[2,3-d]pyrimidine;
7-(5,6-Didehydro-5,6-dideoxy-1-β-D-allofuranosyl)-5-phenyl-4-phenylaminopyrrolo[2,3-d]pyrimidine;
4-Amino-1-(5-amino-5-deoxy-1-β-D-ribofuranosyl)-3-methoxy-pyrazolo[3,4-d]pyrimidine;
4-Amino-1-(5-amino-5-deoxy-1-β-D-ribofuranosyl)-3-phenoxy-pyrazolo[3,4-d]pyrimidine;
4-Amino-1-(5-amino-5-deoxy-1-β-D-ribofuranosyl)-3-phenylthiopyrazolo[3,4-d]pyrimidine;
4-Amino-1-(5-amino-5-deoxy-1-β-D-ribofuranosyl)-3-methylthiorpyrazolo[3,4-d]pyrimidine;
4-Amino-1-(5-amino-5-deoxy-1-β-D-ribofuranosyl)-3-chloro-pyrazolo[3,4-d]pyrimidine;
4-Amino-1-(5-amino-5-deoxy-1-β-D-ribofuranosyl)-3-cyclopropylpyrazolo[3,4-d]pyrimidine;
4-Amino-1-(5-amino-5-deoxy-1-β-D-ribofuranosyl)-3-dimethylamino-pyrazolo[3,4-d]pyrimidine;
4-Amino-1-(5-amino-5-deoxy-1-β-D-ribofuranosyl)-3-fluoropyrazolo[3,4-d]pyrimidine;
4-Amino-1-(5-amino-5-deoxy-1-β-D-ribofuranosyl)-3-(3-pyridyl)pyrazolo[3,4-d]pyrimidine;
1-(5-Amino-5-deoxy-1-β-D-ribofuranosyl)-4-(3-chlorophenyl)-3-(4-methoxyphenyl)pyrazolo[3,4-d]pyrimidine;
1-(5-Amino-5-deoxy-1-β-D-ribofuranosyl)-4-(4-chlorophenyl)-3-(4-methoxyphenyl)pyrazolo[3,4-d]pyrimidine;
1-(5-Amino-5-deoxy-1-β-D-ribofuranosyl)-4-(4-ethoxyphenyl)-3-(4-methoxyphenyl)pyrazolo[3,4-d]pyrimidine;
1-(5-Amino-5-deoxy-1-β-D-ribofuranosyl)-4-(3-carboxamidophenyl-amino)-3-(4-methoxyphenyl)pyrazolo[3,4-d]pyrimidine;
1-(5-Amino-5-Deoxy-1-β-D-ribofuranosyl)-4-(2-furanyl)-3-(4-methoxyphenyl)pyrazolo[3,4-d]pyrimidine;
1-(5-Deoxy-1-β-D-ribofuranosyl)-3-(4-methoxyphenyl)-4-(phenylamino)pyrazolo[3,4-d]pyrimidine;
1-(5-Deoxy-1-β-D-ribofuranosyl)-3-(3-methoxyphenyl)-4-(phenylamino)pyrazolo[3,4-d]pyrimidine;
1-(5-Deoxy-1-β-D-ribofuranosyl)-3-(2-pyridyl)-4-(phenylamino)pyrazolo[3,4-d]pyrimidine;
1-(5-Deoxy-1-β-D-ribofuranosyl)-3-(4-methoxyphenyl)-4-(4-pyridylamino)pyrazolo[3,4-d]pyrimidine;
1-(5-Deoxy-1-β-D-ribofuranosyl)-3-(3-methoxyphenyl)-4-(4-pyridylamino)pyrazolo[3,4-d]pyrimidine;
1-(5-Deoxy-1-β-D-ribofuranosyl)-3-(2-pyridyl)-4-(4-pyridylamino)pyrazolo[3,4-d]pyrimidine;
1-(5-deoxy-1-β-D-ribofuranosyl)-3-(4-methoxyphenyl)-4-(2-methoxyphenylamino)pyrazolo[3,4-d]pyridimine;
1-(5-Deoxy-1-β-D-ribofuranosyl)-3-(3-methoxyphenyl)-4-(2-methoxyphenylamino)pyrazolo[3,4-d]pyridimine;
1-(5-Deoxy-1-β-D-ribofuranosyl)-3-(4-pyridyl)-4-(2-methoxyphenylamino)pyrazolo[3,4-d]pyridimine;
1-(5-Deoxy-1-β-D-ribofuranosyl)-3-(4-methoxyphenyl)-4-(2-imidazolylamino)pyrazolo[3,4-d]pyrimidine;
1-(5-Deoxy-1-β-D-ribofuranosyl)-3-(3-methoxyphenyl)-4-(2-imidazolylamino)pyrazolo[3,4-d]pyrimidine;
1-(5-Deoxy-1-β-D-ribofuranosyl)-3-(2-pyridyl)-4-(2-imidazolylamino)pyrazolo[3,4-d]pyrimidine;
1-(5-Deoxy-1-β-D-ribofuranosyl)-3-(2-pyrazinyl)-4-phenylaminopyrazolo[3,4-d]pyrimidine;
1-(5-Deoxy-1-β-D-ribofuranosyl)-3-(2-pyrazinyl)-4-(N-indolinyl)pyrazolo[3,4-d]pyrimidine;
1-(5,6-Dideoxy-1-β-D-allofuranosyl)-3-phenyl-4-phenylaminopyrazolo[3,4-d]pyrimidine;
4-Amino-1-(5,6-dideoxy-1-β-D-allofuranosyl)-3-iodopyrazolo[3,4-d]pyrimidine;
1-(5-Deoxy-1-β-D-ribofuranosyl)-3-phenyl-4-phenylthiopyrazolo[3,4-d]pyrimidine;
1-(5-Amino-5-deoxy-1-β-D-ribopuranosyl)-3-bromo-4-methylpyrazolo[3,4-d]pyrimidine;
1-(5-Amino-5-deoxy-1-β-D-ribofuranosyl)-4-methyl-3-iodopyrazolo[3,4-d]pyrimidine;

7-(5-Deoxy-β-D-ribofuranosyl)-5-iodo-4-methylpyrrolo[2,3-d]pyrimidine;
4-Methyl-3-phenyl-1-(1-β-D-ribofuranosyl)pyrazolo[3,4-d]pyrimidine;
1-(5-Deoxy-1-β-D-ribofuranosyl)-3-phenyl-4-(phenylmethyl)pyrazolo[3,4-d]pyrimidine; and
7-(5-Amino-5-deoxy-1-β-D-ribofuranosyl)-5-bromo-4-chloropyrrolo [2,3-d]pyrimidine(GP-1-608).

EXAMPLE A

INHIBITION OF ADENOSINE KINASE ACTIVITY

Inhibition of enzyme activity was determined using a 0.1 ml assay mixture containing 50 mM Tris-maleate, pH 7.0, 0.1% (w/v) BSA, 1 mM ATP, 1 mM $MgCl_2$, 0.5 μM [U-$^{14}$C] adenosine (500 mCi/mmol) and 0.1 μg of purified pig heart adenosine kinase. Different concentrations of the test compounds were incubated in the assay mixture for 20 min at 37°C. From each reaction mixture, 20 μl portions were removed and spotted on 2 $cm^2$ pieces of Whatman DE81 filter paper. The papers were then washed to remove [$^{14}$C] adenosine in 1mM ammonium formate followed by deionized water and finally 95% ethanol. The papers were dried, and [$^{14}$C]AMP measured by scintillation counting. Activities were determined from the amount of [$^{14}$C]AMP formed. This is a suitable in vitro test method for determining adenosine kinase enzyme inhibition activity.

$A_1$ receptor binding affinity was determined using 0.5 ml mixture containing 50 mM Tris HCl, pH 7.4, 1 nM [$^3$H]CHA and 0.5 mg of neuronal membrane incubated with different concentrations of the test compound for 60 min at 37°C. The reaction was stopped and unbound [$^3$H]CHA removed by rapid filtration through Whatman GF/B filters. The filter papers were then solubilized and bound [$^3$H]CHA determined by scintillation counting.

Inhibition of adenosine deaminase activity was determined spectrophotometrically using a 1 ml assay mixture containing 50mM potassium phosphate, pH 7.0, 1 mM ADP, 2.5 mM alpha-ketoglutarate, 15 units glutamic dehydrogenase, 0.125 mM NADH, 80 M adenosine and 0.002 units of calf intestinal mucosa adenosine deaminase. Different concentrations of the test compounds were incubated in the assay mixture for 10 min at 37°C. The reaction was monitored continuously for oxidation of NADH from the change in absorbance at 340nm.

Illustrative of the invention, the compounds designated GP-1-515, GP-1-608, GP-1-683, GP-1-695, GP-1-718, GP-1-704, GP-1-665, and GP-1-667, were found to have an $IC_{50}$ of less than 10 nM in the adenosine kinase inhibition assay. The compound GP-1-515 was found to be much less potent in the $A_1$ receptor assay and in the adenosine deaminase inhibition assay, having an $IC_{50}$ greater than 100 μM in the $A_1$ receptor assay and an $IC_{50}$ greater than 1000 μM in the adenosine deaminase inhibition assay.

EXAMPLE B

ADENOSINE KINASE INHIBITION IN INTACT CELLS

Inhibition of adenosine kinase in intact cells was determined from the amount of incorporation of radioisotope from adenosine into the adenylates (AMP, ADP and ATP) in the presence of adenosine deaminase inhibition. Capillary endothelial cells from bovine heart were incubated for 60 min. With 20 μM 2'-deoxycoformycin, a potent adenosine deaminase inhibitor. Different concentrations of the test compounds were then added to the cells and incubated for 15 min. after which 5 μM [$^3$H]adenosine was added and the cells incubated for a further 15 min. The media was then discarded and the cells were treated with 50 μl 0.4 M perchloric acid, centrifuged and the supernatants neutralized with 100 μl alanine: freon (1:4). Radiosotope-labelled adenylates were separated by TLC on PEI cellulose plates developed in methanol:water (1:1) and incorporation of $^3$H determined by scintillation counting.

Illustrative of the invention, the compounds designated GP-1-515, GP-1-683 and GP-1-665 were shown to have an $IC_{50}$ of 9nM, 73 nM and 4.5 nM, respectively, in the adenosine kinase inhibition assay in intact cells.

EXAMPLE C

IMPROVED FUNCTIONAL RECOVERY IN ISOLATED HEARTS

The ability of a number of adenosine kinase inhibitors to improve the recovery of post-ischemic function was examined in an isolated guinea pig heart model.

Isolated guinea pig hearts were cannulated via the ascending aorta and attached to a perfusion apparatus according to the method of Langendorff. The hearts were perfused at a constant pressure of 60 cm of $H_2O$ with a modified Krebs-Hanseleit buffer (pH 7.4) at 37°C. Left ventricular developed pressures (LVDP) were monitored continuously using a latex balloon attached to a pressure transducer. Coronary flows were measured gravimetrically by timed collection of pulmonary effluent. Following equilibration of the hearts for a period of 30 minutes, the hearts were

subjected to 45 minutes of low flow ischemia, by reducing the perfusion pressure to 10 cm of $H_2O$, and then reperfused for 30 minutes by restoring the pressure to its. original level (60 cm of $H_2O$). The adenosine kinase inhibitors GP-1-238, GP-1-515 and GP1-547 were added to the perfusion buffer at the final concentrations specified. The results of these experiments are shown in Table A and demonstrate that adenosine kinase inhibitors enhance recovery of post ischemic function without affecting basal coronary flow.

TABLE A

| GP-1-# | Conc.( M) | Functional Recovery (% preischemic LVDP) | Preischemic Flow (ml/min/g) |
|---|---|---|---|
| Control (n=14) | --- | 66.0 ± 2.1 | 5.9 ± 0.2 |
| 238 (n=6) | 5 | 81.3 ± 3.3* | 5.8 ± 0.3 |
| 515 (n-6) | 0.3 | 79.0 ± 3.4* | 6.5 ± 0.3 |
| 547 | 0.3 | 79.0 ± 3.1* | 6.2 ± 0.3 |

* $p < 0.05$ vs. Control
Conc. = Concentration of test compound added to perfusion medium.

EXAMPLE D

EFFECT OF ADENOSINE KINASE INHIBITION ON ACUTE I.V. HEMODYNAMICS IN THE RAT

The ability of the adenosine kinase inhibitor GP-1-238 to show effects on blood pressure, heart rate or body temperature was compared in anesthetized and conscious rats. Sprague Dawley rats were anesthetized with pentobarbital and catheterized in the jugular vein and carotid artery. GP-1-238 (0.1-5 mg/kg/min) was infused intravenously in stepwise increments (0.2 ml/min x 5 minutes). The experiments in conscious rats were conducted in the same manner after rats had been catheterized and allowed to recover for 2 days following surgery. In conscious rats, in contrast to anesthetized animals, no hemodynamic effects were seen at doses which completely inhibited adenosine kinase in vivo See Figure 1.

EXAMPLE E

FUNCTIONAL BENEFIT OF GP-1-515 IN A PRECLINICAL MODEL OF STABLE ANGINA

GP-1-515, was evaluated for its ability to prevent cumulative cardiac dysfunction associated with repeated episodes of pacing-induced ischemia.

Anesthetized male dogs were instrumented to measure regional myocardial wall thickening during right atrial pacing (Young & Mullane, Am. J. Physio., **1991**, 261:1570-1577). Animals were subjected to six repeated episodes of pacing. A continuous IV infusion of 1 g/kg/min of GP-1-515 or saline (control) -was administered post pace #1. The results of these experiments are shown in Table B and reveal that the adenosine kinase inhibitor, GP-1-515, attenuates the decline in wall thickening associated with pacing induced ischemia.

TABLE B

| % of Non-Ischemic Wall Thickening | | |
|---|---|---|
| Pace # | Saline (n=3) | GP-1-515 (n=4) |
| 1 | 33.3 ± 9.3 | 39.8 ± 3.2 |
| 2 | 40.5 ± 11 | 43.6 ± 12.0 |
| 3 | 27.4 ± 16 | 34.0 ± 19.0 |
| 4 | 19.2 ± 23 | 50.5 ± 21.3 |
| 5 | 22.6 ± 16 | 75.6 ± 19.4 |
| 6 | 13.7 ± 19 | 62.6 ± 17.1 |

EXAMPLE F

EFFICACY OF GP-1-515 IN A PRECLINICAL MODEL OF UNSTABLE ANGINA

GP-1-515 was tested in a canine model of platelet-induced coronary thrombosis (Folts, J., Circulation, **1991**, 83[Suppl. IV]:IV-3-IV-14). In this model, platelets cyclically aggregate and embolize causing cyclic flow reductions

(CFRs) that are quantitated by the frequency and the change in coronary blood flow from low point to peak with each cycle. The results are shown in Table C. Administration of the adenosine kinase inhibitor, GP-1-515, abolished CFRs in 3 of 8 animals and reduced both the frequency and the change in flow in those animals where CFRs were not abolished.

TABLE C

| | Control | 0.03 | 0.1 | 0.3 |
|---|---|---|---|---|
| CFR (freq.) | 5.2 ± 0.2 (n=8) | 3.0 ± 0.6 (n=7) | 3.4 ± 0.8 (n=5) | 4.2 ± 0.6 (n=5) |
| CFR (D flow) | 17 ± 1.7 (n=8) | 10.1 ± 2.6 (n=7) | 11.0 ± 2.1 (n=5) | 14.1 ± 1.9 (n=5) |
| N abolished / N total | 0/8 | 1/8 | 3/8 | 3/8 |

EXAMPLE G

INHIBITION OF NEUTROPHIL ADHERENCE TO FIBROBLASTS OR ENDOTHELIAL CELLS

The ability of an adenosine kinase inhibitor to affect neutrophil adherence to fibroblasts and endothelial cells was evaluated in a cell culture model.

Cultures of human dermal fibroblasts or human umbillical vein endothelial cells were washed and then incubated for 2 hours at 37°C in a 5% $CO_2$ atmosphere in fresh medium containing different concentrations of the adenosine kinase inhibitors GP-1-272 and GP-1-456. These incubations were carried out in the presence of fMLP-stimulated human neutrophils isolated from whole blood (1.25 x $10^6$/ml) with or without adenosine deaminase (0.125 U/ml). At the end of the incubation, the medium was removed and the monolayers of fibroblasts or endothelial cells and adherent neutrophils were fixed by addition of formaldehyde (3.7%) and, after washing to remove non-adherent neutrophils, adherent neutrophils were stained with Weigart's hematoxylin and counted under a light microscope. The results depicted in Figure 2 show that the adenosine kinase inhibitors GP-1-272 and GD-1-456 inhibit neutrophil adhesion to endothelial cells and that this inhibition is reversed by adenosine deaminase treatment.

EXAMPLE H

INHIBITION OF CONTRACTION IN ISOLATED ILEUM

The ability of adenosine kinase inhibition to affect stimulated contraction of muscle strips from the isolated ileum has been investigated.

Segments (about 1 cm) of longitudinal muscle were stripped from the guinea pig ileum, connected to isotonic force transducers and suspended in jacketed tissue baths containing Krebs-Ringer solution aerated with 95% $O_2$/5% $CO_2$. Parallel platinum electrodes were used to deliver electrical current at 1 minute intervals at a voltage adequate to induce contraction of 90% of maximal. The adenosine kinase inhibitors, GP-1-515 or GP-1-547 were added to the tissue baths at different concentrations, with or without either the adenosine receptor antagonist, 8-sulphophenyltheophylline (8SPT) or adenosine deaminase (ADA), and the effects on contraction monitored.

Inhibition of contraction by (A) GP-1-515 and (B) GP-1-547 together with reversal by both 8SPT and ADA are shown in Figure 3.

EXAMPLE I

INHIBITION OF SEIZURES BY ADENOSINE KINASE INHIBITORS

The ability of selected adenosine kinase inhibitors to influence PTZ-induced seizures was evaluated in an experimental animal model.

Male Swiss Webster mice in groups of 6-8 were preinjected intraperitoneally (IP) with either vehicle or an adenosine kinase inhibitor followed 1 hour later by 100 mg/kg pentylenetetrazol (PTZ) administered subcutaneously in the upper back of the animal. After injections of the convulsant, animals were isolated in separate cages and observed for the onset of seizure. Animals were scored as being fully protected from seizure if they failed to seize for a period of 1 hour after PTZ administration (vehicle control animals seized after about 4 minutes). The results are shown in Figure 4.

In Panel A, the adenosine kinase inhibitor, GP-1-456, was administered IP at doses of 1, 5 and 10 mg/kg followed after 45 minutes by PTZ administration.

In Panel B, GP-1-456 was administered IP at a dose of 2.5 mg/kg alone or together with either 34 mg/kg of theophylline or 70 mg/kg of 8-sulfophenyltheophylline followed after 45 minutes by PTZ administration.

In additional studies, mice were given GP-1-456 or GP-1-560 by oral administration 45 minutes prior to PTZ administration. The results are given in Table D and demonstrate that the adenosine kinase inhibitors exhibit oral anticonvulsant -activity.

The ability of the adenosine kinase inhibitors to influence electroshock-induced seizures was also evaluated in an experimental model.

The adenosine kinase inhibitor GP-1-456 or vehicle were administered orally to rats prior to electroshock treatment. After 1 hour, corneal electrodes were applied to the eyes of each animal and an electrical stimulus of 160 mA delivered for 0.2 seconds. Animals were isolated in separate cages and observed for seizure. Abolition of the hindleg tonic extensor component is taken as the end point for this test and reflects ability of the compound to prevent seizure spread.

From the data presented in Table E, an $ED_{50}$ of 0.18 mg/kg was determined for protection of electroshock-induced seizures by the adenosine kinase inhibitor, GP-1-456.

TABLE D

| Treatment | N= | Percent Seizure | Seizure Score | Latency To First Seizure (minutes) |
|---|---|---|---|---|
| Control | 13 | 100 | 3.6±1.2 | 3.3 |
| GP-1-456 (2.5 mg/kg) | 13 | 15 | 0.3±0.4* | 8.2* |
| Control | 8 | 100 | 2.5 ±1.6 | 4.5 |
| GP-1-560 (30 mg/kg) | 7 | 71 | 0.71±0.5 | 4.5 |
| GP-1-560 (50 mg/kg) | 7 | 43 | 1.0±0.9 | 7.0* |

* $p < 0.05$ vs. Control

Data is presented as the mean ± standard deviation.

TABLE E

| GP-1-456 (mg/kg) | No. of Animals (protected/tested) |
|---|---|
| 0.00 | 0/8 |
| 0.05 | 1/8 |
| 0.10 | 2/8 |
| 0.15 | 2/8 |
| 0.19 | 6/8 |
| 0.38 | 6/8 |
| 0.75 | 7/8 |
| 1.50 | 8/8 |

## Claims

### Claims for the following Contracting States : AT, BE, CH, LI, DE, DK, FR, GB, IT, LU, MC, NL, PT, SE

**1.** A compound which is a pyrimidine derivative of the formula:

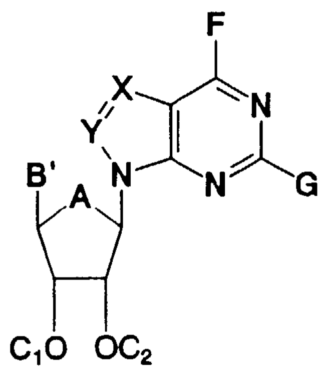

wherein:

(a) A is oxygen, methylene or sulfur;
(b) B' is $-(CH_2)_n-B$ wherein n is 1, 2, 3 or 4 and B is hydrogen, alkyl, alkoxy, amino, alkylamino, acylamino, hydrocarbyloxycarbonylamino, mercapto, alkylthio, azido, cyano, halogen, or B' is alkenyl or alkynyl;
(c) $C_1$ and $C_2$ are each independently hydrogen, acyl, hydrocarbyloxycarbonyl or taken together form a 5-membered ring wherein $C_1$ is a single bond to $C_2$ and $C_2$ is carbonyl or α-alkoxyalkylidene;
(d) X is

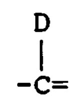

and Y is -N= or

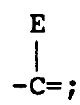

(e) D is hydrogen, halogen, alkyl, aryl, aralkyl, alkenyl, alkynyl, haloalkyl, cyano, cyanoalkyl, acyl, carboxamido, a carboxylic acid or carboxylic acid ester group, alkoxy, aryloxy, aralkyloxy, alkylthio, arylthio, aralkylthio, amino, alkylamino, arylamino, aralkylamino, acylamino, or nitro;
(f) E is hydrogen, halogen, alkyl, or alkylthio;
(g) F is alkyl, aryl, aralkyl, halogen, amino, alkylamino, arylamino, aralkylamino, cyano, cyanoalkyl, alkoxy, aryloxy, aralkoxy, alkylthio, arylthio, aralkylthio; optionally substituted indolinyl or indolyl; pyrrolidinyl or piperazinyl; and
(h) G is hydrogen, halogen, lower alkyl, lower alkoxy, lower alkylamino or lower alkylthio; or a pharmaceutically acceptable salt thereof; with the proviso that: when A is oxygen and
(i) X is

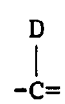

and Y is

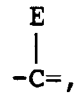

then if B' is methyl, D is halogen, cyano or carboxamido, and F is amino, then G is not hydrogen; or if D is hydrogen, then F is not amino; or

(ii) X is

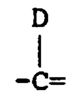

and Y is -N=, if B is hydrogen or halogen, D and G are hydrogen, then F is not amino; or when A is methylene, X is

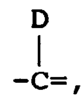

Y is

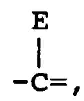

B, D, E and G are hydrogen, then F is not amino.

**2.** A compound which is a pyrimidine derivative of the formula:

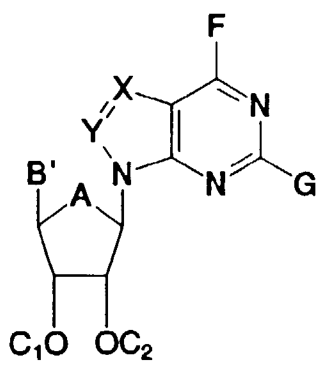

wherein:

(a) A is oxygen, methylene or sulfur;

(b) B' is $-(CH_2)_n-B$ wherein n is 1, 2, 3 or 4 and B is hydroxy, acyloxy, hydrocarbyloxycarbonyloxy, or $-OCONR_2$ wherein each R is independently hydrogen or hydrocarbyl;

(c) $C_1$ and $C_2$ are each independently hydrogen, acyl, hydrocarbyloxycarbonyl or taken together form a 5-membered ring wherein $C_1$ is a single bond to $C_2$ and $C_2$ is carbonyl or α-alkoxyalkylidene;

(d) X is

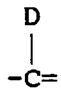

and Y is -N=;

(e) D is halogen, aryl or aralkyl;

(f) F is alkyl, aryl, aralkyl, halogen, amino, alkylamino, arylamino, aralkylamino, cyano, cyanoalkyl, alkoxy, aryloxy, aralkoxy, alkylthio, arylthio, aralkylthio, optionally substituted indolinyl or indolyl, pyrrolidinyl or piperazinyl; and

(g) G is hydrogen, halogen, lower alkyl, lower alkoxy or lower alkylthio; or a pharmaceutically acceptable salt thereof;

with the proviso that when A is oxygen and D is halogen, then F is not amino.

**3.** A compound which is a pyrimidine derivative of the formula:

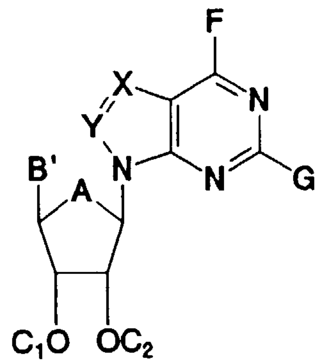

wherein:

(a) A is oxygen, methylene or sulfur;

(b) B' is $-(CH_2)_n$-B wherein n is 1, 2, 3 or 4 and B is hydroxy, acyloxy, hydrocarbyloxycarbonyloxy, or $-OCONR_2$ wherein each R is independently hydrogen or hydrocarbyl;

(c) $C_1$ and $C_2$ are each independently hydrogen, acyl, hydrocarbyloxycarbonyl or taken together form a 5-membered ring wherein $C_1$ is a single bond to $C_2$ and $C_2$ is carbonyl or α-alkoxyalkylidene;

(d) X is

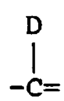

and Y is

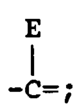

(e) D is aryl or aralkyl;

(f) E is hydrogen, halogen, alkyl or alkylthio;

(g) F is alkyl, aryl, aralkyl, halogen, amino, alkylamino, arylamino, aralkylamino, cyano, cyanoalkyl, alkoxy, aryloxy, aralkyloxy, alkylthio, arylthio, aralkylthio, optionally substituted indolinyl or indolyl, pyrrolidinyl or piperazinyl; and

(h) G is hydrogen, halogen, lower alkyl, lower alkoxy, or lower alkylthio;

or a pharmaceutically acceptable salt thereof;

with the proviso that: when A is oxygen, D is oxadiazolyl, triazolyl or triazinyl, and E and G are both hydrogen, then F is not amino.

**4.** A compound according to any one of claims 1 to 3, in which A is oxygen.

**5.** A compound according to any one of claims 1 to 4, in which F is halogen or an amino, alkylamino, arylamino, aralkylamino, alkylthio, arylthio, alkyl, aryl or aralkyl group.

**6.** A compound according to any one of claims 1 to 5, in which G is hydrogen.

**7.** A compound as claimed in any one of claims 1 to 6, for use in a method of medical treatment by surgery, therapy or diagnosis on the human or animal body.

**8.** A compound as claimed in claim 7, for use in enhancing adnosine levels in a mammal.

**9.** A compound according to claim 7, for use in a method of enhancing local levels of adenosine around cells having an undesirably low ratio of synthesis of adenosine triphosphate/breakdown of adenosine triphosphate.

**10.** A compound according to claim 7, for use in a method of treating or preventing a cardiovascular disorder in a mammal where injury or dysfunction causes or is caused by ischemia, reperfusion or arrhythmia.

**11.** A compound according to claim 7, for use as adjunctive therapy with a procedure in a mammal to protect the heart.

**12.** A compound according to claim 7, for use in a method of preventing or decreasing reperfusion injury to a tissue or organism in a mammal following interrupted or decreased blood flow to that tissue or organism.

**13.** A compound according to claim 7, for use in a method of preventing or decreasing platelet aggregation in a mammal.

**14.** A compound according to claim 7, for use in a method of preventing or decreasing inflammatory responses in a mammal.

**15.** A compound according to claim 7, for use in a method of treating a patient having a chronic low or insufficient adenosine level or who would benefit from an increased central nervous system adenosine level.

**16.** A compound according to claim 7, for use in a method of producing skeletal muscle relaxation or preventing skeletal muscle spasm in a mammal.

**17.** A compound according to claim 7, for use in a method of encouraging a sleep state in a patient.

**18.** A compound according to claim 7, for use in a method of treating anxiety.

**19.** A compound according to claim 7, for use in a method of reducing or preventing neural tissue damage caused by excitotoxicity in a mammal.

**20.** A compound according to claim 7, for use in a method of decreasing neural tissue damage associated with a neurodegenerative disease in an animal.

**21.** A compound according to claim 7, for use in a method of treating or preventing epilepsy or seizures in a mammal.

**22.** A compound according to claim 7, for use in a method of decreasing local contraction of smooth muscle in a mammal.

**23.** A compound according to any one of claims 8, 12 or 19, for use in the treatment of stroke.

**24.** A compound according to claim 14, wherein the inflammatory responses causes arthritis, meningitis, autoimmune disease, inflammatory bowel disease, vasculitis, dermatitis, myositis or renal inflammation.

**25.** Use of a compound having a pyrrolo[2,3-d]pyrimidine skeleton or a pyrazole[3,4-d]pyrimidine skeleton and having an $IC_{50}$ of 1 micromolar or less in an *in vitro* adenosine kinase enzyme inhibition test method in the manufacture of a medicament for use in a method of treatment as defined in any one of claims 8 to 24.

**26.** Use of a compound as claimed in any one of claims 1 to 6 in the manufacture of a medicament for use in a method of treatment as defined in any one of claims 8 to 24.

**27.** Use according to claim 25 or claim 26, in which the medicament is for use as an anti-inflammatory agent.

**28.** Use as claimed in claim 27 in which the compound shows at least a 20% reduction of total mean hind paw edema at a dose of 300 mg/kg day or less in an *in vivo* inflammation test method.

**Claims for the following Contracting States : ES, GR**

**1.** A process for preparing a compound which is a pyrimidine derivative of the formula:

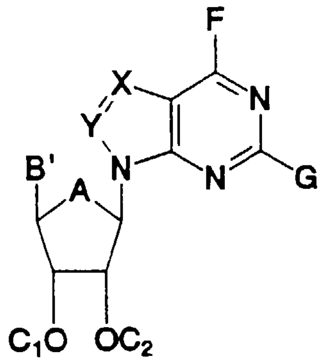

wherein:

(a) A is oxygen, methylene or sulfur;
(b) B' is $-(CH_2)_n-B$ wherein n is 1, 2, 3 or 4 and B is hydrogen, alkyl, alkoxy, amino, alkylamino, acylamino, hydrocarbyloxycarbonylamino, mercapto, alkylthio, azido, cyano, halogen, or B' is alkenyl or alkynyl;
(c) $C_1$ and $C_2$ are each independently hydrogen, acyl, hydrocarbyloxycarbonyl or taken together form a 5-membered ring wherein $C_1$ is a single bond to $C_2$ and $C_2$ is carbonyl or α-alkoxyalkylidene;
(d) X is

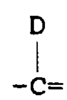

and Y is -N= or

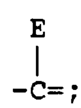

(e) D is hydrogen, halogen, alkyl, aryl, aralkyl, alkenyl, alkynyl, haloalkyl, cyano, cyanoalkyl, acyl, carboxamido, a carboxylic acid or carboxylic acid ester group, alkoxy, aryloxy, aralkyloxy, alkylthio, arylthio, aralkylthio, amino, alkylamino, arylamino, aralkylamino, acylamino, or nitro;
(f) E is hydrogen, halogen, alkyl, or alkylthio;

(g) F is alkyl, aryl, aralkyl, halogen, amino, alkylamino, arylamino, aralkylamino, cyano, cyanoalkyl, alkoxy, aryloxy, aralkoxy, alkylthio, arylthio, aralkylthio; optionally substituted indolinyl or indolyl; pyrrolidinyl or piperazinyl; and

(h) G is hydrogen, halogen, lower alkyl, lower alkoxy, lower alkylamino or lower alkylthio;

or a pharmaceutically acceptable salt thereof; with the proviso that: when A is oxygen and

(i) X is

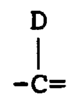

and Y is

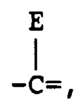

then if B' is methyl, D is halogen, cyano or carboxamido, and F is amino, then G is not hydrogen; or if D is hydrogen, then F is not amino; or

(ii) X is

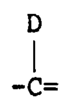

and Y is -N=, if B is hydrogen or halogen, D and G are hydrogen, then F is not amino; or when A is methylene, X is

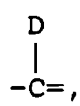

Y is

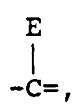

B, D, E and G are hydrogen, then F is not amino which process comprises reacting a compound of the formula:

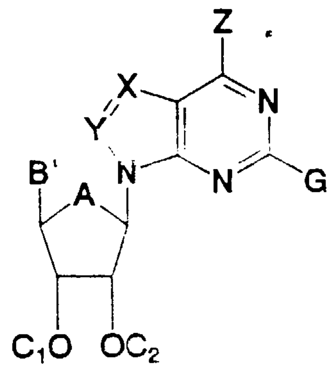

in which Z is a halogen and A, B', $C_1$, $C_2$, G, and X are as defined above or are in a protected form, and Y is -N=, with an appropriate nucleophilic agent and, if necessary, deprotecting the product to form a compound of formula I in which Y is -N=;

or which process comprises reacting a compound of the formula:

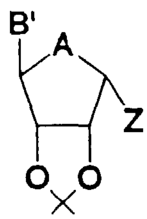

where A and B' are as defined above and Z is a halogen, with a compound of the formula:

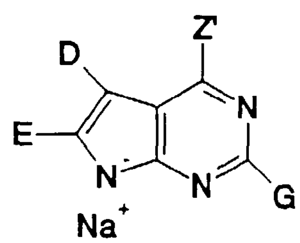

where E, D and G are as defined above and Z' is a halogen to give a compound of the formula:

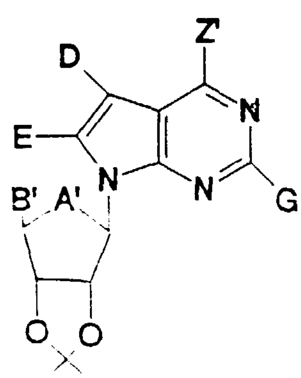

and deprotecting the compound to provide a compound of formula I in which Y is

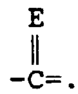

2. A process for preparing a compound which is a pyrimidine derivative of the formula:

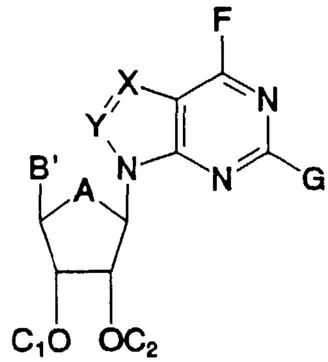

wherein:

(a) A is oxygen, methylene or sulfur;
(b) B' is $-(CH_2)_n-B$ wherein n is 1, 2, 3 or 4 and B is hydroxy, acyloxy, hydrocarbyloxycarbonyloxy, or $-OCONR_2$ wherein each R is independently hydrogen or hydrocarbyl;
(c) $C_1$ and $C_2$ are each independently hydrogen, acyl, hydrocarbyloxycarbonyl or taken together form a 5-membered ring wherein $C_1$ is a single bond to $C_2$ and $C_2$ is carbonyl or α-alkoxyalkylidene;
(d) X is

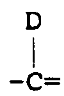

and Y is -N=;
(e) D is halogen, aryl or aralkyl
(f) F is alkyl, aryl, aralkyl, halogen, amino, alkylamino, arylamino, aralkylamino, cyano, cyanoalkyl, alkoxy, aryloxy, aralkoxy, alkylthio, arylthio, aralkylthio, optionally substituted indolinyl or indolyl, pyrrolidinyl or piperazinyl; and
(g) G is hydrogen, halogen, lower alkyl, lower alkoxy or lower alkylthio;

or a pharmaceutically acceptable salt thereof; with the proviso that when A is oxygen and D is halogen, then F is not amino which process comprises reacting a compound of the formula:

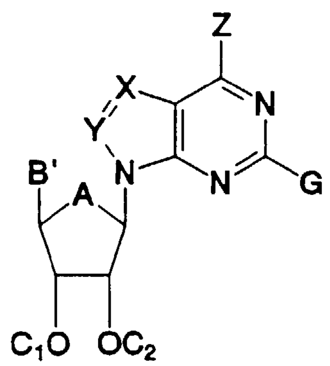

in which Z is a halogen and A, B', $C_1$, $C_2$, G, X and Y are as defined above or are in a protected form, with an appropriate nucleophilic agent and, if necessary, deprotecting the product to form a compound of formula I.

**3.** A process for preparing a compound which is a pyrimidine derivative of the formula:

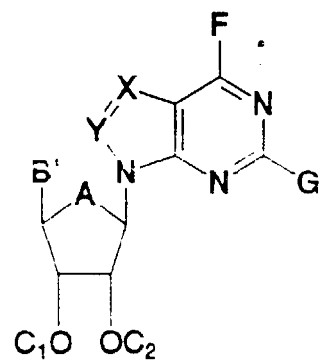

wherein:

(a) A is oxygen, methylene or sulfur;
(b) B' is $-(CH_2)_n-B$ wherein n is 1, 2, 3 or 4 and B is hydroxy, acyloxy, hydrocarbyloxycarbonyloxy, or $-OCONR_2$ wherein each R is independently hydrogen or hydrocarbyl;
(c) $C_1$ and $C_2$ are each independently hydrogen, acyl, hydrocarbyloxycarbonyl or taken together form a 5-membered ring wherein $C_1$ is a single bond to $C_2$ and $C_2$ is carbonyl or α-alkoxyalkylidene;
(d) X is

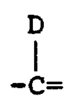

and Y is

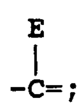

(e) D is aryl or aralkyl;
(f) E is hydrogen, halogen, alkyl or alkylthio;

(g) F is alkyl, aryl, aralkyl, halogen, amino, alkylamino, arylamino, aralkylamino, cyano, cyanoalkyl, alkoxy, aryloxy, aralkyloxy, alkylthio, arylthio, aralkylthio, optionally substituted indolinyl or indolyl, pyrrolidinyl or piperazinyl; and

(h) G is hydrogen, halogen, lower alkyl, lower alkoxy, or lower alkylthio;

or a pharmaceutically acceptable salt thereof;

with the proviso that: when A is oxygen, D is oxadiazolyl, triazolyl or triazinyl, and E and G are both hydrogen, then F is not amino which process comprises reacting a compound of the formula:

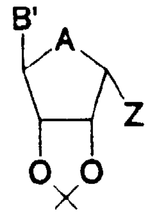

where A and B' are as defined above and Z is a halogen, with a compound of the formula:

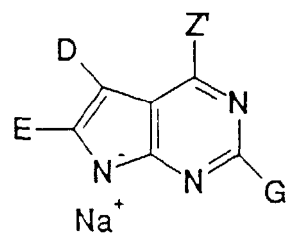

where E, D and G are as defined above and Z' is a halogen, to aive a compound of the formula:

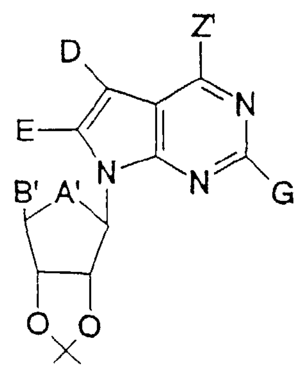

and deprotecting the compound to provide a compound of formula I.

**4.** A process according to any one of claims 1 to 3, in which A is oxygen.

**5.** A process according to any one of claims 1 to 4, in which F is halogen or an amino, alkylamino, arylamino, aralkylamino, alkylthio, arylthio, alkyl, aryl or aralkyl group.

**6.** A process according to any one of claims 1 to 5, in which G is hydrogen.

**7.** Use of a compound as claimed in any one of claims 1 to 6, in the manufacture of a medicament for use in a method of medical treatment by surgery, therapy or diagnosis on the human or animal body.

**8.** Use of a compound as claimed in claim 7, in the manufacture of a medicament for use in enhancing adenosine

levels in a mammal.

**9.** Use of a compound according to claim 7, in the manufacture of a medicament for use in a method of enhancing local levels of adenosine around cells having an undesirably low ratio of synthesis of adenosine triphosphate/ breakdown of adenosine triphosphate.

**10.** Use of a compound according to claim 7, in the manufacture of a medicament for use in a method of treating or preventing a cardiovascular disorder in a mammal where injury or dysfunction causes or is caused by ischemia, reperfusion or arrhythmia.

**11.** Use of a compound according to claim 7, in the manufacture of a medicament for use as adjunctive therapy with a procedure in a mammal to protect the heart.

**12.** Use of a compound according to claim 7, in the manufacture of a medicament for use in a method of preventing or decreasing reperfusion injury to a tissue or organism in a mammal following interrupted or decreased blood flow to that tissue or organism.

**13.** Use of a compound according to claim 7, in the manufacture of a medicament for use in a method of preventing or decreasing platelet aggregation in a mammal.

**14.** Use of a compound according to claim 7, in the manufacture of a medicament for use in a method of preventing or decreasing inflammatory responses in a mammal.

**15.** Use of a compound according to claim 7, in the manufacture of a medicament for use in a method of treating a patient having a chronic low or insufficient adenosine level or who would benefit from an increased central nervous system adenosine level.

**16.** Use of a compound according to claim 7, in the manufacture of a medicament for use in a method of producing skeletal muscle relaxation or preventing skeletal muscle spasm in a mammal.

**17.** Use of a compound according to claim 7, in the manufacture of a medicament for use in a method of encouraging a sleep state in a patient.

**18.** Use of a compound according to claim 7, in the manufacture of a medicament for use in a method of treating anxiety.

**19.** Use of a compound according to claim 7, in the manufacture of a medicament for use in a method of reducing or preventing neural tissue damage caused by excitotoxicity in a mammal.

**20.** Use of a compound according to claim 7, in the manufacture of a medicament for use in a method of decreasing neural tissue damage associated with a neurodegenerative disease in an animal.

**21.** Use of a compound according to claim 7, in the manufacture of a medicament for use in a method of treating or preventing epilepsy or seizures in a mammal.

**22.** Use of a compound according to claim 7, in the manufacture of a medicament for use in a method of decreasing local contraction of smooth muscle in a mammal.

**23.** Use of a compound according to any one of claims 9, 11 or 19, in the manufacture of a medicament for use in the treatment of stroke.

**24.** Use of a compound according to claim 14, wherein the inflammatory responses cause arthritis, meningitis, autoimmune disease, inflammatory bowel disease, vasculitis, dermatitis, myositis or renal inflammation.

**25.** Use of a compound having a pyrrolo[2,3-d]pyrimidine skeleton or a pyrazolo[3,4-d]pyrimidine skeleton having an $IC_{50}$ of about 1 micromolar or less in an *in vitro* adenosine kinase enzyme inhibition test method in the manufacture of a medicament for use in a method of treatment as defined in any one of claims 8 to 24.

**26.** Use of a compound as claimed in any one of claims 1 to 6 in the manufacture of a medicament for use in a method

of treatment as defined in any one of claims 8 to 24.

**27.** A compound which is a pyrimidine derivative of the formula:

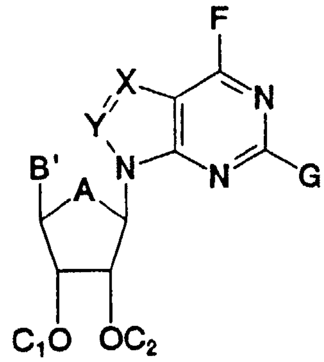

wherein:

(a) A is oxygen, methylene or sulfur;
(b) B' is $-(CH_2)_n-B$ wherein n is 1, 2, 3 or 4 and B is hydrogen, alkyl, alkoxy, amino, alkylamino, acylamino, hydrocarbyloxycarbonylamino, mercapto, alkylthio, azido, cyano, halogen, or B' is alkenyl or alkynyl;
(c) $C_1$ and $C_2$ are each independently hydrogen, acyl, hydrocarbyloxycarbonyl or taken together form a 5-membered ring wherein $C_1$ is a single bond to $C_2$ and $C_2$ is carbonyl or α-alkoxyalkylidene;
(d) X is

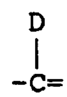

and Y is -N= or

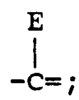

(e) D is hydrogen, halogen, alkyl, aryl, aralkyl, alkenyl, alkynyl, haloalkyl, cyano, cyanoalkyl, acyl, carboxamido, a carboxylic acid or carboxylic acid ester group, alkoxy, aryloxy, aralkyloxy, alkylthio, arylthio, aralkylthio, amino, alkylamino, arylamino, aralkylamino, acylamino, or nitro;
(f) E is hydrogen, halogen, alkyl, or alkylthio;
(g) F is alkyl, aryl, aralkyl, halogen, amino, alkylamino, arylamino, aralkylamino, cyano, cyanoalkyl, alkoxy, aryloxy, aralkoxy, alkylthio, arylthio, aralkylthio; optionally substituted indolinyl or indolyl; pyrrolidinyl or piperazinyl; and
(h) G is hydrogen, halogen, lower alkyl, lower alkoxy, lower alkylamino or lower alkylthio; or a pharmaceutically acceptable salt thereof; with the proviso that: when A is oxygen and

(i) X is

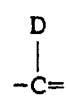

and Y is

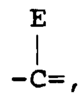

then if B' is methyl, D is halogen, cyano or carboxamido, and F is amino, then G is not hydrogen; or if D is hydrogen, then F is not amino; or
(ii) X is

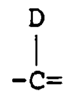

and Y is -N=, if B is hydrogen or halogen, D and G are hydrogen, then F is not amino; or when A is methylene, X is

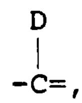

Y is

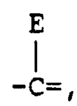

B, D, E and G are hydrogen, then F is not amino.

## Patentansprüche

### Patentansprüche für folgende Vertragsstaaten : AT, BE, CH, LI, DE, DK, FR, GB, IT, LU, MC, NL, PT, SE

1. Verbindung, welche ein Pyrimidinderivat folgender Formel ist:

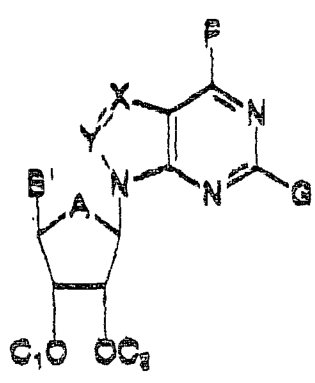

worin:

(a) A Sauerstoff, Methylen oder Schwefel ist;
(b) B' $-(CH_2)_n-$B ist, worin n 1, 2, 3 oder 4 ist und B Wasserstoff, Alkyl, Alkoxy, Amino, Alkylamino, Acylamino, Hydrocarbyloxycarbonylamino, Mercapto, Alkyl-thio, Azido, Cyano, Halogen ist, oder B' Alkenyl oder Alkinyl ist;
(c) $C_1$ und $C_2$ jeweils unabhängig Wasserstoff, Acyl, Hydrocarbyloxycarbonyl sind oder zusammengenommen einen 5-gliedrigen Ring bilden, worin $C_1$ eine Einfachbindung zu $C_2$ ist und $C_2$ Carbonyl oder $\alpha$-Alkoxyalkyliden ist;
(d) X

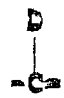

ist und Y -N= oder

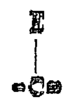

ist;
(e) D Wasserstoff, Halogen, Alkyl, Aryl, Aralkyl, Alkenyl, Alkinyl, Halogenalkyl, Cyano, Cyanoalkyl, Acyl, Carboxamido, eine Carbonsäure- oder eine Carbonsäureestergruppe, Alkoxy, Aryloxy, Aralkyloxy, Alkylthio, Arylthio, Aralkylthio, Amino, Alkylamino, Arylamino, Aralkylamino, Acylamino oder Nitro ist;
(f) E Wasserstoff, Halogen, Alkyl oder Alkylthio ist;
(g) F Alkyl, Aryl, Aralkyl, Halogen, Amino, Alkylamino, Arylamino, Aralkylamino, Cyano, Cyanoalkyl, Alkoxy, Aryloxy, Aralkoxy, Alkylthio, Arylthio, Aralkylthio; wahlweise substituiertes Indolinyl oder Indolyl; Pyrrolidinyl oder Piperazinyl ist; und
(h) G Wasserstoff, Halogen, Niederalkyl, Niederalkoxy, Niederalkylamino oder Niederalkylthio ist;

oder ein pharmazeutisch annehmbares Salz davon; mit der Maßgabe, daß; wenn A Sauerstoff ist und

(i) X

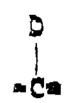

ist und Y

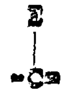

ist, dann, wenn B' Methyl ist, D Halogen, Cyano oder Carboxamido ist und F Amino ist, dann G nicht Wasserstoff ist; oder, wenn D Wasserstoff ist, dann F nicht Amino ist; oder

(ii) X

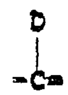

ist und Y -N= ist, wenn B Wasserstoff oder Halogen ist, D und G Wasserstoff sind, dann F nicht Amino ist; oder wenn A Methylen ist, X

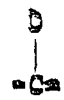

ist, Y

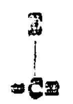

ist, B, D, E und G Wasserstoff sind, dann F nicht Amino ist.

**2.** Verbindung, welche ein Pyrimidinderivat folgender Formel ist:

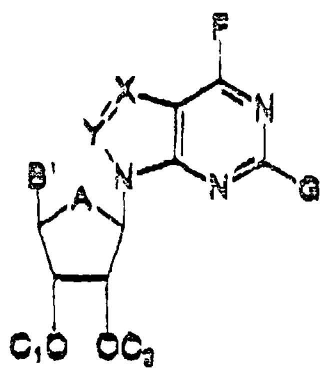

worin:

(a) A Sauerstoff, Methylen oder Schwefel ist;
(b) B' $-(CH_2)_n-B$ ist, worin n 1, 2, 3 oder 4 ist und B Hydroxy, Acyloxy, Hydrocarbyloxycarbonyloxy oder $-OCONR_2$ ist, worin jedes R unabhängig Wasserstoff oder Hydrocarbyl ist;
(c) $C_1$ und $C_2$ jeweils unabhängig Wasserstoff, Acyl, Hydrocarbyloxycarbonyl sind oder zusammengenommen einen 5-gliedrigen Ring bilden, worin $C_1$ eine Einfachbindung zu $C_2$ ist und $C_2$ Carbonyl oder α-Alkoxyalkyliden ist;
(d) X

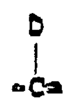

ist und Y -N = ist;
(e) D Halogen, Aryl oder Aralkyl ist;
(f) F Alkyl, Aryl, Aralkyl, Halogen, Amino, Alkylamino, Arylamino, Aralkylamino, Cyano, Cyanoalkyl, Alkoxy,

Aryloxy, Aralkoxy, Alkylthio, Arylthio, Aralkylthio, wahlweise substituiertes Indolinyl oder Indolyl, Pyrrolidinyl oder Piperazinyl ist; und

(g) G Wasserstoff, Halogen, Niederalkyl, Niederalkoxy oder Niederalkylthio ist;

oder ein pharmazeutisch annehmbares Salz davon; mit der Maßgabe, daß: wenn A Sauerstoff ist und D Halogen ist, dann F nicht Amino ist.

**3.** Verbindung, welche ein Pyrimidinderivat folgender Formel ist:

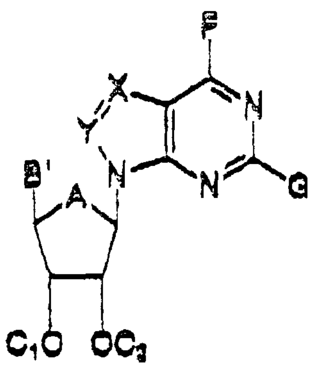

worin:

(a) A Sauerstoff, Methylen oder Schwefel ist;

(b) B' $-(CH_2)_n$-B ist, worin n 1, 2, 3 oder 4 ist und B Hydroxy, Acyloxy, Hydrocarbyloxycarbonyloxy oder $-OCONR_2$ ist, worin jedes R unabhängig Wasserstoff oder Hydrocarbyl ist;

(c) $C_1$ und $C_2$ jeweils unabhängig Wasserstoff, Acyl, Hydrocarbyloxycarbonyl sind oder zusammengenommen einen 5-gliedrigen Ring bilden, worin $C_1$ eine Einfachbindung zu $C_2$ ist und $C_2$ Carbonyl oder $\alpha$-Alkoxyalkyliden ist;

(d) X

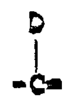

ist und Y

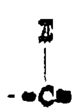

ist;

(e) D Aryl oder Aralkyl ist;

(f) E Wasserstoff, Halogen, Alkyl oder Alkylthio ist;

(g) F Alkyl, Aryl, Aralkyl, Halogen, Amino, Alkylamino, Arylamino, Aralkylamino, Cyano, Cyanoalkyl, Alkoxy, Aryloxy, Aralkyloxy, Alkylthio, Arylthio, Aralkylthio, wahlweise substituiertes Indolinyl oder Indolyl, Pyrrolidinyl oder Piperazinyl ist; und

(h) G Wasserstoff, Halogen, Niederalkyl, Niederalkoxy oder Niederalkylthio ist;

oder ein pharmazeutisch annehmbares Salz davon; mit der Maßgabe, daß: wenn A Sauerstoff ist, D Oxadiazolyl, Triazolyl oder Triazinyl ist und E und G beide Wasserstoff sind, dann F nicht Amino ist.

**4.** Verbindung gemäß mindestens einem der Ansprüche 1 bis 3, worin A Sauerstoff ist.

5. Verbindung gemäß mindestens einem der Ansprüche 1 bis 4, worin F Halogen oder eine Amino-, Alkylamino-, Arylamino-, Aralkylamino-, Alkylthio-, Arylthio-, Alkyl-, Aryl- oder Aralkylgruppe ist.

6. Verbindung gemäß mindestens einem der Ansprüche 1 bis 5, worin G Wasserstoff ist.

7. Verbindung gemäß mindestens einem der Ansprüche 1 bis 6 zur Verwendung bei einem Verfahren der medizinischen Behandlung durch chirurgischen Eingriff, Therapie oder Diagnose beim menschlichen oder tierischen Körper.

8. Verbindung gemäß Anspruch 7 zur Verwendung bei der Steigerung von Adenosinspiegel in einem Säuger.

9. Verbindung gemäß Anspruch 7 zur Verwendung bei einem Verfahren der Steigerung lokaler Spiegel an Adenosin um Zellen mit einem unerwünscht niedrigen Verhältnis von Synthese an Adenosintriphosphat/Abbau an Adenosintriphosphat.

10. Verbindung gemäß Anspruch 7 zur Verwendung bei einem Verfahren der Behandlung oder Vorbeugung bzw. Verhinderung einer kardiovaskulären Störung in einem Säuger, wo eine Verletzung oder Dysfunktion Ischämie, Reperfusion oder Arrhythmie verursacht oder durch diese verursacht wird.

11. Verbindung gemäß Anspruch 7 zur Verwendung als unterstützende Therapie mit einer Vorgehensweise in einem Säuger zum Schutz des Herzens.

12. Verbindung gemäß Anspruch 7 zur Verwendung bei einem Verfahren zur Verhinderung oder Verringerung von Reperfusionsverletzung bei einem Gewebe oder Organisumus in einem Säuger, gefolgt von unterbrochenem oder vermindertem Blutfluß zu dem Gewebe oder Organismus.

13. Verbindung gemäß Anspruch 7 zur Verwendung bei einem Verfahren zur Verhinderung oder Verringerung von Blutplättchenaggregation in einem Säuger.

14. Verbindung gemäß Anspruch 7 zur Verwendung bei einem Verfahren zur Verhinderung oder Verringerung von Entzündungsreaktionen in einem Säuger.

15. Verbindung gemäß Anspruch 7 zur Verwendung bei einem Verfahren zur Behandlung eines Patienten mit einem chronisch niedrigen oder unzureichenden Adenosinspiegel, oder welcher aus einem erhöhten Adenosinspiegel des Zentralen Nervensystems Nutzen ziehen würde.

16. Verbindung gemäß Anspruch 7 zur Verwendung bei einem Verfahren zur Erzeugung von Skelettmuskel-Relaxation oder zur Verhinderung von Skelettmuskel-Spasmus in einem Säuger.

17. Verbindung gemäß Anspruch 7 zur Verwendung bei einem Verfahren zur Förderung eines Schlafzustandes bei einem Patienten.

18. Verbindung gemäß Anspruch 7 zur Verwendung bei einem Verfahren zur Behandlung von Angst.

19. Verbindung gemäß Anspruch 7 zur Verwendung bei einem Verfahren zur Verringerung oder Verhinderung von durch Excitotoxizität verursachter Nervengewebeschädigung in einem Säuger.

20. Verbindung gemäß Anspruch 7 zur Verwendung bei einem Verfahren zur Verringerung von mit einer neurodegenerativen Erkrankung in Verbindung stehender Nervengewebeschädigung in einem Tier.

21. Verbindung gemäß Anspruch 7 zur Verwendung bei einem Verfahren zur Behandlung oder Verhinderung von Epilepsie oder Anfällen bei einem Säuger.

22. Verbindung gemäß Anspruch 7 zur Verwendung bei einem Verfahren der Senkung der lokalen Kontraktion glatter Muskulatur in einem Säuger.

23. Verbindung gemäß mindestens einem der Ansprüche 8, 12 oder 19 zu Verwendung bei der Behandlung eines Schlaganfalles.

**24.** Verbindung gemäß Anspruch 14, wobei die Entzündungsreaktionen Arthritis, Meningitis, Autoimmunerkrankung, Darmentzündungserkrankung, Vasculitis, Dermatitis, Myositis oder Nierenentzündung verursachen.

**25.** Verwendung einer Verbindung mit einem Pyrrolo[2,3-d]pyrimidin-Gerüst oder einem Pyrazolo[3,4-d]pyrimidin-Gerüst und einem $IC_{50}$-Wert von 1 mikromolar oder weniger in einem *in vitro*-Adenosin-Kinaseenzym-Inhibitionstestverfahren bei der Herstellung eines Medikamentes zur Verwendung bei einem Behandlungsverfahren, wie in mindestens einem der Ansprüch 8 bis 24 definiert.

**26.** Verwendung einer Verbindung gemäß mindestens einem der Ansprüche 1 bis 6 bei der Herstellung eines Medikamentes zur Verwendung bei einem Behandlungsverfahren, wie in mindestens einem der Ansprüch 8 bis 24 definiert.

**27.** Verwendung gemäß Anspruch 25 oder Anspruch 26, wobei das Medikament zur Verwendung als ein entzündungshemmendes Mittel ist.

**28.** Verwendung gemäß Anspruch 27, wobei die Verbindung mindestens eine 20%ige Verringerung des gesamten mittleren Rückpfotenödems bei einer Dosis von 300 mg/kg pro Tag oder weniger bei einem *in vivo*-Entzündungstestverfahren zeigt.

**Patentansprüche für folgende Vertragsstaaten : ES, GR**

**1.** Verfahren zur Herstellung einer Verbindung, welche ein Pyrimidinderivat folgender Formel ist:

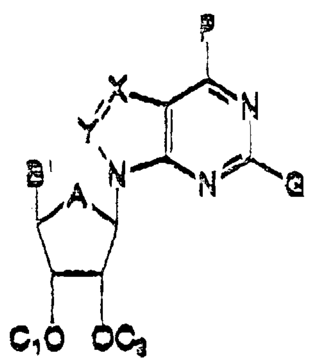

worin;

(a) A Sauerstoff, Methylen oder Schwefel ist;
(b) B' $-(CH_2)_n$-B ist, worin n 1, 2, 3 oder 4 ist und B Wasserstoff, Alkyl, Alkoxy, Amino, Alkylamino, Acylamino, Hydrocarbyloxycarbonylamino, Mercapto, Alkylthio, Azido, Cyano, Halogen ist, oder B' Alkenyl oder Alkinyl ist;
(c) $C_1$ und $C_2$ jeweils unabhängig Wasserstoff, Acyl, Hydrocarbyloxycarbonyl sind oder zusammengenommen einen 5-gliedrigen Ring bilden, worin $C_1$ eine Einfachbindung zu $C_2$ ist und $C_2$ Carbonyl oder α-Alkoxyalkyliden ist;
(d) X

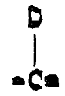

ist und Y -N= oder

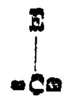

ist;

(e) D Wasserstoff, Halogen, Alkyl, Aryl, Aralkyl, Alkenyl, Alkinyl, Halogenalkyl, Cyano, Cyanoalkyl, Acyl, Carboxamido, eine Carbonsäure- oder eine Carbonsäureestergruppe, Alkoxy, Aryloxy, Aralkyloxy, Alkylthio, Arylthio, Aralkylthio, Amino, Alkylamino, Arylamino, Aralkylamino, Acylamino oder Nitro ist;

(f) E Wasserstoff, Halogen, Alkyl oder Alkylthio ist;

(g) F Alkyl, Aryl, Aralkyl, Halogen, Amino, Alkylamino, Arylamino, Aralkylamino, Cyano, Cyanoalkyl, Alkoxy, Aryloxy, Aralkoxy, Alkylthio, Arylthio, Aralkylthio; wahlweise substituiertes Indolinyl oder Indolyl; Pyrrolidinyl oder Piperazinyl ist; und

(h) G Wasserstoff, Halogen, Niederalkyl, Niederalkoxy, Niederalkylamino oder Niederalkylthio ist;

oder ein pharmazeutisch annehmbares Salz davon; mit der Maßgabe, daß: wenn A Sauerstoff ist und

(i) X

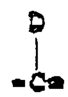

ist und Y

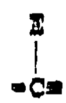

ist, dann, wenn B' Methyl ist, D Halogen, Cyano oder Carboxamido ist und F Amino ist, dann G nicht Wasserstoff ist; oder, wenn D Wasserstoff ist, dann F nicht Amino ist; oder

(ii) X

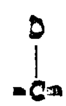

ist und Y -N= ist, wenn B Wasserstoff oder Halogen ist, D und G Wasserstoff sind, dann F nicht Amino ist; oder wenn A Methylen ist, X

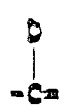

ist, Y

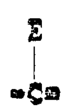

ist, B, D, E und G Wasserstoff sind, dann F nicht Amino ist, wobei das Verfahren die Umsetzung einer Ver-

bindung der folgenden Formel:

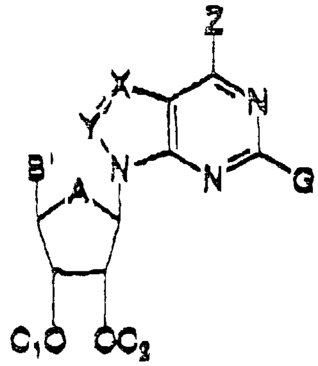

worin Z ein Halogen ist und A, B', $C_1$, $C_2$, G und X wie oben definiert sind oder in einer geschützten Form vorliegen und Y - N = ist;
mit einem geeigneten nukleophilen Mittel, und, sofern erforderlich, das Entschützen des Produktes zur Bildung einer Verbindung von Formel I, worin Y - N = ist, umfaßt;
oder wobei das Verfahren das Umsetzen einer Verbindung der Formel:

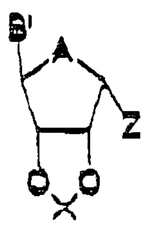

,

worin A und B' wie oben definiert sind und Z ein Halogen ist, mit einer Verbindung der Formel:

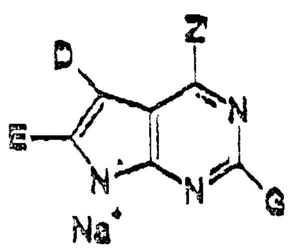

,

worin E, D und G wie oben definiert sind und Z' ein Halogen ist, zum Erhalt einer Verbindung der Formel:

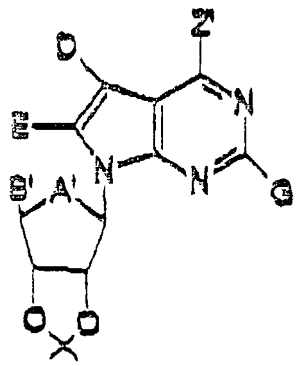

und das Entschützen der Verbindung zur Bereitstellung einer Verbindung der Formel I, worin Y

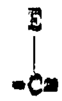

ist, umfäßt.

**2.** Verfahren zur Herstellung einer Verbindung, welche ein Pyrimidinderivat folgender Formel ist:

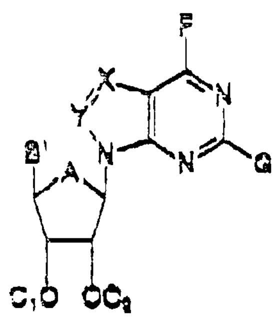

worin:

(a) A Sauerstoff, Methylen oder Schwefel ist;
(b) B' $-(CH_2)_n-B$ ist, worin n 1, 2, 3 oder 4 ist und B Hydroxy, Acyloxy, Hydrocarbyloxycarbonyloxy oder $-OCONR_2$ ist, worin jedes R unabhängig Wasserstoff oder Hydrocarbyl ist;
(c) $C_1$ und $C_2$ jeweils unabhängig Wasserstoff, Acyl, Hydrocarbyloxycarbonyl sind oder zusammengenommen einen 5-gliedrigen Ring bilden, worin $C_1$ eine Einfachbindung zu $C_2$ ist und $C_2$ Carbonyl oder $\alpha$-Alkoxyalkyliden ist;
(d) X

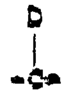

ist und Y -N = ist;
(e) D Halogen, Aryl oder Aralkyl ist;
(f) F Alkyl, Aryl, Aralkyl, Halogen, Amino, Alkylamino, Arylamino, Aralkylamino, Cyano, Cyanoalkyl, Alkoxy, Aryloxy, Aralkoxy, Alkylthio, Arylthio, Aralkylthio, wahlweise substituiertes Indolinyl oder Indolyl, Pyrrolidinyl oder Piperazinyl ist; und

(g) G Wasserstoff, Halogen, Niederalkyl, Niederalkoxy oder Niederalkylthio ist;

oder ein pharmazeutisch annehmbares Salz davon; mit der Maßgabe, daß: wenn A Sauerstoff ist und D Halogen ist, dann F nicht Amino ist, wobei das Verfahren das Umsetzen einer Verbindung der folgenden Formel:

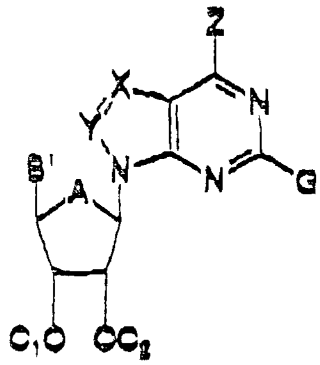

worin Z ein Halogen ist und A, B', $C_1$, $C_2$, G, X und Y wie oben definiert sind oder in einer geschützten Form vorliegen,
mit einem geeigneten nukleophilen Mittel, und, sofern erforderlich, das Entschützen des Produktes zur Bildung einer Verbindung von Formel I umfaßt.

**3.** Verfahren zur Herstellung einer Verbindung, welche ein Pyrimidinderivat folgender Formel ist:

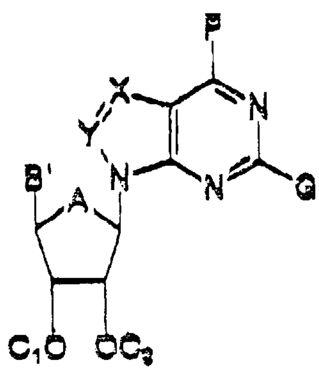

worin;

(a) A Sauerstoff, Methylen oder Schwefel ist;
(b) B' $-(CH_2)_n-B$ ist, worin n 1, 2, 3 oder 4 ist und B Hydroxy, Acyloxy, Hydrocarbyloxycarbonyloxy oder $-OCONR_2$ ist, worin jedes R unabhängig Wasserstoff oder Hydrocarbyl ist;
(c) $C_1$ und $C_2$ jeweils unabhängig Wasserstoff, Acyl, Hydrocarbyloxycarbonyl sind oder zusammengenommen einen 5-gliedrigen Ring bilden, worin $C_1$ eine Einfachbindung zu $C_2$ ist und $C_2$ Carbonyl oder α-Alkoxyalkyliden ist;
(d) X

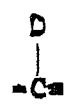

ist und Y

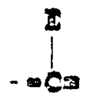

ist;

(e) D Aryl oder Aralkyl ist;

(f) E Wasserstoff, Halogen, Alkyl oder Alkylthio ist;

(g) F Alkyl, Aryl, Aralkyl, Halogen, Amino, Alkylamino, Arylamino, Aralkylamino, Cyano, Cyanoalkyl, Alkoxy, Aryloxy, Aralkyloxy, Alkylthio, Arylthio, Aralkylthio, wahlweise substituiertes Indolinyl oder Indolyl, Pyrrolidinyl oder Piperazinyl ist; und

(h) G Wasserstoff, Halogen, Niederalkyl, Niederalkoxy oder Niederalkylthio ist;

oder ein pharmazeutisch annehmbares Salz davon; mit der Maßgabe, daß; wenn A Sauerstoff ist, D Oxadiazolyl, Triazolyl oder Triazinyl ist und E und G beide Wasserstoff sind, dann F nicht Amino ist, wobei das Verfahren das Umsetzen einer Verbindung der Formel:

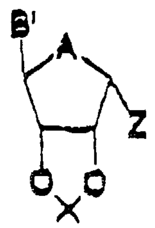

,

worin A und B' wie oben definiert sind und Z ein Halogen ist, mit einer Verbindung der Formel:

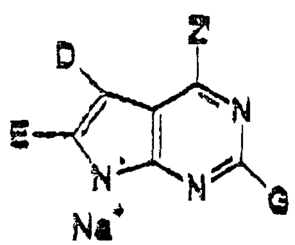

,

worin E, D und G wie oben definiert sind und Z' ein Halogen ist, zum Erhalt einer Verbindung der Formel:

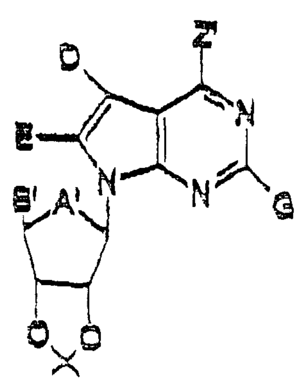

und das Entschützen der Verbindung zur Bereitstellung einer Verbindung der Formel I umfaßt.

4. Verfahren gemäß mindestens einem der Ansprüche 1 bis 3, worin A Sauerstoff ist.

5. Verfahren gemäß mindestens einem der Anspruche 1 bis 4, worin F Halogen oder eine Amino-, Alkylamino-, Arylamino-, Aralkylamino-, Alkylthio-, Arylthio-, Alkyl-, Aryl- oder Aralkylgruppe ist.

6. Verfahren gemäß mindestens einem der Ansprüche 1 bis 5, worin G Wasserstoff ist.

7. Verwendung einer Verbindung gemäß mindestens einem der Ansprüche 1 bis 6 bei der Herstellung eines Medikamentes zur Verwendung bei einem Verfahren der medizinischen Behandlung durch chirurgischen Eingriff, Therapie oder Diagnose beim menschlichen oder tierischen Körper.

8. Verwendung einer Verbindung gemäß Anspruch 7 bei der Herstellung eines Medikamentes zur Verwendung bei der Steigerung von Adenosinspiegel in einem Säuger.

9. Verwendung einer Verbindung gemäß Anspruch 7 bei der Herstellung eines Medikamentes zur Verwendung bei einem Verfahren der Steigerung lokaler Spiegel an Adenosin um Zellen mit einem unerwünscht niedrigen Verhältnis von Synthese an Adenosintriphosphat/Abbau an Adenosintriphosphat.

10. Verwendung einer Verbindung gemäß Anspruch 7 bei der Herstellung eines Medikamentes zur Verwendung bei einem Verfahren der Behandlung oder Vorbeugung bzw. Verhinderung einer kardiovaskulären Störung in einem Säuger, wo eine Verletzung oder Dysfunktion Ischämie, Reperfusion oder Arrhythmie verursacht oder durch diese verursacht wird.

11. Verwendung einer Verbindung gemäß Anspruch 7 bei der Herstellung eines Medikamentes zur Verwendung als unterstützende Therapie mit einer Vorgehensweise in einem Säuger zum Schutz des Herzens.

12. Verwendung einer Verbindung gemäß Anspruch 7 bei der Herstellung eines Medikamentes zur Verwendung bei einem Verfahren zur Verhinderung oder Verringerung von Reperfusionsverletzung bei einem Gewebe oder Organisumus in einem Säuger, gefolgt von unterbrochenem oder vermindertem Blutfluß zu dem Gewebe oder Organismus.

13. Verwendung einer Verbindung gemäß Anspruch 7 bei der Herstellung eines Medikamentes zur Verwendung bei einem Verfahren zur Verhinderung oder Verringerung von Blutplättchenaggregation in einem Säuger.

14. Verwendung einer Verbindung gemäß Anspruch 7 bei der Herstellung eines Medikamentes zur Verwendung bei einem Verfahren zur Verhinderung oder Verringerung von Entzündungsreaktionen in einem Säuger.

15. Verwendung einer Verbindung gemäß Anspruch 7 bei der Herstellung eines Medikamentes zur Verwendung bei einem Verfahren zur Behandlung eines Patienten mit einem chronisch niedrigen oder unzureichenden Adenosinspiegel, oder welcher aus einem erhöhten Adenosinspiegel des Zentralen Nervensystems Nutzen ziehen würde.

16. Verwendung einer Verbindung gemäß Anspruch 7 bei der Herstellung eines Medikamentes zur Verwendung bei einem Verfahren zur Erzeugung von Skelettmuskel-Relaxation oder zur Verhinderung von Skelettmuskel-Spasmus in einem Säuger.

17. Verwendung einer Verbindung gemäß Anspruch 7 bei der Herstellung eines Medikamentes zur Verwendung bei einem Verfahren zur Förderung eines Schlafzustandes bei einem Patienten.

18. Verwendung einer Verbindung gemäß Anspruch 7 bei der Herstellung eines Medikamentes zur Verwendung bei einem Verfahren zur Behandlung von Angst.

19. Verwendung einer Verbindung gemäß Anspruch 7 bei der Herstellung eines Medikamentes zur Verwendung bei einem Verfahren zur Verringerung oder Verhinderung von durch Excitotoxizität verursachter Nervengewebeschädigung in einem Säuger.

20. Verwendung einer Verbindung gemäß Anspruch 7 bei der Herstellung eines Medikamentes zur Verwendung bei einem Verfahren zur Verringerung von mit einer neurodegenerativen Erkrankung in Verbindung stehender Nervengewebeschädigung in einem Tier.

**21.** Verwendung einer Verbindung gemäß Anspruch 7 bei der Herstellung eines Medikamentes zur Verwendung bei einem Verfahren zur Behandlung oder Verhinderung von Epilepsie oder Anfällen bei einem Säuger.

**22.** Verwendung einer Verbindung gemäß Anspruch 7 bei der Herstellung eines Medikamentes zur Verwendung bei einem Verfahren der Senkung der lokalen Kontraktion glatter Muskulatur in einem Säuger.

**23.** Verwendung einer Verbindung gemäß mindestens einem der Ansprüche 9, 11 oder 19 bei der Herstellung eines Medikamentes zu Verwendung bei der Behandlung eines Schlaganfalles.

**24.** Verwendung einer Verbindung gemäß Anspruch 14, wobei die Eutzündungsreaktionen Arthritis, Meningitis, Autoimmunerkrankung, Darmentzündungserkrankung, Vasculitis, Dermatitis, Myositis oder Nierenentzündung verursachen.

**25.** Verwendung einer Verbindung mit einem Pyrrolo[2,3-d]pyrimidin-Gerüst oder einem Pyrazolo[3,4-d]pyrimidin-Gerüst und einem $IC_{50}$-Wert von etwa 1 mikromolar oder weniger in einem *in vitro*-Adenosin-Kinaseenzym-Inhibitionstestverfahren bei der Herstellung eines Medikamentes zur Verwendung bei einem Behandlungsverfahren, wie in mindestens einem der Ansprüch 8 bis 24 definiert.

**26.** Verwendung einer Verbindung gemäß mindestens einem der Ansprüche 1 bis 6 bei der Herstellung eines Medikamentes zur Verwendung bei einem Behandlungsverfahren, wie in mindestens einem der Ansprüch 8 bis 24 definiert.

**27.** Verbindung, welche ein Pyrimidinderivat folgender Formel ist:

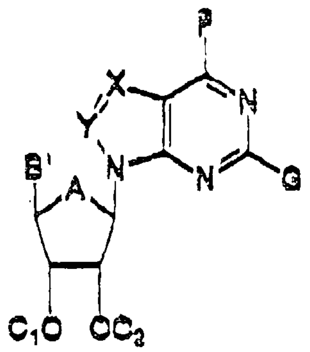

worin:

(a) A Sauerstoff, Methylen oder Schwefel ist;
(b) B' $-(CH_2)_n-B$ ist, worin n 1, 2, 3 oder 4 ist und B Wasserstoff, Alkyl, Alkoxy, Amino, Alkylamino, Acylamino, Hydrocarbyloxycarbonylamino, Mercapto, Alkylthio, Azido, Cyano, Halogen ist, oder B' Alkenyl oder Alkinyl ist;
(c) $C_1$ und $C_2$ jeweils unabhängig Wasserstoff, Acyl, Hydrocarbyloxycarbonyl sind oder zusammengenommen einen 5-gliedrigen Ring bilden, worin $C_1$ eine Einfachbindung zu $C_2$ ist und $C_2$ Carbonyl oder α-Alkoxyalkyliden ist;
(d) X

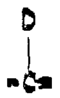

ist und Y -N= oder

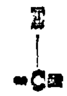

ist;

(e) D Wasserstoff, Halogen, Alkyl, Aryl, Aralkyl, Alkenyl, Alkinyl, Halogenalkyl, Cyano, Cyanoalkyl, Acyl, Carboxamido, eine Carbonsäure- oder eine Carbonsäureestergruppe, Alkoxy, Aryloxy, Aralkyloxy, Alkylthio, Arylthio, Aralkylthio, Amino, Alkylamino, Arylamino, Aralkylamino, Acylamino oder Nitro ist;

(f) E Wasserstoff, Halogen, Alkyl oder Alkylthio ist;

(g) F Alkyl, Aryl, Aralkyl, Halogen, Amino, Alkylamino, Arylamino, Aralkylamino, Cyano, Cyanoalkyl, Alkoxy, Aryloxy, Aralkoxy, Alkylthio, Arylthio, Aralkylthio; wahlweise substituiertes Indolinyl oder Indolyl; Pyrrolidinyl oder Piperazinyl ist; und

(h) G Wasserstoff, Halogen, Niederalkyl, Niederalkoxy, Niederalkylamino oder Niederalkylthio ist;

oder ein pharmazeutisch annehmbares Salz davon; mit der Maßgabe, daß: wenn A Sauerstoff ist und

(i) X

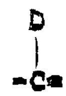

ist und Y

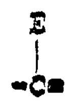

ist, dann, wenn B' Methyl ist, D Halogen, Cyano oder Carboxamido ist und F Amino ist, dann G nicht Wasserstoff ist; oder, wenn D Wasserstoff ist, dann F nicht Amino ist; oder

(ii) X

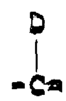

ist und Y -N= ist, wenn B Wasserstoff oder Halogen ist, D und G Wasserstoff sind, dann F nicht Amino ist; oder wenn A Methylen ist, X

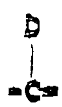

ist, Y

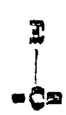

ist, B, D, E und G Wasserstoff sind, dann F nicht Amino ist.

## Revendications

**Revendications pour les Etats contractants suivants : AT, BE, CH, LI, DE, DK, FR, GB, IT, LU, MC, NL, PT, SE**

**1.** Composé qui est un dérivé de pyrimidine, de formule :

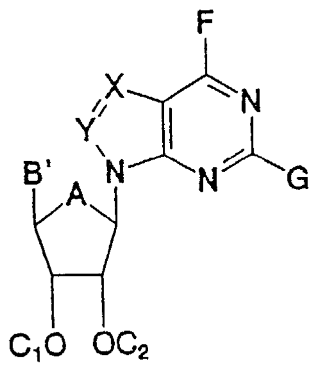

dans laquelle :

(a) A représente un atome d'oxygène, un groupe méthylène ou un atome de soufre ;
(b) B' représente $-(CH_2)_n-B$ où n est 1, 2, 3 ou 4 et B représente un atome d'hydrogène, un groupe alkyle, alcoxy, amino, alkylamino, acylamino, hydrocarbyloxycarbonylamino, mercapto, alkylthio, azido, cyano, halogéno, ou B' représente un groupe alcényle ou alcynyle ;
(c) $C_1$ et $C_2$ représentent chacun indépendamment un atome d'hydrogène, un groupe acyle, hydrocarbyloxycarbonyle ou, pris ensemble, forment un cycle à 5 maillons dans lequel $C_1$ représente une liaison simple avec $C_2$ et $C_2$ représente un groupe carbonyle ou α-alcoxyalkylidène ;
(d) X représente

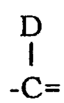

et Y représente -N= ou

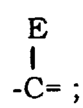

(e) D représente un atome d'hydrogène ou d'halogène, un groupe alkyle, aryle, aralkyle, alcényle, alcynyle, halogénoalkyle, cyano, cyanoalkyle, acyle, carboxamido, acide carboxylique ou ester d'acide carboxylique, alcoxy, aryloxy, aralkyloxy, alkylthio, arylthio, aralkylthio, amino, alkylamino, arylamino, aralkylamino, acylamino ou nitro ;
(f) E représente un atome d'hydrogène ou d'halogène, un groupe alkyle ou alkylthio ;
(g) F représente un groupe alkyle, aryle, aralkyle, halogéno, amino, alkylamino, arylamino, aralkylamino, cyano, cyanoalkyle, alcoxy, aryloxy, aralcoxy, alkylthio, arylthio, aralkylthio; indolinyle ou indolyle éventuellement substitué ; pyrrolidinyle ou pipérazinyle ; et
(h) G représente un atome d'hydrogène ou d'halogène, un groupe alkyle inférieur, alcoxy inférieur, alkylamino inférieur ou alkylthio inférieur ;

ou un de ses sels pharmaceutiquement acceptables ; étant entendu que : lorsque A représente un atome d'oxygène et

(i) X représente

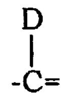

et Y représente

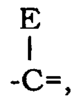

alors, si B' représente un groupe méthyle, D un groupe halogéno, cyano ou carboxamido, et F représente un groupe amino, G ne peut pas représenter un atome d'hydrogène ; ou si D représente un atome d'hydrogène, alors F ne peut pas être un groupe amino ; ou
(ii) X représente

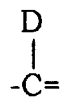

et Y représente -N=, si B représente un atome d'hydrogène ou d'halogène, D et G représentent chacun un atome d'hydrogène, alors F ne peut pas être un groupe amino ; ou lorsque A représente un groupe méthylène, X représente

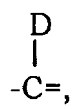

Y représente

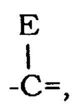

B, D, E et G représentent un atome d'hydrogène, alors F ne peut pas être un groupe amino.

**2.** Composé qui est un dérivé de pyrimidine de formule :

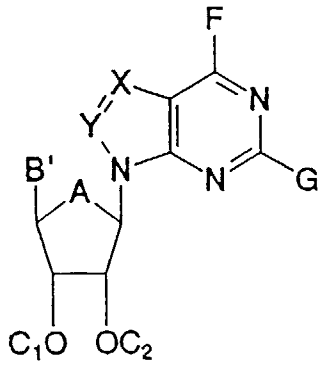

dans laquelle :

(a) A représente un atome d'oxygène, un groupe méthylène ou un atome de soufre ;
(b) B' représente $-(CH_2)_n$-B où n est 1, 2, 3 ou 4 et B représente un groupe hydroxy, acyloxy, hydrocarbyloxycarbonyloxy, ou $-OCONR_2$ où chaque R représente indépendamment un atome d'hydrogène ou un groupe hydrocarbyle ;
(c) $C_1$ et $C_2$ représentent chacun indépendamment un atome d'hydrogène, un groupe acyle, hydrocarbyloxycarbonyle ou, pris ensemble, forment un cycle à 5 maillons dans lequel $C_1$ représente une liaison simple avec $C_2$ et $C_2$ représente un groupe carbonyle ou α-alcoxyalkylidène ;
(d) X représente

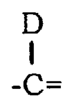

et Y représente -N= ;
(e) D représente un groupe halogéno, aryle ou aralkyle ;
(f) F représente un groupe alkyle, aryle, aralkyle, halogéno, amino, alkylamino, arylamino, aralkylamino, cyano, cyanoalkyle, alcoxy, aryloxy, aralcoxy, alkylthio, arylthio, aralkylthio, indolinyle ou indolyle éventuellement substitué, pyrrolidinyle ou pipérazinyle ; et
(g) G représente un atome d'hydrogène ou d'halogène, un groupe alkyle inférieur, alcoxy inférieur ou alkylthio inférieur ; ou un de ses sels pharmaceutiquement acceptables ;
étant entendu que, lorsque A représente un atome d'oxygène et D un atome d'halogène, alors F ne peut pas être un groupe amino.

**3.** Composé qui est un dérivé de pyrimidine, de formule :

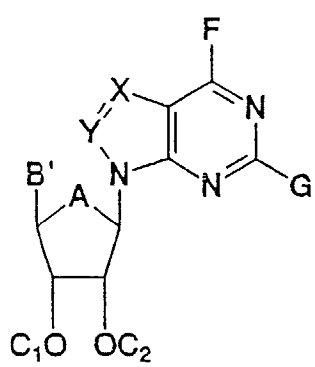

dans laquelle :

(a) A représente un atome d'oxygène, un groupe méthylène ou un atome de soufre ;

(b) B' représente $-(CH_2)_n-B$ où n est 1, 2, 3 ou 4 et B représente un groupe hydroxy, acyloxy, hydrocarbyloxy-carbonyloxy, ou $-OCONR_2$ où chaque R représente indépendamment un atome d'hydrogène ou un groupe hydrocarbyle ;

(c) $C_1$ et $C_2$ représentent chacun indépendamment un atome d'hydrogène, un groupe acyle, hydrocarbyloxycarbonyle ou, pris ensemble, forment un cycle à 5 maillons dans lequel $C_1$ représente une liaison simple avec $C_2$ et $C_2$ représente un groupe carbonyle ou $\alpha$-alcoxyalkylidène ;

(d) X représente

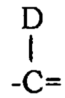

et Y représente

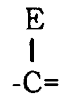

;

(e) D représente un groupe aryle ou aralkyle ;

(f) E représente un atome d'hydrogène, d'halogène, un groupe alkyle ou alkylthio ;

(g) F représente un groupe alkyle, aryle, aralkyle, halogéno, amino, alkylamino, arylamino, aralkylamino, cyano, cyanoalkyle, alcoxy, aryloxy, aralkyloxy, alkylthio, arylthio, aralkylthio, indolinyle ou indolyle éventuellement substitué, pyrrolidinyle ou pipérazinyle ; et

(h) G représente un atome d'hydrogène, d'halogène, un groupe alkyle inférieur, alcoxy inférieur ou alkylthio inférieur ; ou un de ses sels pharmaceutiquement acceptables ;

étant entendu que : lorsque A représente un atome d'oxygène, D est un groupe oxadiazolyle, triazolyle ou triazinyle, et E et G sont tous les deux un atome d'hydrogène, alors F ne peut pas être un groupe amino.

**4.** Composé selon l'une quelconque des revendications 1 à 3, dans lequel A est un atome d'oxygène.

**5.** Composé selon l'une quelconque des revendications 1 à 4, dans lequel F représente un atome d'halogène ou un groupe amino, alkylamino, arylamino, aralkylamino, alkylthio, arylthio, alkyle, aryle ou aralkyle.

**6.** Composé selon l'une quelconque des revendications 1 à 5, dans lequel G représente un atome d'hydrogène.

**7.** Composé tel que revendiqué dans l'une quelconque des revendications 1 à 6, destiné à être utilisé dans une méthode de traitement médical par chirurgie, de thérapie ou de diagnostic sur le corps humain ou animal.

**8.** Composé tel que revendiqué selon la revendication 7, destiné à être utilisé pour élever les taux d'adénosine chez un mammifère.

**9.** Composé selon la revendication 7, destiné à être utilisé dans une méthode servant à élever localement les taux d'adénosine autour de cellules ayant un faible rapport indésirable de synthèse d'adénosine-triphosphate / décomposition d'adénosine-triphosphate.

**10.** Composé selon la revendication 7, destiné à être utilisé dans une méthode de traitement ou de prévention d'un trouble cardiovasculaire chez un mammifère, lorsqu'une lésion ou un dysfonctionnement provoque, ou est provoqué par, une ischémie, une reperfusion ou une arythmie.

**11.** Composé selon la revendication 7, destiné à être utilisé comme traitement d'appoint pour la protection du coeur chez un mammifère.

**12.** Composé selon la revendication 7, destiné à être utilisé dans une méthode de prévention ou de diminution de

lésions provoquées par la reperfusion dans un tissu ou organisme chez un mammifère, après une irrigation interrompue ou diminuée de ce tissu ou organisme.

**13.** Composé selon la revendication 7, destiné à être utilisé dans une méthode de prévention ou de diminution de l'agrégation plaquettaire chez un mammifère.

**14.** Composé selon la revendication 7, destiné à être utilisé dans une méthode de prévention ou de diminution des réactions inflammatoires chez un mammifère.

**15.** Composé selon la revendication 7, destiné à être utilisé dans une méthode de traitement d'un patient ayant un faible taux chronique ou insuffisant d'adénosine, ou pour qui un taux accru d'adénosine dans le système nerveux central serait profitable.

**16.** Composé selon la revendication 7, destiné à être utilisé dans une méthode produisant une relaxation des muscles du squelette ou prévenant des spasmes des muscles du squelette chez un mammifère.

**17.** Composé selon la revendication 7, destiné à être utilisé dans une méthode favorisant un état de sommeil chez un patient.

**18.** Composé selon la revendication 7, destiné à être utilisé dans une méthode de traitement de l'anxiété.

**19.** Composé selon la revendication 7, destiné à être utilisé dans une méthode de réduction ou de prévention de lésions du tissu nerveux provoquées par l'excitotoxicité chez un mammifère.

**20.** Composé selon la revendication 7, destiné à être utilisé dans une méthode de diminution des lésions du tissu nerveux liées à une maladie neurodégénérative chez un animal.

**21.** Composé selon la revendication 7, destiné à être utilisé dans une méthode de traitement ou de prévention de l'épilepsie ou de crises chez un mammifère.

**22.** Composé selon la revendication 7, destiné à être utilisé dans une méthode de diminution de la contraction locale des muscles lisses chez un mammifère.

**23.** Composé selon l'une quelconque des revendications 8, 12 ou 19, destiné à être utilisé dans le traitement d'une accident vasculaire cérébral.

**24.** Composé selon la revendication 14, destiné aux réactions inflammatoires provoquant de l'arthrite, une méningite, une maladie autoimmune, une maladie inflammatoire des intestins, une vascularite, une dermatose, une myosite ou une inflammation rénale.

**25.** Utilisation d'un composé ayant un squelette pyrrolo[3,4-d]-pyrimidine ou un squelette pyrazole[3,4-d]-pyrimidine ayant une $CI_{50}$ de 1 micromolaire ou inférieure, dans une méthode de test in vitro d'inhibition de l'enzyme adénosine kinase, pour la fabrication d'un médicament destiné à être utilisé dans une méthode de traitement telle que définie selon l'une quelconque des revendications 8 à 24.

**26.** Utilisation d'un composé tel que revendiqué selon l'une quelconque des revendications 1 à 6, dans la fabrication d'un médicament destiné à être utilisé dans une méthode de traitement telle que définie selon l'une quelconque des revendications 8 à 24.

**27.** Utilisation selon la revendication 25 ou 26, dans laquelle le médicament est destiné à être utilisé comme agent anti-inflammatoire.

**28.** Utilisation selon la revendication 27, dans laquelle le composé réduit d'au moins 20 % la moyenne totale de l'oedème de la patte arrière à une dose de 300 mg/kg jour ou inférieure, dans une méthode de test d'inflammation in vivo.

**Revendications pour les Etats contractants suivants : ES, GR**

1. Procédé de préparation d'un composé qui est un dérivé de pyrimidine, de formule :

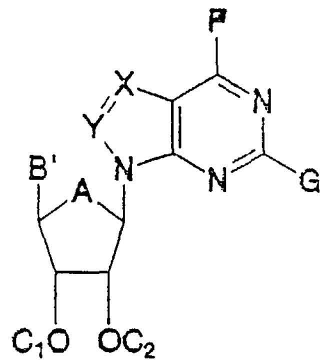

dans laquelle :

(a) A représente un atome d'oxygène, un groupe méthylène ou un atome de soufre ;
(b) B' représente $-(CH_2)_n-B$ où n est 1, 2, 3 ou 4 et B représente un atome d'hydrogène, un groupe alkyle, alcoxy, amino, alkylamino, acylamino, hydrocarbyloxycarbonylamino, mercapto, alkylthio, azido, cyano, halogéno, ou B' représente un groupe alcényle ou alcynyle ;
(c) $C_1$ et $C_2$ représentent chacun indépendamment un atome d'hydrogène, un groupe acyle, hydrocarbyloxycarbonyle ou, pris ensemble, forment un cycle à 5 maillons dans lequel $C_1$ représente une liaison simple avec $C_2$ et $C_2$ représente un groupe carbonyle ou α-alcoxyalkylidène ;
(d) X représente

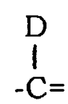

et Y représente -N= ou

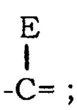

(e) D représente un atome d'hydrogène ou d'halogène, un groupe alkyle, aryle, aralkyle, alcényle, alcynyle, halogénoalkyle, cyano, cyanoalkyle, acyle, carboxamido, acide carboxylique ou ester d'acide carboxylique, alcoxy, aryloxy, aralkyloxy, alkylthio, arylthio, aralkylthio, amino, alkylamino, arylamino, aralkylamino, acylamino ou nitro ;
(f) E représente un atome d'hydrogène ou d'halogène, un groupe alkyle ou alkylthio ;
(g) F représente un groupe alkyle, aryle, aralkyle, halogéno, amino, alkylamino, arylamino, aralkylamino, cyano, cyanoalkyle, alcoxy, aryloxy, aralcoxy, alkylthio, arylthio, aralkylthio; indolinyle ou indolyle éventuellement substitué ; pyrrolidinyle ou pipérazinyle ; et
(h) G représente un atome d'hydrogène ou d'halogène, un groupe alkyle inférieur, alcoxy inférieur, alkylamino inférieur ou alkylthio inférieur ;

ou un de ses sels pharmaceutiquement acceptables ; étant entendu que : lorsque A représente un atome d'oxygène et

(i) X représente

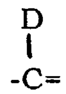

et Y représente

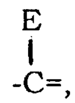

alors, si B' représente un groupe méthyle, D un groupe halogéno, cyano ou carboxamido, et F représente un groupe amino, G ne peut pas représenter un atome d'hydrogène; ou si D représente un atome d'hydrogène, alors F ne peut pas être un groupe amino ; ou

(ii) X représente

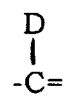

et Y représente -N=, si B représente un atome d'hydrogène ou d'halogène, D et G représentent chacun un atome d'hydrogène, alors F ne peut pas être un groupe amino ; ou lorsque A représente un groupe méthylène, X représente

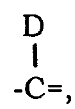

Y représente

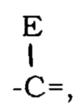

B, D, E et G représentent un atome d'hydrogène, alors F ne peut pas être un groupe amino, ledit procédé comprenant la réaction d'un composé de formule :

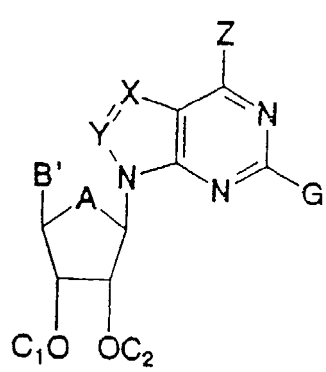

dans laquelle Z représente un atome d'halogène et A, B', $C_1$, $C_2$, G et X sont tels que définis ci-dessus ou sont sous une forme protégée, et Y représente -N=, avec un agent nucléophile approprié et, si nécessaire, la déprotection du produit pour former un composé de formule I dans laquelle Y représente -N= ; ou ledit procédé comprenant la réaction d'un composé de formule :

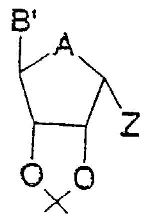

dans laquelle A et B' sont tels que définis ci-dessus et Z représente un atome d'halogène, avec un composé de formule :

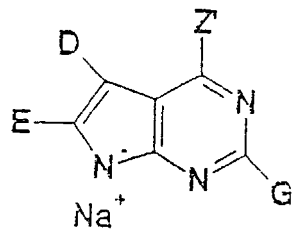

dans laquelle E, D et G sont tels que définis ci-dessus et Z' est un atome d'halogène, pour donner un composé de formule :

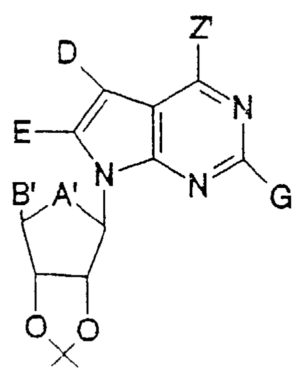

et la déprotection du composé pour fournir un composé de formule I dans laquelle Y représente

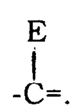

**2.** Procédé de préparation d'un composé qui est un dérivé de pyrimidine de formule :

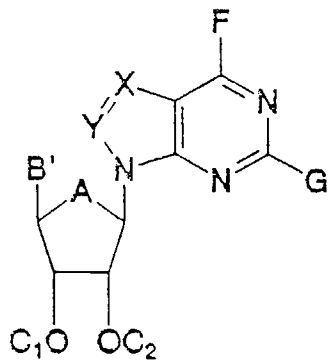

dans laquelle :

(a) A représente un atome d'oxygène, un groupe méthylène ou un atome de soufre ;
(b) B' représente $-(CH_2)_n$-B où n est 1, 2, 3 ou 4 et B représente un groupe hydroxy, acyloxy, hydrocarbyloxy-carbonyloxy, ou $-OCONR_2$ où chaque R représente indépendamment un atome d'hydrogène ou un groupe hydrocarbyle ;
(c) $C_1$ et $C_2$ représentent chacun indépendamment un atome d'hydrogène, un groupe acyle, hydrocarbyloxycarbonyle ou, pris ensemble, forment un cycle à 5 maillons dans lequel $C_1$ représente une liaison simple avec $C_2$ et $C_2$ représente un groupe carbonyle ou α-alcoxyalkylidène ;
(d) X représente

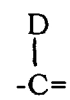

et Y représente -N= ;
(e) D représente un groupe halogéno, aryle ou aralkyle ;
(f) F représente un groupe alkyle, aryle, aralkyle, halogéno, amino, alkylamino, arylamino, aralkylamino, cyano, cyanoalkyle, alcoxy, aryloxy, aralcoxy, alkylthio, arylthio, aralkylthio, indolinyle ou indolyle éventuellement substitué, pyrrolidinyle ou pipérazinyle ; et
(g) G représente un atome d'hydrogène ou d'halogène, un groupe alkyle inférieur, alcoxy inférieur ou alkylthio inférieur ;
ou un de ses sels pharmaceutiquement acceptables ;
étant entendu que: lorsque A représente un atome d'oxygène et D un atome d'halogène, alors F ne peut pas être un groupe amino, ledit procédé comprenant la réaction d'un composé de formule :

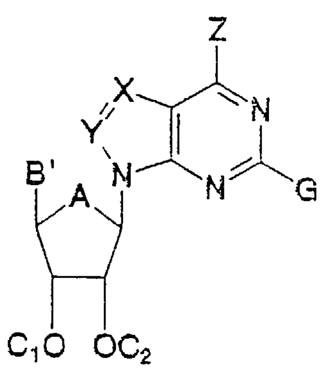

dans laquelle Z est un atome d'halogène et A, B', $C_1$, $C_2$, G, X et Y sont tels que définis ci-dessus ou sont sous une forme protégée, avec un agent nucléophile approprié et, si nécessaire, la déprotection du produit

pour former un composé de formule I.

**3.** Procédé de préparation d'un composé qui est un dérivé de pyrimidine, de formule :

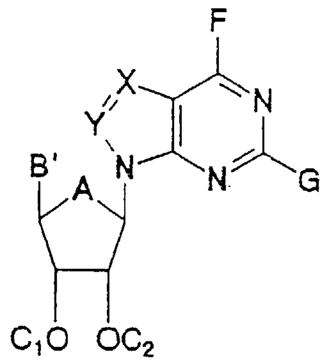

dans laquelle :

(a) A représente un atome d'oxygène, un groupe méthylène ou un atome de soufre ;
(b) B' représente $-(CH_2)_n-B$ où n est 1, 2, 3 ou 4 et B représente un groupe hydroxy, acyloxy, hydrocarbyloxycarbonyloxy, ou $-OCONR_2$ où chaque R représente indépendamment un atome d'hydrogène ou un groupe hydrocarbyle ;
(c) $C_1$ et $C_2$ représentent chacun indépendamment un atome d'hydrogène, un groupe acyle, hydrocarbyloxycarbonyle ou, pris ensemble, forment un cycle à 5 maillons dans lequel $C_1$ représente une liaison simple avec $C_2$ et $C_2$ représente un groupe carbonyle ou α-alcoxyalkylidène ;
(d) X représente

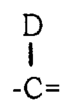

et Y représente

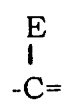 ;

(e) D représente un groupe aryle ou aralkyle ;
(f) E représente un atome d'hydrogène, d'halogène, un groupe alkyle ou alkylthio ;
(g) F représente un groupe alkyle, aryle, aralkyle, halogéno, amino, alkylamino, arylamino, aralkylamino, cyano, cyanoalkyle, alcoxy, aryloxy, aralkyloxy, alkylthio, arylthio, aralkylthio, indolinyle ou indolyle éventuellement substitué, pyrrolidinyle ou pipérazinyle ; et
(h) G représente un atome d'hydrogène, d'halogène, un groupe alkyle inférieur, alcoxy inférieur ou alkylthio inférieur ;

ou un de ses sels pharmaceutiquement acceptables ; étant entendu que: lorsque A représente un atome d'oxygène, D est un groupe oxadiazolyle, triazolyle ou triazinyle, et E et G sont tous les deux un atome d'hydrogène, alors F ne peut pas être un groupe amino, ledit procédé comprenant la réaction d'un composé de formule :

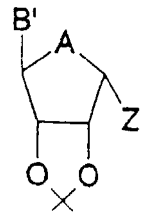

dans laquelle A et B' sont tels que définis ci-dessus et Z est un atome d'halogène, avec un composé de formule :

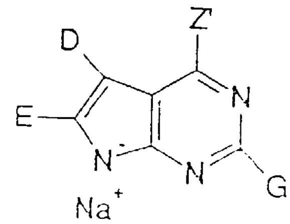

dans laquelle E, D et G sont tels que définis ci-dessus et Z' est un atome d'halogène, pour donner un composé de formule :

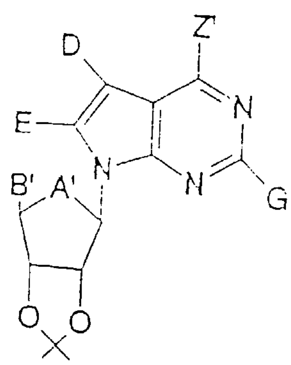

et la déprotection du composé pour fournir un composé de formule I.

**4.** Procédé selon l'une quelconque des revendications 1 à 3, dans lequel A est un atome d'oxygène.

**5.** Procédé selon l'une quelconque des revendications 1 à 4, dans lequel F représente un atome d'halogène ou un groupe amino, alkylamino, arylamino, aralkylamino, alkylthio, arylthio, alkyle, aryle ou aralkyle.

**6.** Procédé selon l'une quelconque des revendications 1 à 5, dans lequel G représente un atome d'hydrogène.

**7.** Utilisation d'un composé tel que revendiqué dans l'une quelconque des revendications 1 à 6, dans la fabrication d'un médicament destiné à être utilisé dans une méthode de traitement médical par chirurgie, de thérapie ou de diagnostic sur le corps humain ou animal.

**8.** Utilisation d'un composé telle que revendiquée à la revendication 7, dans la fabrication d'un médicament destiné à être utilisé pour élever les taux d'adénosine chez un mammifère.

**9.** Utilisation d'un composé selon la revendication 7, dans la fabrication d'un médicament destiné à être utilisé dans une méthode servant à élever localement les taux d'adénosine autour de cellules ayant un faible rapport indésirable de synthèse d'adénosine-triphosphate/décomposition d'adénosinetriphosphate.

**10.** Utilisation d'un composé selon la revendication 7, dans la fabrication d'un médicament destiné à être utilisé dans

une méthode de traitement ou de prévention d'un trouble cardiovasculaire chez un mammifère, lorsqu'une lésion ou un dysfonctionnement provoque, ou est provoqué par, une ischémie, une reperfusion ou une arythmie.

**11.** Utilisation d'un composé selon la revendication 7, dans la fabrication d'un médicament destiné à être utilisé comme traitement d'appoint pour la protection du coeur chez un mammifère.

**12.** Utilisation d'un composé selon la revendication 7, dans la fabrication d'un médicament destiné à être utilisé dans une méthode de prévention ou de diminution de lésions provoquées par la reperfusion dans un tissu ou organisme chez un mammifère, après une irrigation interrompue ou diminuée de ce tissu ou organisme.

**13.** Utilisation d'un composé selon la revendication 7, dans la fabrication d'un médicament destiné à être utilisé dans une méthode de prévention ou de diminution de l'agrégation plaquettaire chez un mammifère.

**14.** Utilisation d'un composé selon la revendication 7, dans la fabrication d'un médicament destiné à être utilisé dans une méthode de prévention ou de diminution des réactions inflammatoires chez un mammifère.

**15.** Utilisation d'un composé selon la revendication 7, dans la fabrication d'un médicament destiné à être utilisé dans une méthode de traitement d'un patient ayant un faible taux chronique ou insuffisant d'adénosine, ou pour qui un taux accru d'adénosine dans le système nerveux central serait profitable.

**16.** Utilisation d'un composé selon la revendication 7, dans la fabrication d'un médicament destiné à être utilisé dans une méthode produisant une relaxation des muscles du squelette ou prévenant des spasmes des muscles du squelette chez un mammifère.

**17.** Utilisation d'un composé selon la revendication 7, dans la fabrication d'un médicament destiné à être utilisé dans une méthode favorisant un état de sommeil chez un patient.

**18.** Utilisation d'un composé selon la revendication 7, dans la fabrication d'un médicament destiné à être utilisé dans une méthode de traitement de l'anxiété.

**19.** Utilisation d'un composé selon la revendication 7, dans la fabrication d'un médicament destiné à être utilisé dans une méthode de réduction ou de prévention de lésions du tissu nerveux provoquées par l'excitotoxicité chez un mammifère.

**20.** Utilisation d'un composé selon la revendication 7, dans la fabrication d'un médicament destiné à être utilisé dans une méthode de diminution des lésions du tissu nerveux liées à une maladie neurodégénérative chez un animal.

**21.** Utilisation d'un composé selon la revendication 7, dans la fabrication d'un médicament destiné à être utilisé dans une méthode de traitement ou de prévention de l'épilepsie ou de crises chez un mammifère.

**22.** Utilisation d'un composé selon la revendication 7, dans la fabrication d'un médicament destiné à être utilisé dans une méthode de diminution de la contraction locale des muscles lisses chez un mammifère.

**23.** Utilisation d'un composé selon l'une quelconque des revendications 9, 11 ou 19, dans la fabrication d'un médicament destiné à être utilisé dans le traitement d'une accident vasculaire cérébral.

**24.** Utilisation d'un composé selon la revendication 14, destinée aux réactions inflammatoires provoquant de l'arthrite, une méningite, une maladie autoimmune, une maladie inflammatoire des intestins, une vascularite, une dermatose, une myosite ou une inflammation rénale.

**25.** Utilisation d'un composé ayant un squelette pyrrolo[3,4-d]-pyrimidine ou un squelette pyrazolo[3,4-d]-pyrimidine ayant une $CI_{50}$ environ 1 micromolaire ou inférieure, dans une méthode de test in vitro d'inhibition de l'enzyme adénosine kinase, pour la fabrication d'un médicament destiné à être utilisé dans une méthode de traitement telle que définie selon l'une quelconque des revendications 8 à 24.

**26.** Utilisation d'un composé tel que revendiqué selon l'une quelconque des revendications 1 à 6, dans la fabrication d'un médicament destiné à être utilisé dans une méthode de traitement telle que définie selon l'une quelconque des revendications 8 à 24.

**27.** Composé qui est un dérivé de pyrimidine de formule :

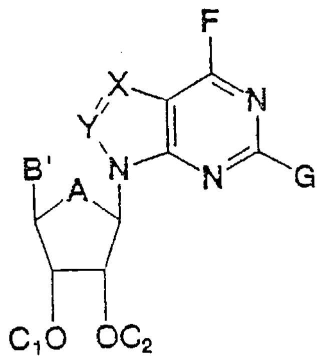

dans laquelle :

(a) A représente un atome d'oxygène, un groupe méthylène ou un atome de soufre ;
(b) B' représente $-(CH_2)_n-B$ où n est 1, 2, 3 ou 4 et B représente un atome d'hydrogène, un groupe alkyle, alcoxy, amino, alkylamino, acylamino, hydrocarbyloxycarbonylamino, mercapto, alkylthio, azido, cyano, halogéno, ou B' représente un groupe alcényle ou alcynyle ;
(c) $C_1$ et $C_2$ représentent chacun indépendamment un atome d'hydrogène, un groupe acyle, hydrocarbyloxycarbonyle ou, pris ensemble, forment un cycle à 5 maillons dans lequel $C_1$ représente une liaison simple avec $C_2$ et $C_2$ représente un groupe carbonyle ou $\alpha$-alcoxyalkylidène ;
(d) X représente

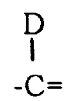

et Y représente -N= ou

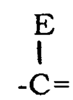
;

(e) D représente un atome d'hydrogène, d'halogène, un groupe alkyle, aryle, aralkyle, alcényle, alcynyle, halogénoalkyle, cyano, cyanoalkyle, acyle, carboxamido, acide carboxylique ou ester d'acide carboxylique, alcoxy, aryloxy, aralkyloxy, alkylthio, arylthio, aralkylthio, amino, alkylamino, arylamino, aralkylamino, acylamino ou nitro ;
(f) E représente un atome d'hydrogène, d'halogène, un groupe alkyle ou alkylthio ;
(g) F représente un groupe alkyle, aryle, aralkyle, halogéno, amino, alkylamino, arylamino, aralkylamino, cyano, cyanoalkyle, alcoxy, aryloxy, aralcoxy, alkylthio, arylthio, aralkylthio; indolinyle ou indolyle éventuellement substitué ; pyrrolidinyle ou pipérazinyle ; et
(h) G représente un atome d'hydrogène, d'halogène, un groupe alkyle inférieur, alcoxy inférieur, alkylamino inférieur ou alkylthio inférieur ;

ou un de ses sels pharmaceutiquement acceptables ; étant entendu que : lorsaue A représente un atome d'oxygène et

(i) X représente

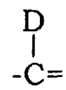

et Y représente

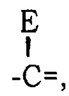

alors, si B' représente un groupe méthyle, D un groupe halogéno, cyano ou carboxyamido, et F représente un groupe amino, G ne peut pas représenter un atome d'hydrogène ; ou si D représente un atome d'hydrogène, alors F ne peut pas être un groupe amino ; ou
(ii) X représente

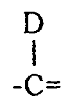

et Y représente -N=. si B représente un atome d'hydrogène ou d'halogène, D et G représentent chacun un atome d'hydrogène, alors F ne peut pas être un groupe amino ;
ou lorsque A représente un groupe méthylène, X représente

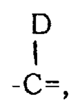

Y représente

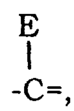

B, D, E et G représentent un atome d'hydrogène, alors F ne peut pas être un groupe amino.

Figure 1

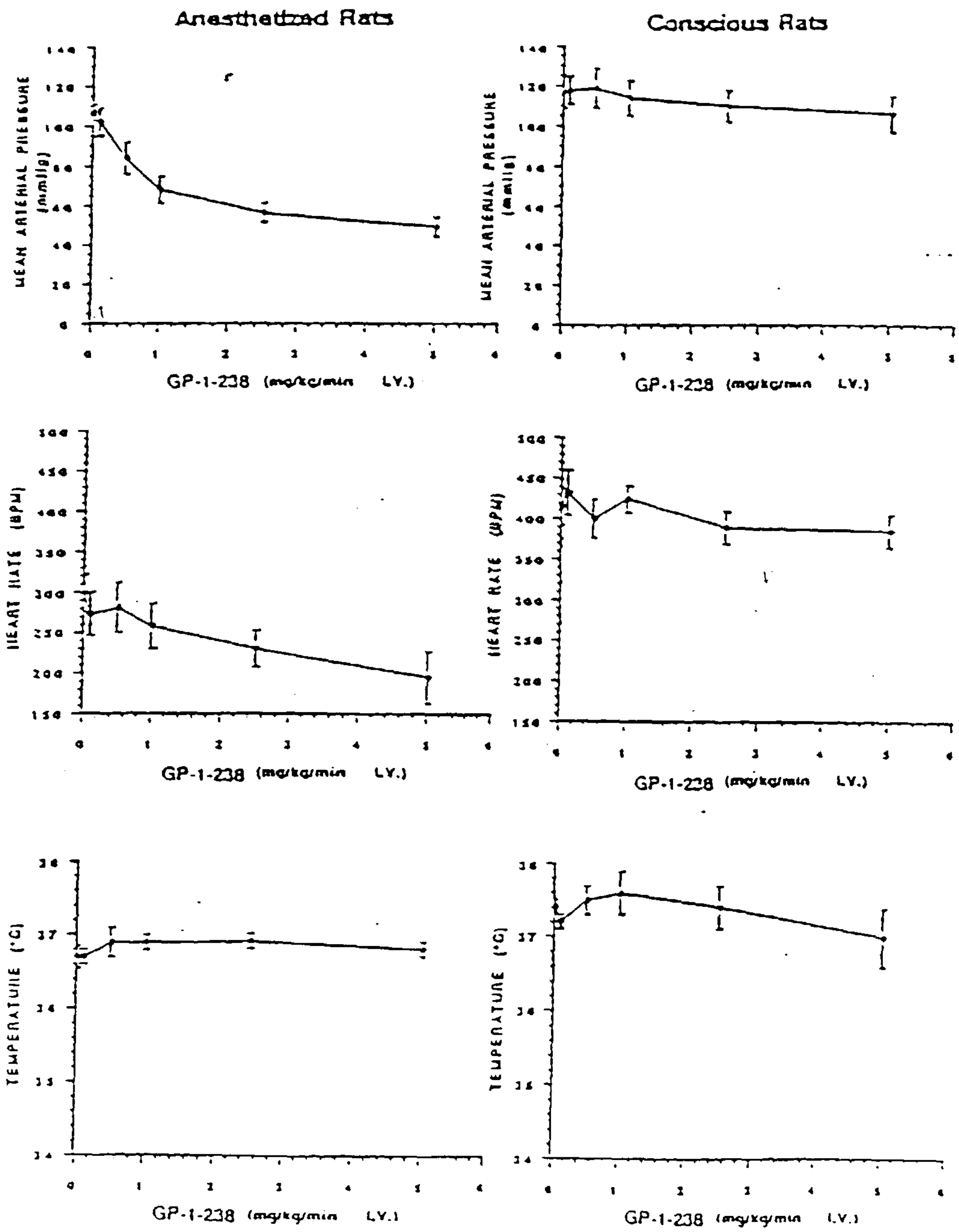
Anesthetized Rats
Conscious Rats
MEAN ARTERIAL PRESSURE (mmHg)
HEART RATE (BPM)
TEMPERATURE (°C)
GP-1-238 (mg/kg/min I.V.)

Figure 2

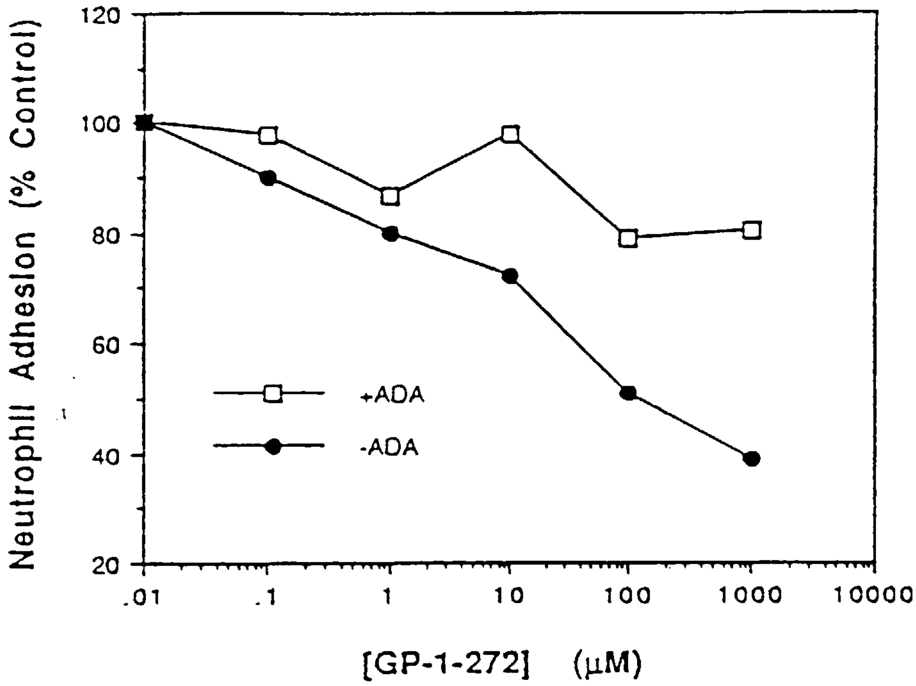
120
100
80
60
40
20
Neutrophil Adhesion (% Control)
+ADA
-ADA
.01
.1
1
10
100
1000
10000
[GP-1-272] (μM)

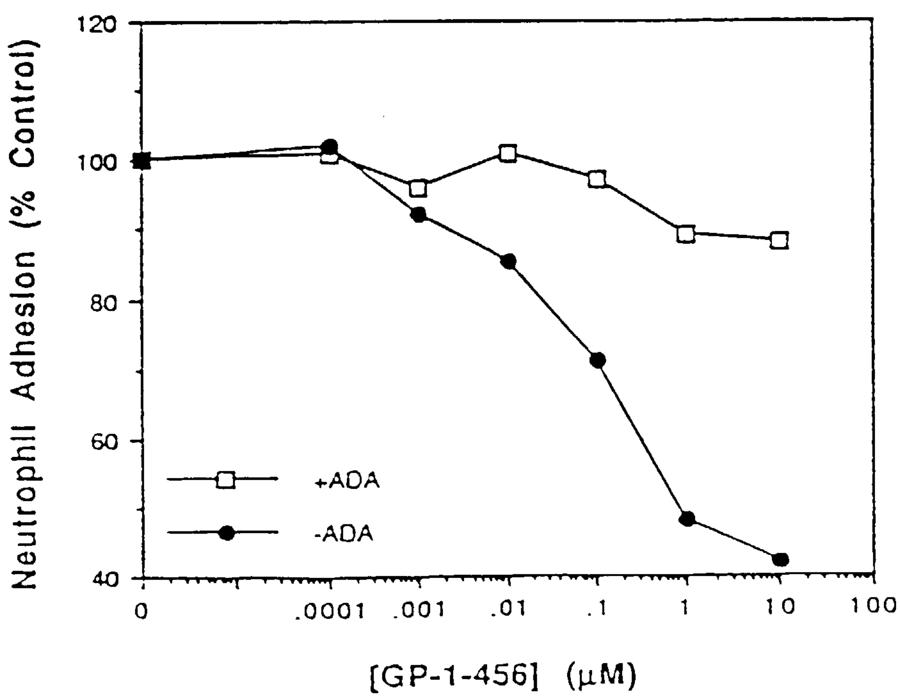
120
100
80
60
40
Neutrophil Adhesion (% Control)
+ADA
-ADA
0
.0001
.001
.01
.1
1
10
100
[GP-1-456] (μM)

Figure 3

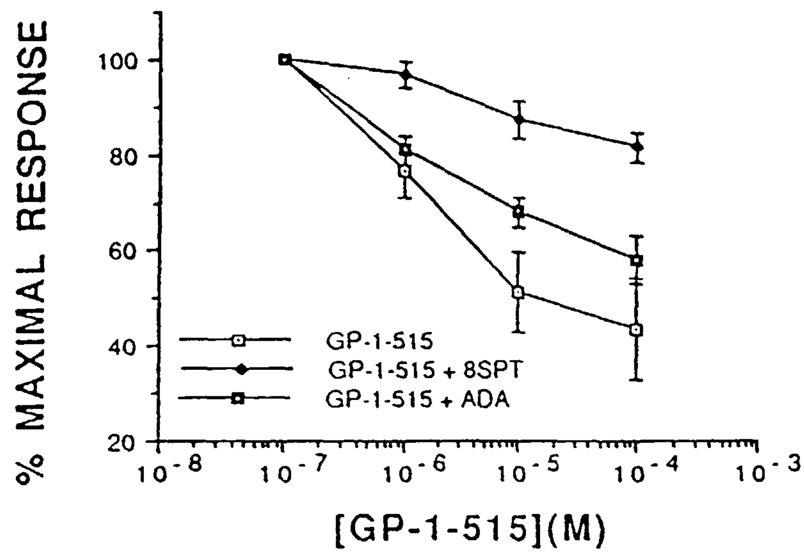
% MAXIMAL RESPONSE
100
80
60
40
20
GP-1-515
GP-1-515 + 8SPT
GP-1-515 + ADA
10^-8
10^-7
10^-6
10^-5
10^-4
10^-3
[GP-1-515](M)

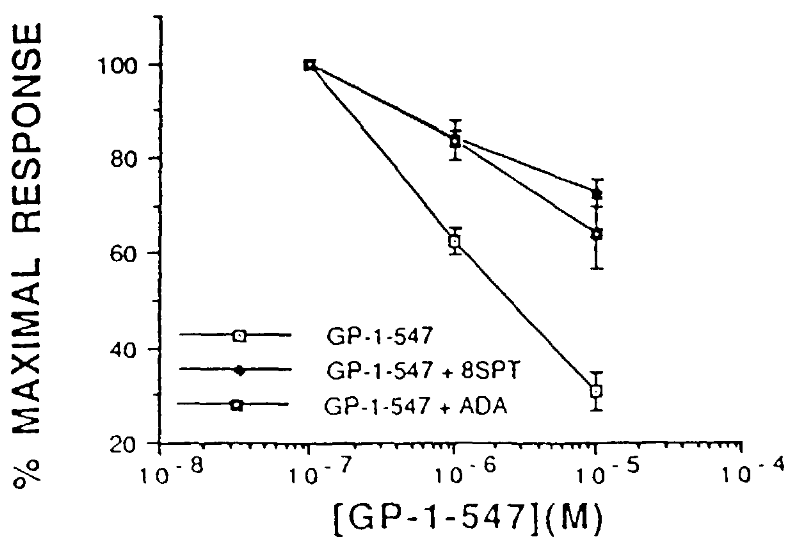
% MAXIMAL RESPONSE
100
80
60
40
20
GP-1-547
GP-1-547 + 8SPT
GP-1-547 + ADA
10^-8
10^-7
10^-6
10^-5
10^-4
[GP-1-547](M)

Figure 4

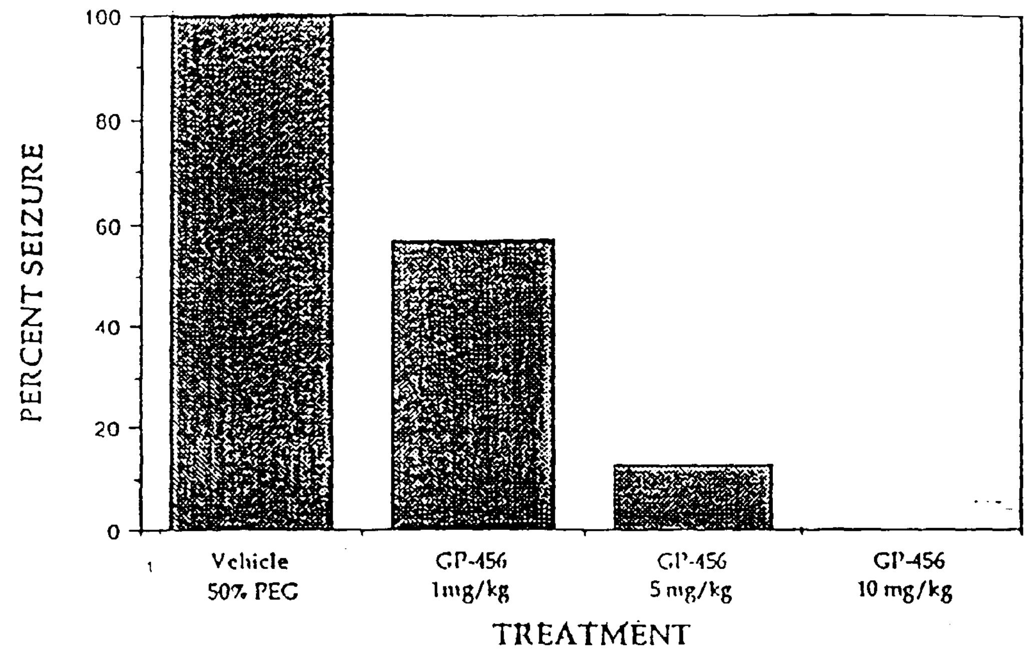
100
80
60
40
20
0
PERCENT SEIZURE
Vehicle
50% PEG
GP-456
1mg/kg
GP-456
5 mg/kg
GP-456
10 mg/kg
TREATMENT

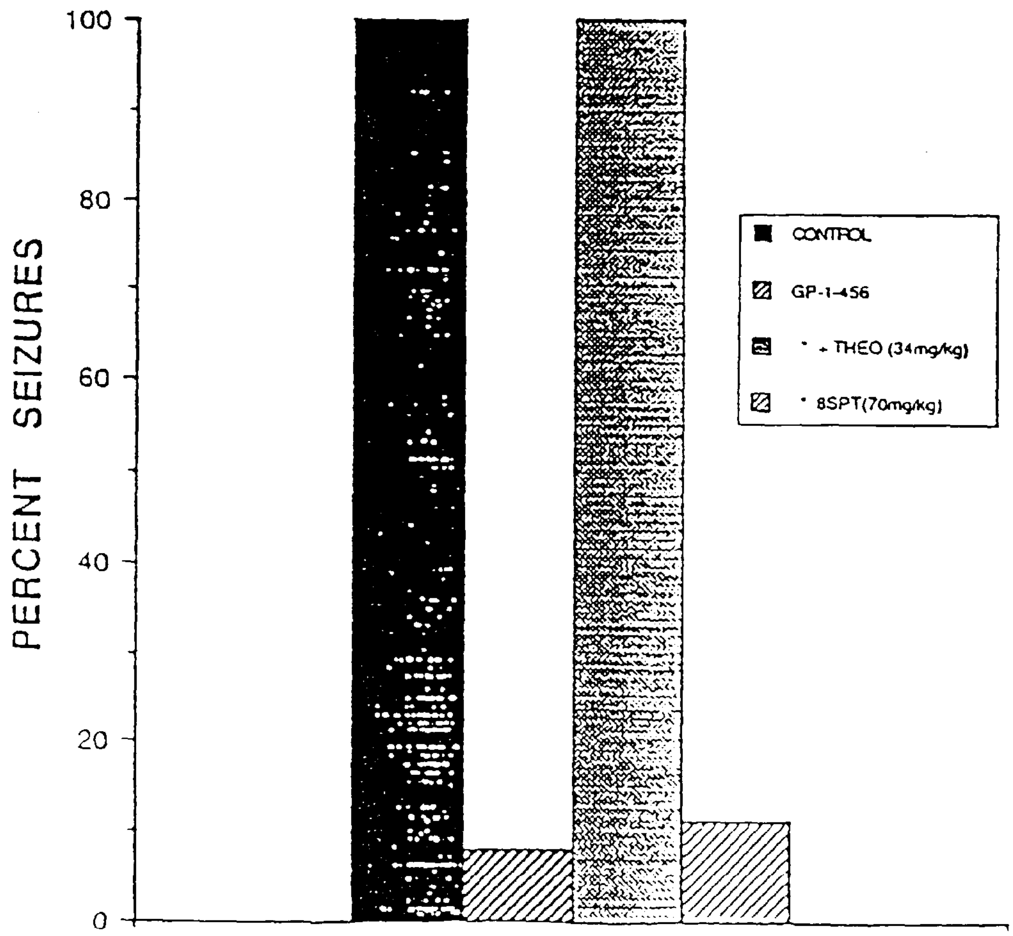
100
80
60
40
20
0
PERCENT SEIZURES
CONTROL
GP-1-456
" + THEO (34mg/kg)
" 8SPT(70mg/kg)

Figure 5

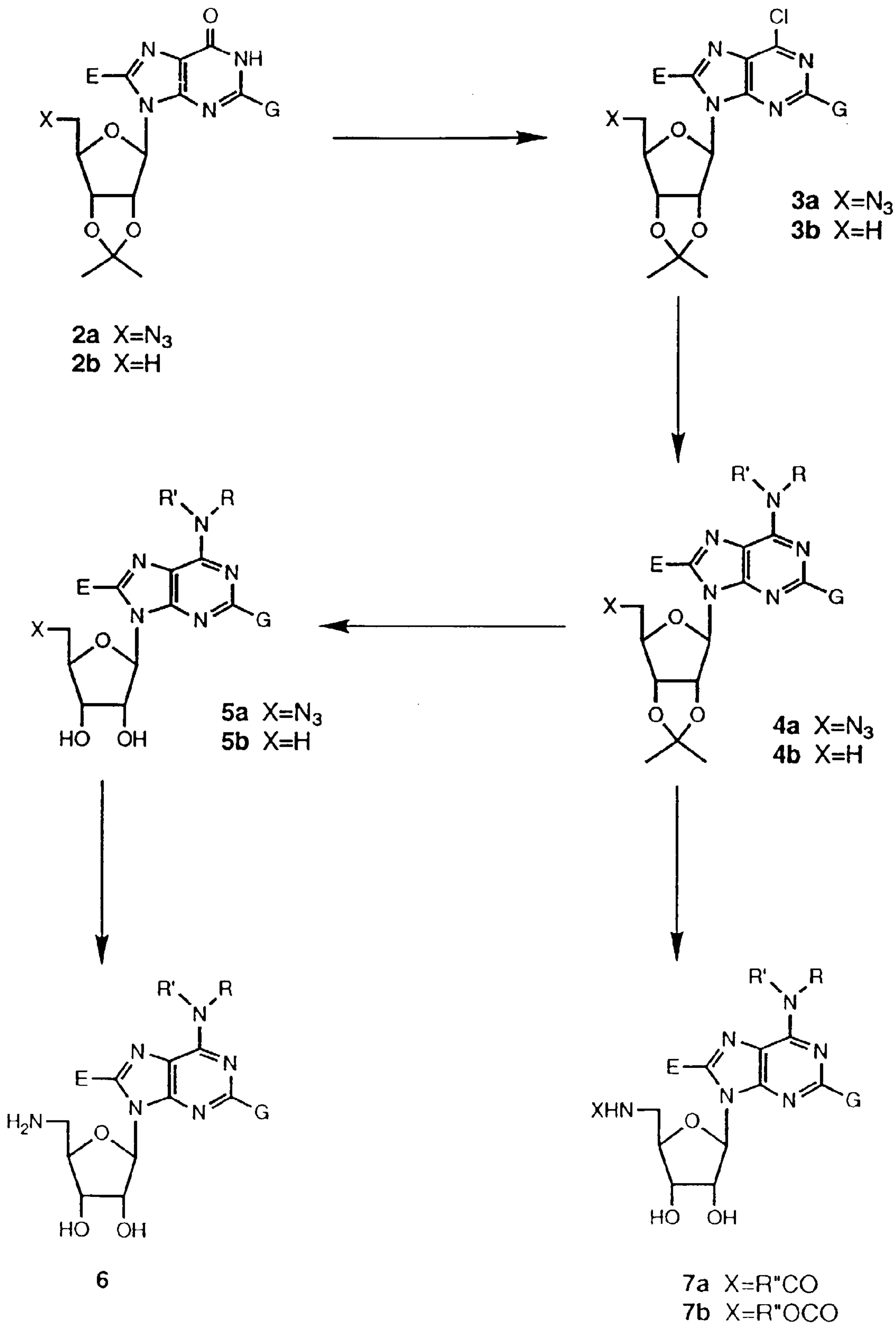

2a X=$N_3$
2b X=H

3a X=$N_3$
3b X=H

4a X=$N_3$
4b X=H

5a X=$N_3$
5b X=H

6

7a X=R"CO
7b X=R"OCO

Figure 6

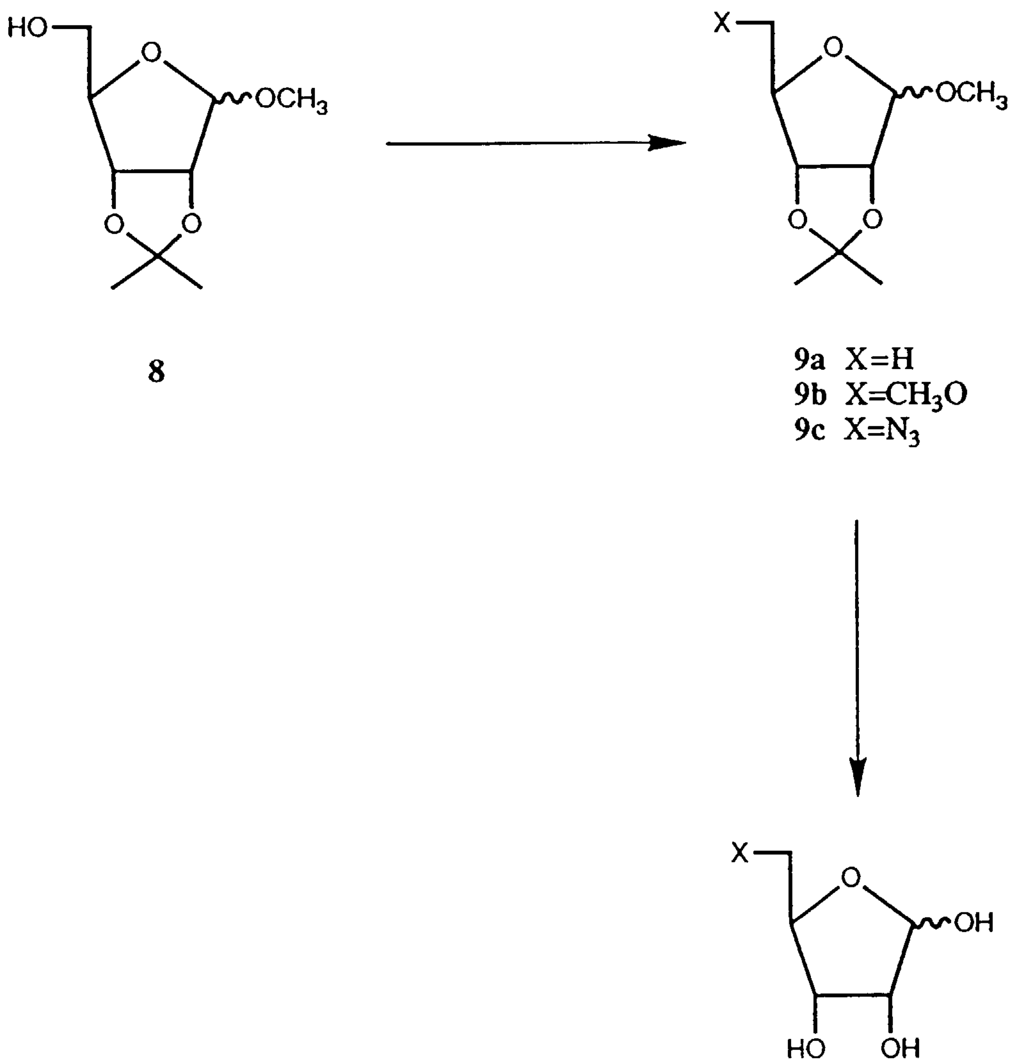

**8**

**9a** X=H
**9b** X=$CH_3O$
**9c** X=$N_3$

**10a** X=H
**10b** X=$CH_3O$
**10c** X=$N_3$

Figure 7

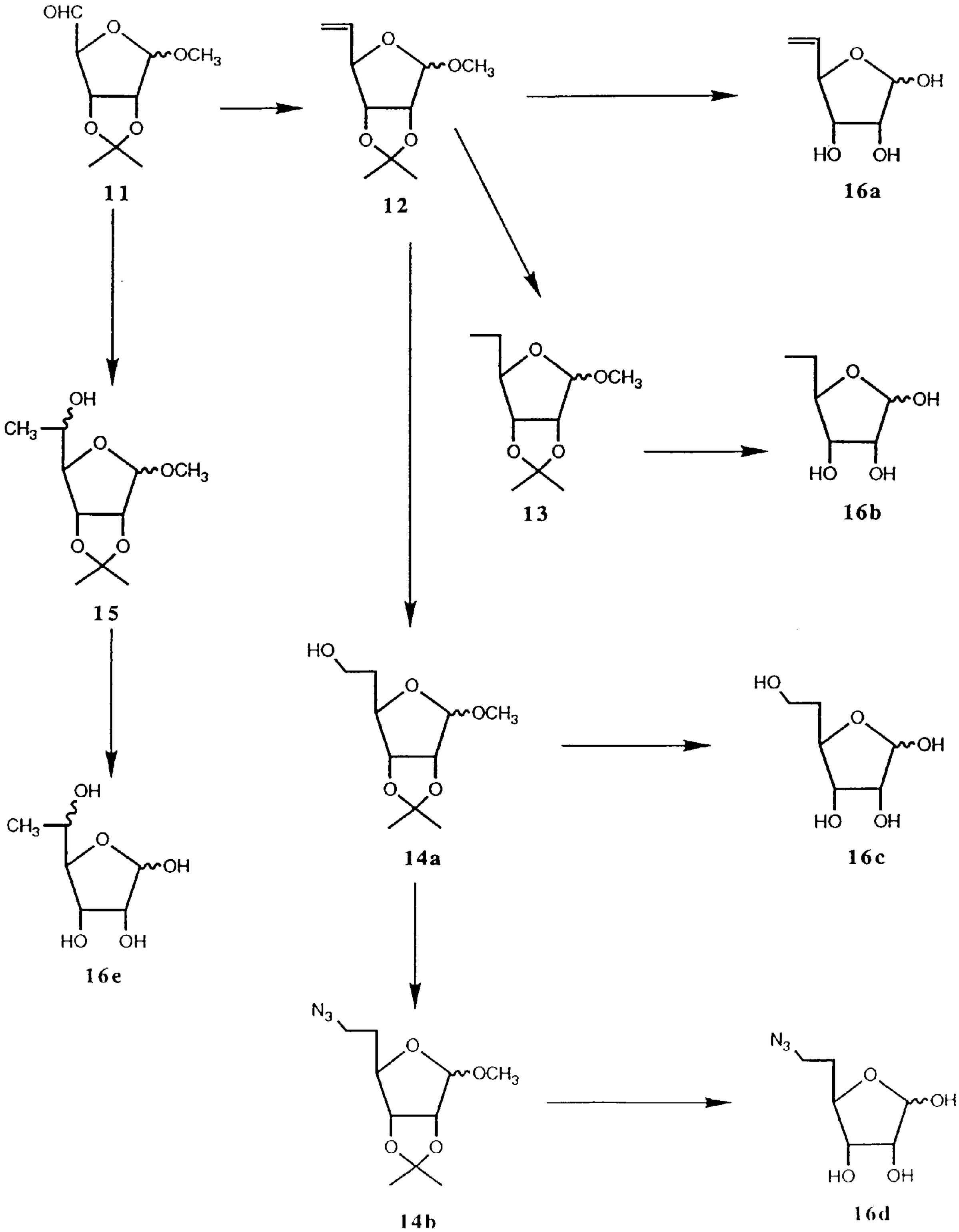

11

12

16a

13

16b

15

14a

16c

16e

14b

16d

Figure 8

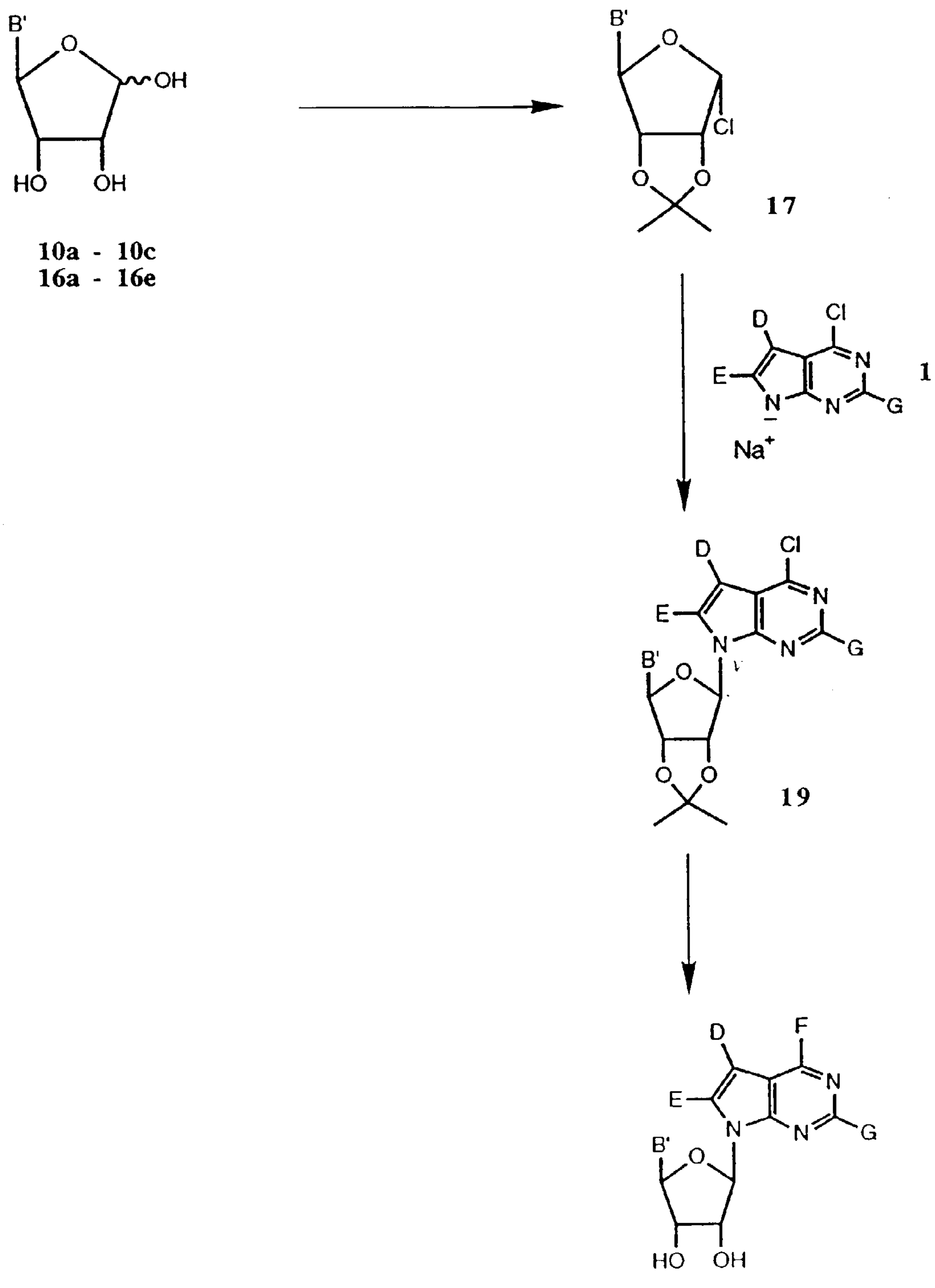

10a - 10c
16a - 16e

17

18

19

20a F=Cl
20b F=$NH_2$ or NHR
20c F=RS
20d F=R

Figure 9

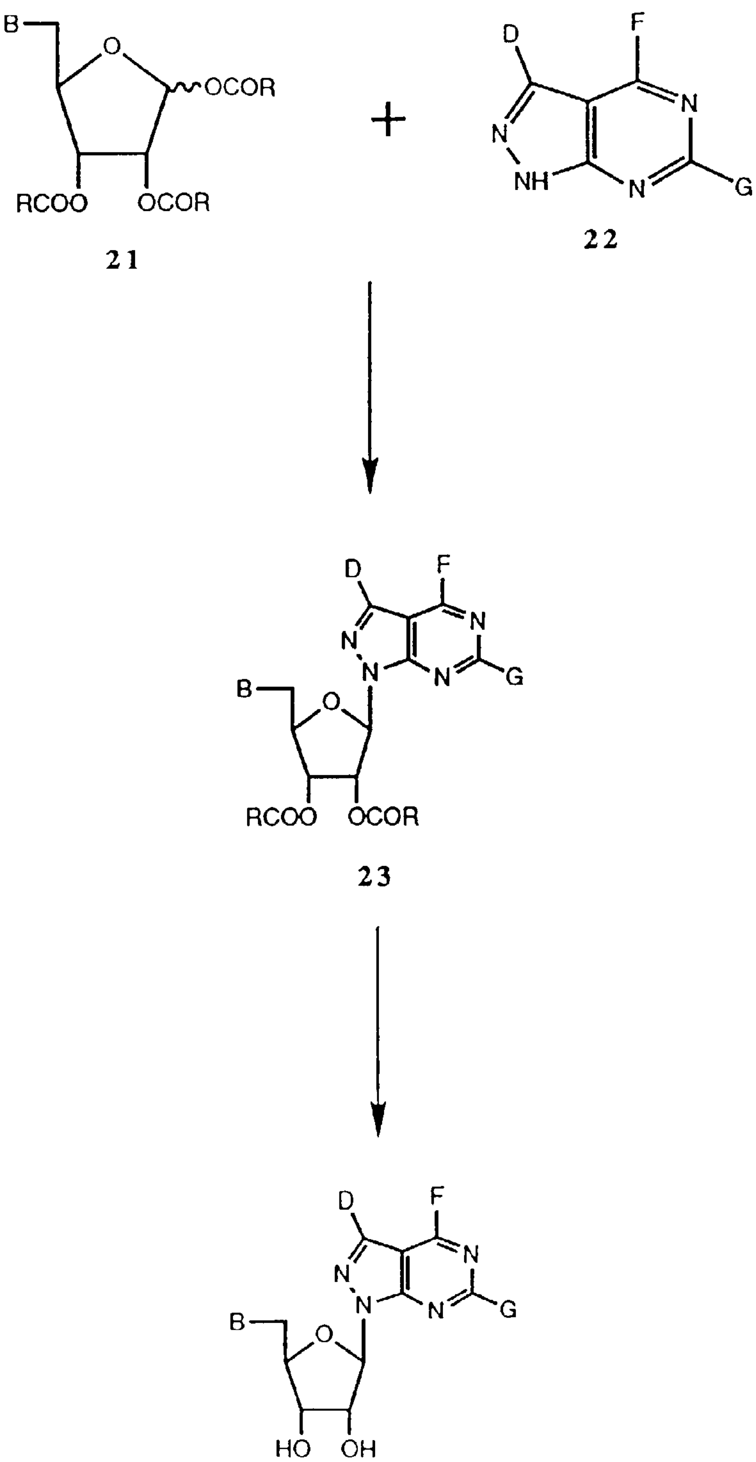

21

22

23

**24a** F=O(H)
**24b** F=$NH_2$ or NHR
**24c** F=R

FIGURE 10

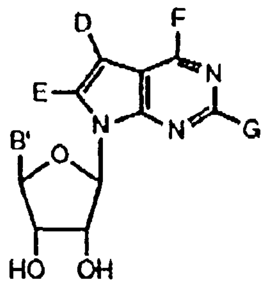

| B | D | E | F | G |
|---|---|---|---|---|
| $CH_3$ | Br | H | Cl | H |
| $CH_3$ | I | H | Cl | H |
| $CH_3$ | I | H | SH | H |
| $-CH_2-CH_3$ | Br | H | Cl | H |
| $-CH_2-CH_3$ | I | H | Cl | H |
| $-CH=CH_2$ | Br | H | Cl | H |
| $-CH=CH_2$ | I | H | Cl | H |
| $-CH_2-N_3$ | I | H | Cl | H |
| $-CH_2-CH_2-N_3$ | I | H | Cl | H |
| $-CH_2-N_3$ | Br | H | Cl | H |
| $CH_3$ | Br | H | HN-aryl | H |
| $CH_3$ | I | H | HN-aryl | H |
| $CH_2OH$ | I | H | HN-aryl | H |
| $CH_2OH$ | Br | H | HN-aryl | H |